# EUROPEAN PATENT APPLICATION

(11) **EP 3 398 602 A1**
(43) Date of publication of application: **07.11.2018**
(21) Application number: 18164803.1
(22) Date of filing: 22.01.2015
(51) Int. Cl.: A61K 31/4745, A61K 31/4353, A61K 45/06, C07D 221/06, A61P 25/14, A61K 31/13, A61K 31/198, A61K 31/4045, A61K 31/428, A61K 31/473, A61K 31/48, A61K 31/5513, C07D 455/06, C07D 487/04

(54) **BENZOQUINOLINE INHIBITORS OF VESICULAR MONOAMINE TRANSPORTER 2**

(30) Priority: 27.01.2014 US 201461932103 P
(62) Divisional of application: 15740017.7
(71) Applicant: Auspex Pharmaceuticals, Inc., North Wales, PA 19454 (US)
(72) Inventor: STAMLER, David, North Wales, PA 19454 (US)
(74) Representative: D Young & Co LLP

(57) **Abstract**

The present invention relates to benzoquinoline inhibitors of vesicular monoamine transporter 2 (VMAT2) of formula I, for use in the treatment of Parkinson's disease levodopa-induced dyskinesia, wherein R1-R27 are hydrogen or deuterium; and at least one of R1-R27 is deuterium.

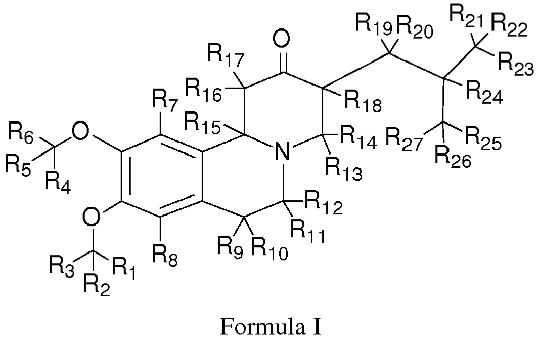

## Description

This application claims the benefit of priority of United States provisional application No. 61/932,103, filed January 27, 2014, the disclosure of which is hereby incorporated by reference as if written herein in its entirety.

Disclosed herein are new benzoquinoline compounds, pharmaceutical compositions made thereof, and methods to inhibit vesicular monoamine transporter 2 (VMAT2) activity in a subject are also provided for, for the treatment of Parkinson's disease levodopa-induced dyskinesia.

Parkinson's disease (PD) is a degenerative disorder characterized by the loss of substantia nigra pars compacta dopaminergic neurons and the subsequent loss of dopaminergic input to the striatum. As the degenerative process evolves, dopamine replacement therapy becomes necessary to help alleviate motor dysfunction.

Levodopa is the most effective agent to alleviate motor dysfunction in Parkinson's disease but its long-term use is associated with the development of dyskinesias. Although the pathogenic processes behind the development of levodopa induced dyskinesias are still being elucidated, it appears that chronic administration of this short-lived agent results in nonphysiologic pulsatile stimulation of striatal neurons and abnormal firing patterns in the basal ganglia.

The pathophysiologic mechanisms that underlie motor complications are not completely understood, but substantial support has been garnered for the idea that nonphysiologic pulsatile stimulation of striatal neurons contributes to a disturbance of basal ganglia homeostasis. Motor complications include motor fluctuations, defined as a loss of clinical benefit before the next levodopa dose (i.e., effect wearing off), and abnormal involuntary movements, termed dyskinesias.

Dyskinesias are usually choreiform but may resemble dystonia, myoclonus, or other movement disorders. Peak-dose dyskinesias are the most common type of dyskinesia. They occur during peaks of levodopa-derived dopamine in the brain, when the patient is otherwise experiencing a beneficial response (the 'on' state). Peak dose dyskinesias worsen with increases in dopaminergic therapy and lessen with reductions in dopaminergic therapy. Some patients exhibit diphasic dyskinesia or dyskinesia-improvement-dyskinesia, which occurs when levodopa-derived dopamine concentrations are increasing or decreasing and the patient is turning 'on' and 'off.' Diphasic dyskinesias are usually more dystonic and preferentially involve the lower extremities compared to peak-dose dyskinesias.

When patients experience motor fluctuations without dyskinesias, it is usually a relatively simple matter to smooth out the clinical response. Levodopa doses can be given closer together, or adjunctive medications that reduce 'off' time can be added. However, once a patient has motor fluctuations and peak-dose dyskinesias, it becomes difficult to smooth the clinical response. Increases in dopaminergic treatment then increase dyskinesias, and decreases in dopaminergic treatment reduce dyskinesias
but worsen parkinsonian motor symptoms.

Tetrabenazine (Nitoman, Xenazine, Ro 1-9569), 1,3,4,6,7,11b-Hexahydro- 9,10-dimethoxy-3-(2-methylpropyl)-2*H*-benzo[a]quinoline, is a vesicular monoamine transporter 2 (VMAT2) inhibitor. Tetrabenazine is commonly prescribed for the treatment of Huntington's disease (Savani et al., Neurology 2007, 68(10), 797; and Kenney et al., Expert Review of Neurotherapeutics 2006, 6(1), 7-17) and has been studied in humans for the treatment of levidopa-induced dyskinesia. Brusa et al., Funct. Neurol., 2013, 28(2), 101-105.

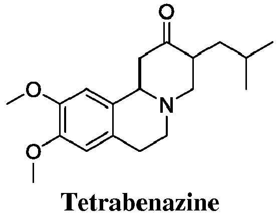

*In vivo,* tetrabenazine is rapidly and extensively metabolized to its reduced form, dihydrotetrabenazine (CAS # 3466-75-9), 1,3,4,6,7,11b-hexahydro-9,10-dimethoxy-3-(2-methylpropyl)-2H-benzo[a]quinolizin-2-ol. Dihydrotetrabenazine is a VMAT2 inhibitor and an active metabolite of tetrabenazine. Dihydrotetrabenazine is also currently under investigation for the treatment of Huntington's disease, hemiballismus, senile chorea, tic disorders, tardive dyskinesia, dystonia, Tourette's syndrome, depression, cancer, rheumatoid arthritis, psychosis, multiple sclerosis, and asthma. WO 2005077946; WO 2007017643; WO 2007017654; WO 2009056885; WO 2010026434; and Zheng et al., The AAPS Journal, 2006, (8)4, E682-692.

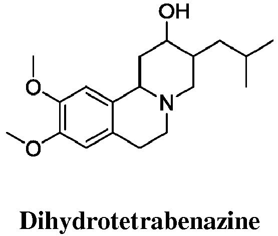

NBI-98854 (CAS # 1025504-59-9), (S)-(2R,3R,11bR)-3-isobutyl-9,10-dimethoxy-2,3,4,6,7,11b-hexahydro-1H-pyrido[2,1-a]isoquinolin-2-yl 2-amino-3-methylbutanoate, is a VMAT2 inhibitor. NBI-98854 is currently under investigation for the treatment of movement disorders including tardive dyskinesia. WO 2008058261; WO 2011153157; and US 8,039,627. NBI-98854, a valine ester of (+)-α-dihydrotetrabenazine, in humans is slowly hydrolyzed to (+)-α-dihydrotetrabenazine which is an active metabolite of tetrabenazine.

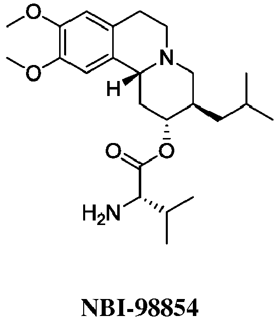

A racemic mixture of [(3R,11bR)/(3S,11bS)]-3-(2-hydroxy-2-methylpropyl)-9,10-di(methoxy-d₃)-1,3,4,6,7,11b-hexahydro-pyrido[2,1-a]isoquinolin-2-one (d₆-Tetrabenazine Metabolite M4 - structures shown below) 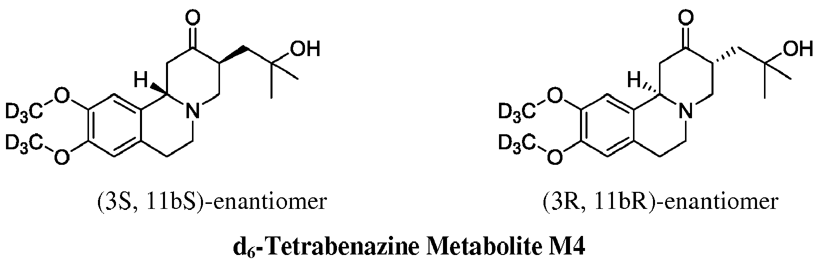 and a diastereomeric mixture of 3-(2-Hydroxy-9,10-di(methoxy-d₃)-1,3,4,6,7,11b-hexahydro-2H-pyrido[2,1-a]isoquinolin-3-yl)-2-methyl-propionic acid (d₆-Tetrabenazine Metabolite M1 - structures shown below) 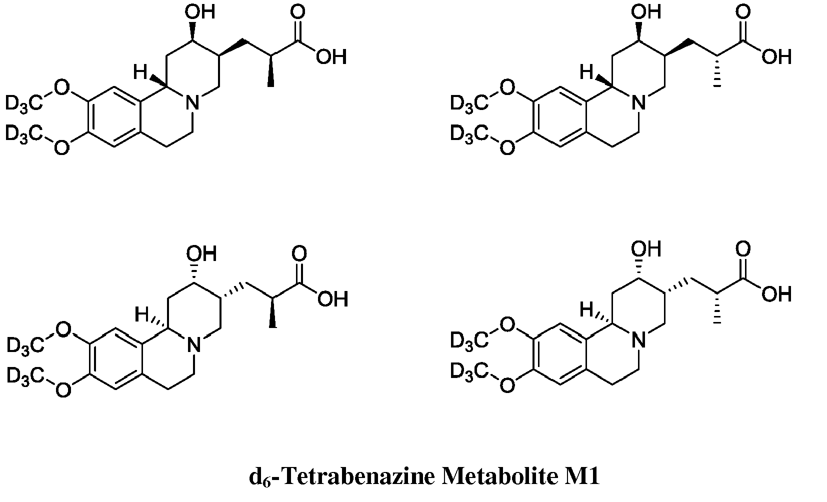 are metabolites of d₆-tetrabenazine and/or d₆-dihydrotetrabenazine. d₆-Tetrabenazine and d₆-dihydrotetrabenazine, as well as the M1 and M4 metabolites, are VMAT2 inhibitors. d₆-Tetrabenazine and d₆-dihydrotetrabenazine are currently under investigation for the treatment of Huntington's disease and other VMAT2-mediated disorders. US 8,524,733, US 20100130480, and US 20120003330.

Tetrabenazine, dihydrotetrabenazine, and NBI-98854 are subject to extensive oxidative metabolism, including O-demethylation of the methoxy groups, as well as hydroxylation of the isobutyl group (Schwartz et al., Biochem. Pharmacol., 1966, 15, 645-655). Adverse effects associated with the administration of tetrabenazine, dihydrotetrabenazine, and/or NBI-98854 include neuroleptic malignant syndrome, drowsiness, fatigue, nervousness, anxiety, insomnia, agitation, confusion, orthostatic hypotension, nausea, dizziness, depression, and Parkinsonism.

### Deuterium Kinetic Isotope Effect

In order to eliminate foreign substances such as therapeutic agents, the animal body expresses various enzymes, such as the cytochrome P₄₅₀ enzymes (CYPs), esterases, proteases, reductases, dehydrogenases, and monoamine oxidases, to react with and convert these foreign substances to more polar intermediates or metabolites for renal excretion. Such metabolic reactions frequently involve the oxidation of a carbon-hydrogen (C-H) bond to either a carbon-oxygen (C-O) or a carbon-carbon (C-C) π-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately lead to administration of multiple or high daily doses.

The relationship between the activation energy and the rate of reaction may be quantified by the Arrhenius equation, k = Ae^{-Eact/RT}. The Arrhenius equation states that, at a given temperature, the rate of a chemical reaction depends exponentially on the activation energy (E_{act}).

The transition state in a reaction is a short lived state along the reaction pathway during which the original bonds have stretched to their limit. By definition, the activation energy E_{act} for a reaction is the energy required to reach the transition state of that reaction. Once the transition state is reached, the molecules can either revert to the original reactants, or form new bonds giving rise to reaction products. A catalyst facilitates a reaction process by lowering the activation energy leading to a transition state. Enzymes are examples of biological catalysts.

Carbon-hydrogen bond strength is directly proportional to the absolute value of the ground-state vibrational energy of the bond. This vibrational energy depends on the mass of the atoms that form the bond, and increases as the mass of one or both of the atoms making the bond increases. Since deuterium (D) has twice the mass of protium (¹H), a C-D bond is stronger than the corresponding C-¹H bond. If a C-¹H bond is broken during a rate-determining step in a chemical reaction (i.e. the step with the highest transition state energy), then substituting a deuterium for that protium will cause a decrease in the reaction rate. This phenomenon is known as the Deuterium Kinetic Isotope Effect (DKIE). The magnitude of the DKIE can be expressed as the ratio between the rates of a given reaction in which a C-¹H bond is broken, and the same reaction where deuterium is substituted for protium. The DKIE can range from about 1 (no isotope effect) to very large numbers, such as 50 or more. Substitution of tritium for hydrogen results in yet a stronger bond than deuterium and gives numerically larger isotope effects

Deuterium (²H or D) is a stable and non-radioactive isotope of hydrogen which has approximately twice the mass of protium (¹H), the most common isotope of hydrogen. Deuterium oxide (D₂O or "heavy water") looks and tastes like H₂O, but has different physical properties.

When pure D₂O is given to rodents, it is readily absorbed. The quantity of deuterium required to induce toxicity is extremely high. When about 0-15% of the body water has been replaced by D₂O, animals are healthy but are unable to gain weight as fast as the control (untreated) group. When about 15-20% of the body water has been replaced with D₂O, the animals become excitable. When about 20-25% of the body water has been replaced with D₂O, the animals become so excitable that they go into frequent convulsions when stimulated. Skin lesions, ulcers on the paws and muzzles, and necrosis of the tails appear. The animals also become very aggressive. When about 30% of the body water has been replaced with D₂O, the animals refuse to eat and become comatose. Their body weight drops sharply and their metabolic rates drop far below normal, with death occurring at about 30 to about 35% replacement with D₂O. The effects are reversible unless more than thirty percent of the previous body weight has been lost due to D₂O. Studies have also shown that the use of D₂O can delay the growth of cancer cells and enhance the cytotoxicity of certain antineoplastic agents.

Deuteration of pharmaceuticals to improve pharmacokinetics (PK), pharmacodynamics (PD), and toxicity profiles has been demonstrated previously with some classes of drugs. For example, the DKIE was used to decrease the hepatotoxicity of halothane, presumably by limiting the production of reactive species such as trifluoroacetyl chloride. However, this method may not be applicable to all drug classes. For example, deuterium incorporation can lead to metabolic switching. Metabolic switching occurs when xenogens, sequestered by Phase I enzymes, bind transiently and re-bind in a variety of conformations prior to the chemical reaction (e.g., oxidation). Metabolic switching is enabled by the relatively vast size of binding pockets in many Phase I enzymes and the promiscuous nature of many metabolic reactions. Metabolic switching can lead to different proportions of known metabolites as well as altogether new metabolites. This new metabolic profile may impart more or less toxicity. Such pitfalls are non-obvious and are not predictable *a priori* for any drug class.

Tetrabenazine, dyhydrotetrabenazine, and NBI-98854 are VMAT2 inhibitors. The carbon-hydrogen bonds of tetrabenazine, dyhydrotetrabenazine, and NBI-98854 contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation may produce a detectable Deuterium Kinetic Isotope Effect (DKIE) that could affect the pharmacokinetic, pharmacologic and/or toxicologic profiles of tetrabenazine, dyhydrotetrabenazine, and/or NBI-98854 in comparison with tetrabenazine, dyhydrotetrabenazine, and/or NBI-98854 having naturally occurring levels of deuterium.

Based on discoveries made in our laboratory, as well as considering the literature, tetrabenazine, dyhydrotetrabenazine, and/or NBI-98854 are metabolized in humans at the isobutyl and methoxy groups. The current approach has the potential to prevent metabolism at these sites. Other sites on the molecule may also undergo transformations leading to metabolites with as-yet-unknown pharmacology/toxicology. Limiting the production of these metabolites has the potential to decrease the danger of the administration of such drugs and may even allow increased dosage and/or increased efficacy. All of these transformations can occur through polymorphically-expressed enzymes, exacerbating interpatient variability. Further, some disorders are best treated when the subject is medicated around the clock or for an extended period of time. For all of the foregoing reasons, a medicine with a longer half-life may result in greater efficacy and cost savings. Various deuteration patterns can be used to (a) reduce or eliminate unwanted metabolites, (b) increase the half-life of the parent drug, (c) decrease the number of doses needed to achieve a desired effect, (d) decrease the amount of a dose needed to achieve a desired effect, (e) increase the formation of active metabolites, if any are formed, (f) decrease the production of deleterious metabolites in specific tissues, and/or (g) create a more effective drug and/or a safer drug for polypharmacy, whether the polypharmacy be intentional or not. The deuteration approach has the strong potential to slow the metabolism of tetrabenazine, dyhydrotetrabenazine, and/or NBI-98854 and attenuate interpatient variability.

Novel compounds and pharmaceutical compositions, certain of which have been found to inhibit VMAT2 have been discovered, together with methods of synthesizing and using the compounds, including methods for the treatment of VMAT2-mediated disorders in a patient by administering the compounds as disclosed herein.

In certain embodiments of the present invention, compounds have structural Formula I: 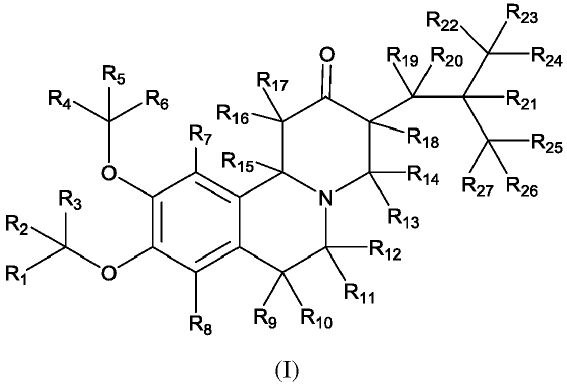 or a salt, solvate, or prodrug thereof, wherein:
R₁-R₂₇ are independently selected from the group consisting of hydrogen and deuterium; and
at least one of R₁-R₂₇ is deuterium.

In certain embodiments, Formula I can include a single enantiomer, a mixture of the (+)-enantiomer and the (-)-enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, an individual diastereomer, or a mixture of diastereomers thereof.

In certain embodiments of the present invention, compounds have structural Formula II: 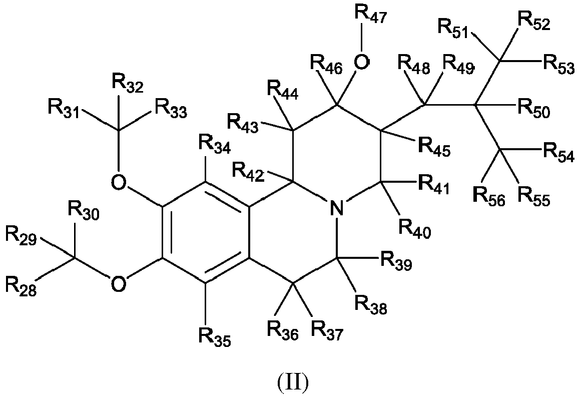 or a salt thereof, wherein:
R₂₈-R₄₆ and R₄₈-R₅₆ are independently selected from the group consisting of hydrogen and deuterium;
R₄₇ is selected from the group consisting of hydrogen, deuterium, -C(O)O-alkyl and -C(O)-C₁₋₆alkyl, or a group cleavable under physiological conditions, wherein said alkyl or C₁₋₆alkyl is optionally substituted with one or more substituents selected from the group consisting of -NH-C(NH)NH2, -CO₂H,-CO₂alkyl, -SH, -C(O)NH₂, -NH₂, phenyl, -OH, 4-hydroxyphenyl, imidazolyl, and indolyl, and any R₄₆ substituent is further optionally substituted with deuterium; and
at least one of R₂₈-R₅₆ is deuterium or contains deuterium.

In certain embodiments, the compounds of Formula II have alpha stereochemistry.

In further embodiments, the compounds of Formula II have beta stereochemistry.

In yet further embodiments, the compounds of Formula II are a mixture of alpha and beta stereoisomers. In yet furher embodiments, the ratio of alpha/beta stereoisomers is at least 100:1, at least 50:1, at least 20:1, at least 10:1, at least 5:1, at least 4:1, at least 3:1, or at least 2:1. In yet furher embodiments, the ratio of beta/alpha stereoisomers is at least 100:1, at least 50:1, at least 20:1, at least 10:1, at least 5:1, at least 4:1, at least 3:1, or at least 2:1.

In certain embodiments, if R₅₀-R₅₆ are deuterium, at least one of R₁-R₄₉ is deuterium.

In certain embodiments of the present invention, compounds have structural Formula III: 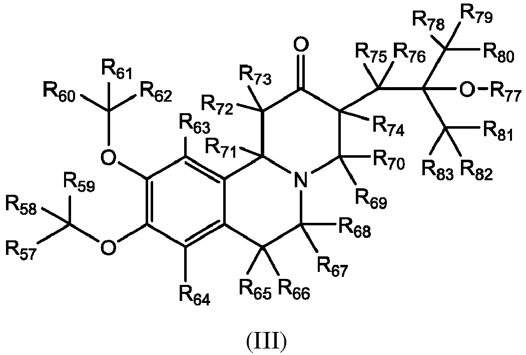 or a salt, stereoisomer, or racemic mixture thereof, wherein:
R₅₇-R₈₃ are independently selected from the group consisting of hydrogen and deuterium; and
at least one of R₅₇-R₈₃ is deuterium.

In certain embodiments of the present invention, compounds have structural Formula IV: 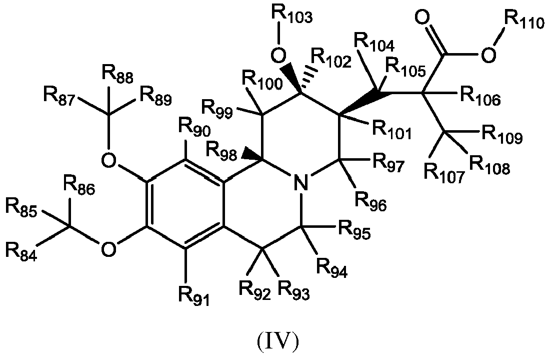 or a salt, diastereomer, or mixture of diastereomers thereof, wherein:
R₈₄-R₁₁₀ are independently selected from the group consisting of hydrogen and deuterium; and
at least one of R₈₄-R₁₁₀ is deuterium.

Certain compounds disclosed herein may possess useful VMAT2 inhibiting activity, and may be used in the treatment or prophylaxis of a disorder in which VMAT2 plays an active role. Thus, certain embodiments also provide pharmaceutical compositions comprising one or more compounds disclosed herein together with a pharmaceutically acceptable carrier, as well as methods of making and using the compounds and compositions. Certain embodiments provide methods for inhibiting VMAT2. Other embodiments provide methods for treating a VMAT2-mediated disorder in a patient in need of such treatment, comprising administering to said patient a therapeutically effective amount of a compound or composition according to the present invention. Also provided is the use of certain compounds disclosed herein for use in the manufacture of a medicament for the prevention or treatment of a disorder ameliorated by the inhibition of VMAT2.

In certain embodiments, disclosed herein is a compound of structural Formula I 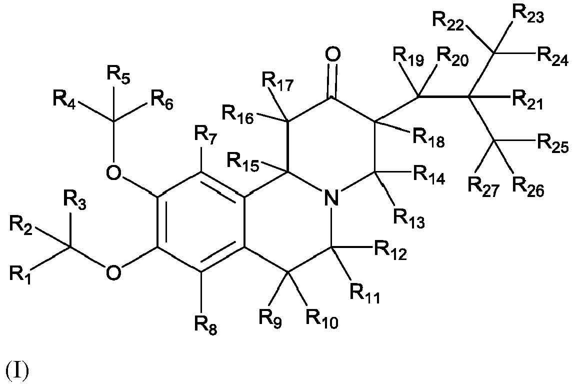 or a salt, stereoisomer, or racemic mixture thereof, wherein:
R₁-R₂₇ are independently selected from the group consisting of hydrogen and deuterium; and
at least one of R₁-R₂₇ is deuterium;
for use in the treatment of Parkinson's disease levodopa-induced dyskinesia.

In further embodiments at least one of R₁-R₂₇ independently has deuterium enrichment of no less than about 10%.

In further embodiments at least one of R₁-R₂₇ independently has deuterium enrichment of no less than about 50%.

In further embodiments at least one of R₁-R₂₇ independently has deuterium enrichment of no less than about 90%.

In further embodiments at least one of R₁-R₂₇ independently has deuterium enrichment of no less than about 98%.

In further embodiments said compound has the structural formula:

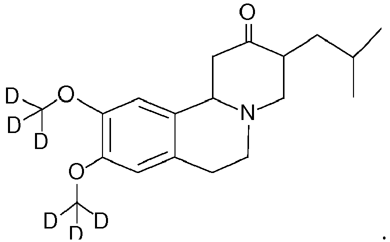

In certain embodiments, disclosed herein is a compound of structural Formula II 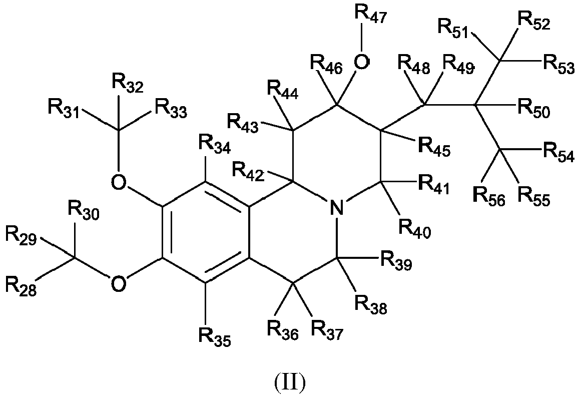 or a salt, stereoisomer, or racemic mixture thereof, wherein:
R₂₈-R₅₆ are independently selected from the group consisting of hydrogen and deuterium; and
at least one of R₂₈-R₅₆ is deuterium;
for use in the treatment of Parkinson's disease levodopa-induced dyskinesia.

In further embodiments at least one of R₂₈-R₅₆ independently has deuterium enrichment of no less than about 10%.

In further embodiments at least one of R₂₈-R₅₆ independently has deuterium enrichment of no less than about 50%.

In further embodiments at least one of R₂₈-R₅₆ independently has deuterium enrichment of no less than about 90%.

In further embodiments at least one of R₂₈-R₅₆ independently has deuterium enrichment of no less than about 98%.

In further embodiments said compound has the structural formula:

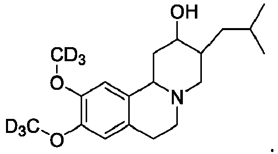

In further embodiments said compound is the alpha stereoisomer.

In further embodiments said compound is the beta stereoisomer.

In further embodiments said compound has the structural formula:

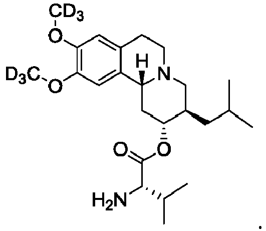

In certain embodiments, disclosed herein is a compound of structural Formula III 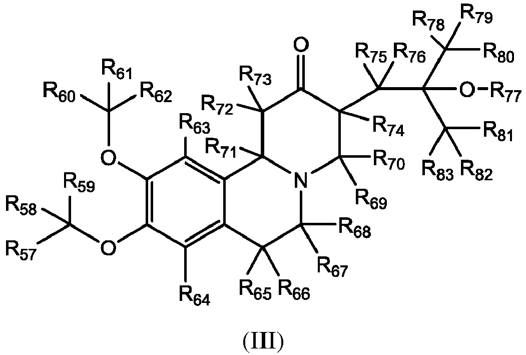 or a salt, stereoisomer, or racemic mixture thereof, wherein:
R₅₇-R₈₃ are independently selected from the group consisting of hydrogen and deuterium; and
at least one of R₅₇-R₈₃ is deuterium;
for use in the treatment of Parkinson's disease levodopa-induced dyskinesia.

In further embodiments at least one of R₅₇-R₈₃ independently has deuterium enrichment of no less than about 10%.

In further embodiments at least one of R₅₇-R₈₃ independently has deuterium enrichment of no less than about 50%.

In further embodiments at least one of R₅₇-R₈₃ independently has deuterium enrichment of no less than about 90%.

In further embodiments at least one of R₅₇-R₈₃ independently has deuterium enrichment of no less than about 98%.

In further embodiments said compound has the structural formula: 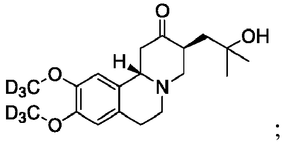 or the 3S,11bS enantiomer, 3R,11bR enantiomer, or a racemic mixture of the the 3S,11bS and 3R,11bR enantiomers.

In certain embodiments, disclosed herein is a compound of structural Formula IV 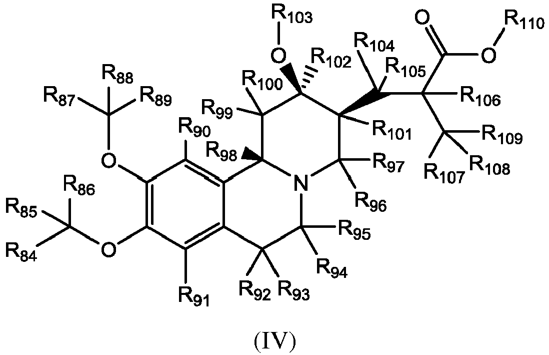 or a salt, stereoisomer, or racemic mixture thereof, wherein:
R₈₄-R₁₁₀ are independently selected from the group consisting of hydrogen and deuterium; and
at least one of R₈₄-R₁₁₀ is deuterium;
for use in the treatment of Parkinson's disease levodopa-induced dyskinesia.

In further embodiments at least one of R₈₄-R₁₁₀ independently has deuterium enrichment of no less than about 10%.

In further embodiments at least one of R₈₄-R₁₁₀ independently has deuterium enrichment of no less than about 50%.

In further embodiments at least one of R₈₄-R₁₁₀ independently has deuterium enrichment of no less than about 90%.

In further embodiments at least one of R₈₄-R₁₁₀ independently has deuterium enrichment of no less than about 98%.

In further embodiments said compound has the structural formula: 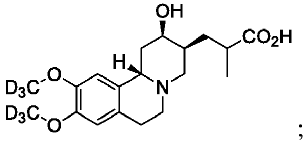 or a diastereomer, or mixture of diastereomers thereof.

In further embodiments of any of the foregoing compounds each position represented as D has deuterium enrichment of no less than about 10%.

In further embodiments of any of the foregoing compounds each position represented as D has deuterium enrichment of no less than about 50%.

In further embodiments of any of the foregoing compounds each position represented as D has deuterium enrichment of no less than about 90%.

In further embodiments of any of the foregoing compounds each position represented as D has deuterium enrichment of no less than about 98%.

The compounds as disclosed herein may also contain less prevalent isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen.

In certain embodiments, the compound disclosed herein may expose a patient to a maximum of about 0.000005% D₂O or about 0.00001% DHO, assuming that all of the C-D bonds in the compound as disclosed herein are metabolized and released as D₂O or DHO. In certain embodiments, the levels of D₂O shown to cause toxicity in animals is much greater than even the maximum limit of exposure caused by administration of the deuterium enriched compound as disclosed herein. Thus, in certain embodiments, the deuterium-enriched compound disclosed herein should not cause any additional toxicity due to the formation of D₂O or DHO upon drug metabolism.

In certain embodiments, the deuterated compounds disclosed herein maintain the beneficial aspects of the corresponding non-isotopically enriched molecules while substantially increasing the maximum tolerated dose, decreasing toxicity, increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions.

All publications and references cited herein are expressly incorporated herein by reference in their entirety. However, with respect to any similar or identical terms found in both the incorporated publications or references and those explicitly put forth or defined in this document, then those terms definitions or meanings explicitly put forth in this document shall control in all respects.

As used herein, the terms below have the meanings indicated.

The singular forms "a," "an," and "the" may refer to plural articles unless specifically stated otherwise.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

When ranges of values are disclosed, and the notation "from n₁ ... to n₂" or "n₁-n₂" is used, where n₁ and n₂ are the numbers, then unless otherwise specified, this notation is intended to include the numbers themselves and the range between them. This range may be integral or continuous between and including the end values.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods known to one of ordinary skill in the art, including mass spectrometry and nuclear magnetic resonance spectroscopy.

The term "is/are deuterium," when used to describe a given position in a molecule such as R₁-R₁₁₀ or the symbol "D", when used to represent a given position in a drawing of a molecular structure, means that the specified position is enriched with deuterium above the naturally occurring distribution of deuterium. In one embodiment deuterium enrichment is no less than about 1%, in another no less than about 5%, in another no less than about 10%, in another no less than about 20%, in another no less than about 50%, in another no less than about 70%, in another no less than about 80%, in another no less than about 90%, or in another no less than about 98% of deuterium at the specified position.

The term "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

Asymmetric centers exist in the compounds disclosed herein. These centers are designated by the symbols "R" or "S," depending on the configuration of substituents around the chiral carbon atom. It should be understood that the invention encompasses all stereochemical isomeric forms, including diastereomeric, enantiomeric, and epimeric forms, as well as D-isomers and L-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention. Additionally, the compounds disclosed herein can exist in unsolvated as well as solvated forms with pharmaceutically acceptable solvents such as water, ethanol, and the like. In general, the solvated forms are considered equivalent to the unsolvated forms.

The terms "alpha-dihydrotetrabenazine", "a-dihydrotetrabenazine", or the terms "alpha" or "alpha stereoisomer" or the symbol "α" as applied to dihydrotetrabenazine refers to either of the dihydrotetrabenazine stereoisomers having the structural formulas shown below, or a mixture thereof:

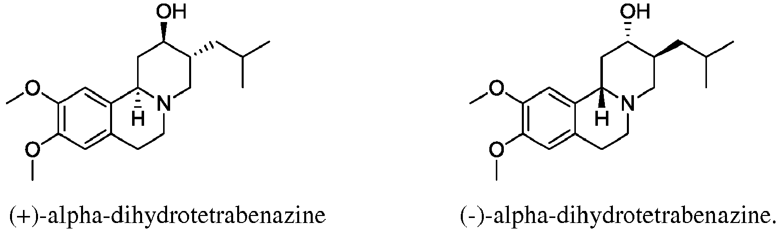

The terms "alpha" or "alpha stereoisomer" or the symbol "α" as applied to a compound of Formula II refers to either of the stereoisomers of compounds of Formula II shown below, or a mixture thereof: 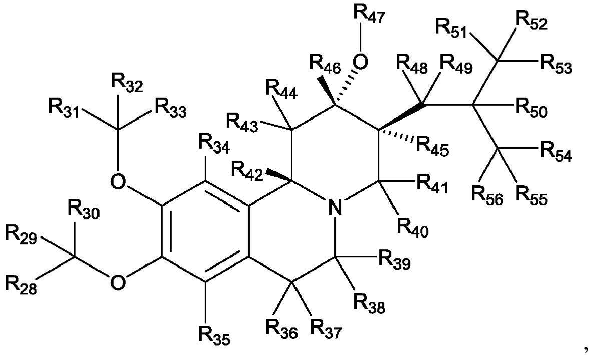 and

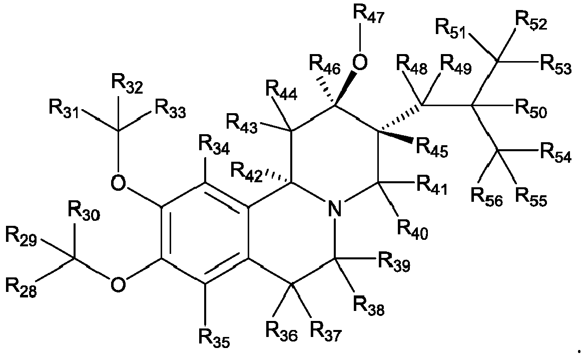

The terms "beta-dihydrotetrabenazine", "β-dihydrotetrabenazine", or the terms "beta" or "beta stereoisomer" or the symbol "β" as applied to dihydrotetrabenazine refers to either of the dihydrotetrabenazine stereoisomers having the structural formulas shown below, or a mixture thereof:

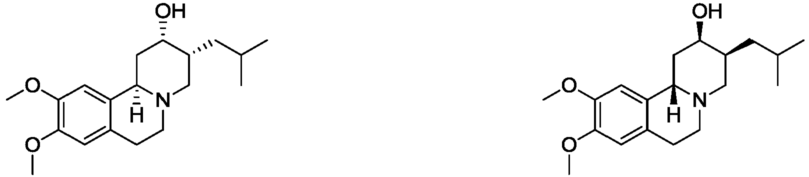

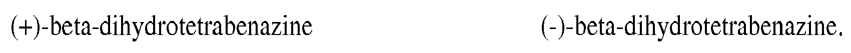

The terms "beta" or "beta stereoisomer" or the symbol "β" as applied to a compound of Formula II refers to either of the stereoisomers of compounds of Formula II shown below, or a mixture thereof: 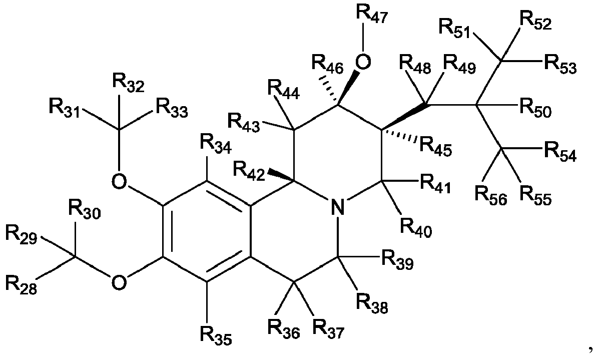 and

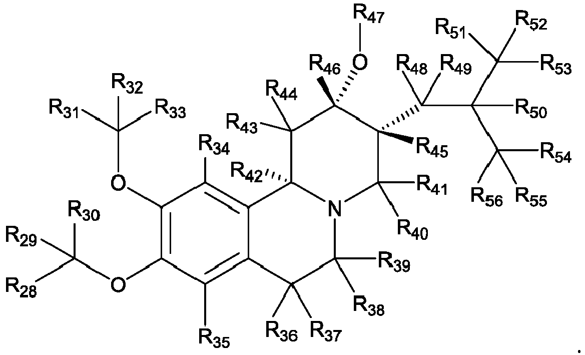

The terms "3S,11bS enantiomer" or the term "3R,11bR enantiomer" refers to either of the d₆-tetrabenazine M4 metabolite stereoisomers having the structural formulas shown below: 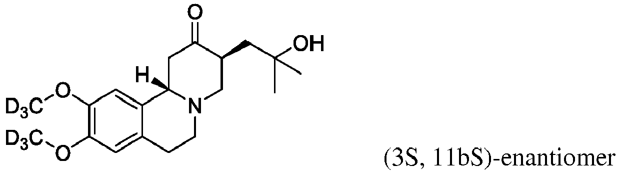 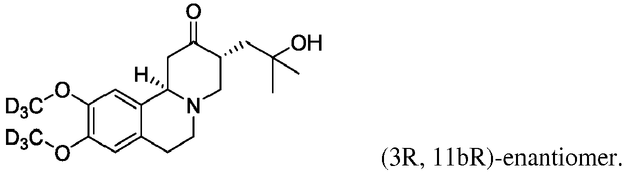 In certain embodiments, a chemical structure may be drawn as either the 3S,11bS enantiomer or the 3R,11bR enantiomer, but the text of the specification may indicate that the 3S,11bS enantiomer, the 3R,11bR enantiomer, a racemic mixture thereof, or all of the foregoing may be intended to be described.

The terms "(3S, 11bS)-enantiomer" or "(3R, 11bR)-enantiomer" or the as applied to a compound of Formula I refers to either of the stereoisomers of compounds of Formula III shown below:

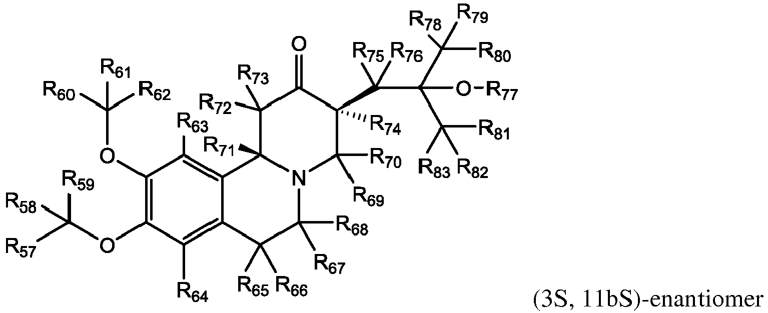

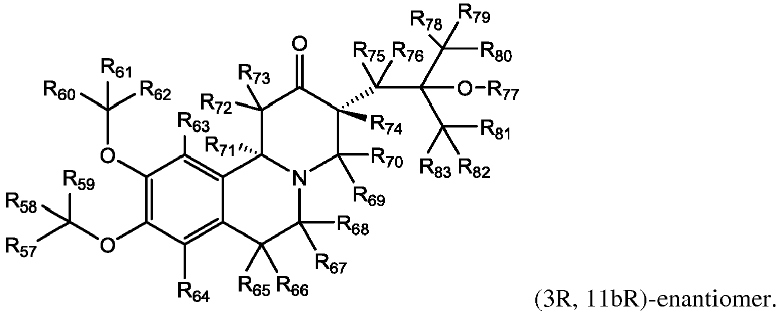

The term "mixture of diastereomers" refers to either of the d₆-tetrabenazine M1 metabolite stereoisomers having the structural formulas shown below: 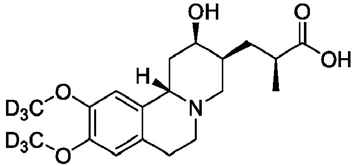 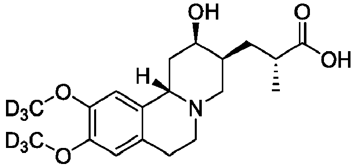 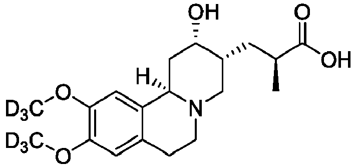 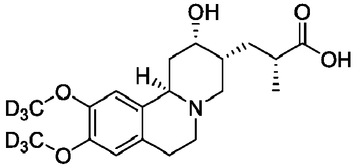 In certain embodiments, a chemical structure may be drawn as one of the diastereomers shown above, but the text of the specification may indicate that each individual diastereomer or a mixture thereof, or all of the foregoing may be intended to be described.

The term "mixture of diastereomers" as applied to a compound of Formula IV refers to a mixture of the stereoisomers of compounds of Formula IV shown below:

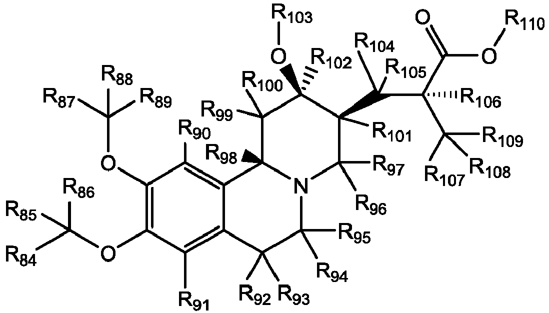

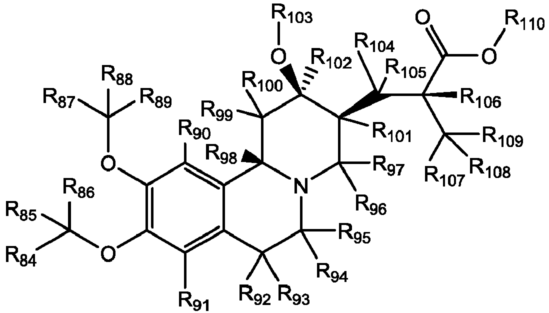

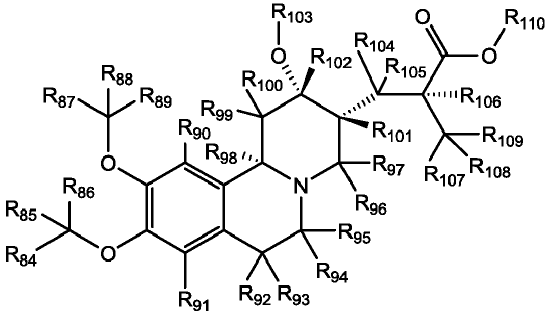

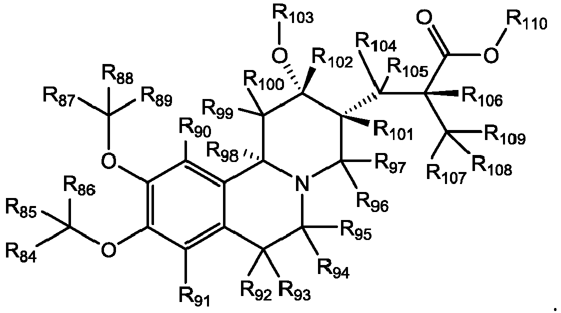

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disorder" as used herein is intended to be generally synonymous, and is used interchangeably with, the terms "disease", "syndrome", and "condition" (as in medical condition), in that all reflect an abnormal condition of the human or animal body or of one of its parts that impairs normal functioning, is typically manifested by distinguishing signs and symptoms.

The terms "treat," "treating," and "treatment" are meant to include alleviating or abrogating a disorder or one or more of the symptoms associated with a disorder; or alleviating or eradicating the cause(s) of the disorder itself. As used herein, reference to "treatment" of a disorder is intended to include prevention. The terms "prevent," "preventing," and "prevention" refer to a method of delaying or precluding the onset of a disorder; and/or its attendant symptoms, barring a subject from acquiring a disorder or reducing a subject's risk of acquiring a disorder.

The term "therapeutically effective amount" refers to the amount of a compound that, when administered, is sufficient to prevent development of, or alleviate to some extent, one or more of the symptoms of the disorder being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

The term "subject" refers to an animal, including, but not limited to, a primate (e.g., human, monkey, chimpanzee, gorilla, and the like), rodents (e.g., rats, mice, gerbils, hamsters, ferrets, and the like), lagomorphs, swine (e.g., pig, miniature pig), equine, canine, feline, and the like. The terms "subject" and "patient" are used interchangeably herein in reference, for example, to a mammalian subject, such as a human patient.

The term "combination therapy" means the administration of two or more therapeutic agents to treat a therapeutic disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the disorders described herein.

The term "Parkinson's disease levodopa-induced dyskinesia," "levodopa-induced dyskinesia," or "LID" refers to an abnormal muscular activity disorder characterized by either disordered or excessive movement (referred to as "hyperkinesia" or "dyskinesia"), or slowness, or a lack of movement (referred to as "hypokinesia," "bradykinesia," or "akinesia"). Based on their relationship with levodopa dosing, levodopa-induced dyskinesias are classified as peak-dose, diphasic, off state, on state, and yo yo dyskinesias. Peak-dose dyskinesias are the most common forms of LID and are related to peak plasma (and possibly high striatal) levels of levodopa. They involve the head, trunk, and limbs, and sometimes respiratory muscles. Dose reduction can ameliorate them, frequently at the cost of deterioration of parkinsonism. Peak-dose dyskinesias are usually choreiform, though in the later stages dystonia can superimpose. Diphasic dyskinesias develop when plasma levodopa levels are rising or falling, but not with the peak levels. They are also called D-I-D (dyskinesia-improvement-dyskinesia). D-I-D are commonly dystonic in nature, though chorea or mixed pattern may occur. They do not respond to levodopa dose reduction and may rather improve with high dose of levodopa. "Off" state dystonias occur when plasma levodopa levels are low (for example, in the morning). They are usually pure dystonia occurring as painful spasms in one foot. They respond to levodopa therapy. Rare forms of LID include "on" state dystonias (occurring during higher levels of levodopa) and yo-yo dyskinesia (completely unpredictable pattern).

The term "VMAT2" refers to vesicular monoamine transporter 2, an integral membrane protein that acts to transport monoamines-particularly neurotransmitters such as dopamine, norepinephrine,serotonin, and histamine-from cellular cytosol into synaptic vesicles.

The term "VMAT2-mediated disorder," refers to a disorder that is characterized by abnormal VMAT2 activity. A VMAT2-mediated disorder may be completely or partially mediated by modulating VMAT2. In particular, a VMAT2-mediated disorder is one in which inhibition of VMAT2 results in some effect on the underlying disorder e.g., administration of a VMAT2 inhibitor results in some improvement in at least some of the patients being treated.

The term "VMAT2 inhibitor", "inhibit VMAT2", or "inhibition of VMAT2" refers to the ability of a compound disclosed herein to alter the function of VMAT2. A VMAT2 inhibitor may block or reduce the activity of VMAT2 by forming a reversible or irreversible covalent bond between the inhibitor and VMAT2 or through formation of a noncovalently bound complex. Such inhibition may be manifest only in particular cell types or may be contingent on a particular biological event. The term "VMAT2 inhibitor", "inhibit VMAT2", or "inhibition of VMAT2" also refers to altering the function of VMAT2 by decreasing the probability that a complex forms between a VMAT2 and a natural substrate

The term "therapeutically acceptable" refers to those compounds (or salts, prodrugs, tautomers, zwitterionic forms, etc.) which are suitable for use in contact with the tissues of patients without excessive toxicity, irritation, allergic response, immunogenecity, are commensurate with a reasonable benefit/risk ratio, and are effective for their intended use.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenecity, or other problems or complications, commensurate with a reasonable benefit/risk ratio. S*ee,* Remington: The Science and Practice of Pharmacy, 21st Edition; Lippincott Williams & Wilkins: Philadelphia, PA, 2005**;** Handbook of Pharmaceutical Excipients, 5th Edition; Rowe et al., Eds., The Pharmaceutical Press and the American Pharmaceutical Association: 2005**;** and Handbook of Pharmaceutical Additives, 3rd Edition; Ash and Ash Eds., Gower Publishing Company: 2007**;** Pharmaceutical Preformulation and Formulation, Gibson Ed., CRC Press LLC: Boca Raton, FL, 2004).

The terms "active ingredient," "active compound," and "active substance" refer to a compound, which is administered, alone or in combination with one or more pharmaceutically acceptable excipients or carriers, to a subject for treating, preventing, or ameliorating one or more symptoms of a disorder.

The terms "drug," "therapeutic agent," and "chemotherapeutic agent" refer to a compound, or a pharmaceutical composition thereof, which is administered to a subject for treating, preventing, or ameliorating one or more symptoms of a disorder.

The term "release controlling excipient" refers to an excipient whose primary function is to modify the duration or place of release of the active substance from a dosage form as compared with a conventional immediate release dosage form.

The term "nonrelease controlling excipient" refers to an excipient whose primary function do not include modifying the duration or place of release of the active substance from a dosage form as compared with a conventional immediate release dosage form.

The term "prodrug" refers to a compound functional derivative of the compound as disclosed herein and is readily convertible into the parent compound in vivo. Prodrugs are often useful because, in some situations, they may be easier to administer than the parent compound. They may, for instance, be bioavailable by oral administration whereas the parent compound is not. The prodrug may also have enhanced solubility in pharmaceutical compositions over the parent compound. A prodrug may be converted into the parent drug by various mechanisms, including enzymatic processes and metabolic hydrolysis. See Harper, Progress in Drug Research 1962, 4, 221-294; Morozowich et al. in "Design of Biopharmaceutical Properties through Prodrugs and Analogs," Roche Ed., APHA Acad. Pharm. Sci. 1977**;** "Bioreversible Carriers in Drug in Drug Design, Theory and Application," Roche Ed., APHA Acad. Pharm. Sci. 1987**;** "Design of Prodrugs," Bundgaard, Elsevier, 1985**;** Wang et al., Curr. Pharm. Design 1999, 5, 265-287; Pauletti et al., Adv. Drug. Delivery Rev. 1997, 27, 235-256; Mizen et al., Pharm. Biotech. 1998, 11, 345-365; Gaignault et al., Pract. Med. Chem. 1996, 671-696; Asgharnejad in "Transport Processes in Pharmaceutical Systems," Amidon et al., Ed., Marcell Dekker, 185-218, 2000; Balant et al., Eur. J. Drug Metab. Pharmacokinet. 1990, 15, 143-53; Balimane and Sinko, Adv. Drug Delivery Rev. 1999, 39, 183-209; Browne, Clin. Neuropharmacol. 1997, 20, 1-12; Bundgaard, Arch. Pharm. Chem. 1979, 86, 1-39; Bundgaard, Controlled Drug Delivery 1987, 17, 179-96; Bundgaard, Adv. Drug Delivery Rev. 1992, 8, 1-38; Fleisher et al., Adv. Drug Delivery Rev. 1996, 19, 115-130; Fleisher et al., Methods Enzymol. 1985, 112, 360-381; Farquhar et al., J. Pharm. Sci. 1983, 72, 324-325; Freeman et al., J. Chem. Soc., Chem. Commun. 1991, 875-877; Friis and Bundgaard, Eur. J. Pharm. Sci. 1996, 4, 49-59; Gangwar et al., Des. Biopharm. Prop. Prodrugs Analogs, 1977, 409-421; Nathwani and Wood, Drugs 1993, 45, 866-94; Sinhababu and Thakker, Adv. Drug Delivery Rev. 1996, 19, 241-273; Stella et al., Drugs 1985, 29, 455-73; Tan et al., Adv. Drug Delivery Rev. 1999, 39, 117-151; Taylor, Adv. Drug Delivery Rev. 1996, 19, 131-148; Valentino and Borchardt, Drug Discovery Today 1997, 2, 148-155; Wiebe and Knaus, Adv. Drug Delivery Rev. 1999, 39, 63-80; Waller et al., Br. J. Clin. Pharmac. 1989, 28, 497-507.

The compounds disclosed herein can exist as therapeutically acceptable salts. The term "therapeutically acceptable salt," as used herein, represents salts or zwitterionic forms of the compounds disclosed herein which are therapeutically acceptable as defined herein. The salts can be prepared during the final isolation and purification of the compounds or separately by reacting the appropriate compound with a suitable acid or base.Therapeutically acceptable salts include acid and basic addition salts. For a more complete discussion of the preparation and selection of salts, refer to "Handbook of Pharmaceutical Salts, Properties, and Use," Stah and Wermuth, Ed., (Wiley-VCH and VHCA, Zurich, 2002) and Berge et al., J. Pharm. Sci. 1977, 66, 1-19.

Suitable acids for use in the preparation of pharmaceutically acceptable salts include, but are not limited to, acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

Suitable bases for use in the preparation of pharmaceutically acceptable salts, including, but not limited to, inorganic bases, such as magnesium hydroxide, calcium hydroxide, potassium hydroxide, zinc hydroxide, or sodium hydroxide; and organic bases, such as primary, secondary, tertiary, and quaternary, aliphatic and aromatic amines, including L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1*H*-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, *N*-methyl-D-glucamine, 2-amino-2-(hydroxymethyl)-1,3-propanediol, and tromethamine.

While it may be possible for the compounds of the subject invention to be administered as the raw chemical, it is also possible to present them as a pharmaceutical composition. Accordingly, provided herein are pharmaceutical compositions which comprise one or more of certain compounds disclosed herein, or one or more pharmaceutically acceptable salts, prodrugs, or solvates thereof, together with one or more pharmaceutically acceptable carriers thereof and optionally one or more other therapeutic ingredients. Proper formulation is dependent upon the route of administration chosen. Any of the well-known techniques, carriers, and excipients may be used as suitable and as understood in the art; *e.g.,* in Remington's Pharmaceutical Sciences. The pharmaceutical compositions disclosed herein may be manufactured in any manner known in the art, *e.g.,* by means of conventional mixing, dissolving, granulating, dragee-making, levigating, emulsifying, encapsulating, entrapping or compression processes. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see, Remington: The Science and Practice of Pharmacy,* supra; Modified-Release Drug Deliver Technology, Rathbone et al., Eds., Drugs and the Pharmaceutical Science, Marcel Dekker, Inc., New York, NY, 2002; Vol. 126).

The compositions include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The compositions may conveniently be presented in unit dosage form and may be prepared by any of the methods well known in the art of pharmacy. Typically, these methods include the step of bringing into association a compound of the subject invention or a pharmaceutically salt, prodrug, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

Formulations of the compounds disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

The compounds may be formulated for parenteral administration by injection, e.g., by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds may also be formulated in rectal compositions such as suppositories or retention enemas, *e.g*., containing conventional suppository bases such as cocoa butter, polyethylene glycol, or other glycerides.

Certain compounds disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semiliquid preparations suitable for penetration through the skin to the site of inflammation such as gels, liniments, lotions, creams, ointments or pastes, and drops suitable for administration to the eye, ear or nose.

For administration by inhalation, compounds may be delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Preferred unit dosage formulations are those containing an effective dose, as herein below recited, or an appropriate fraction thereof, of the active ingredient.

Compounds may be administered orally or via injection at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

The amount of active ingredient that may be combined with the carrier materials to produce a single dosage form will vary depending upon the host treated and the particular mode of administration.

In certain embodiments, the compounds disclosed herein may be formulated or administered using any of formulations and methods disclosed in U.S. Patent Application Serial No. 14/030,322, filed September 18, 2013, which is hereby incorporated by reference in its entirety.

The compounds can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the disorder being treated. Also, the route of administration may vary depending on the disorder and its severity.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disorder.

In the case wherein the patient's status does improve, upon the doctor's discretion the administration of the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disorder is retained. Patients can, however, require intermittent treatment on a long-term basis upon any recurrence of symptoms.

Disclosed herein are methods of treating a VMAT2-mediated disorder comprising administering to a subject having or suspected of having such a disorder, a therapeutically effective amount of a compound as disclosed herein or a pharmaceutically acceptable salt, solvate, or prodrug thereof.

VMAT2-mediated disorders, include, but are not limited to, Parkinson's disease levodopa-induced dyskinesia or levodopa-induced dyskinesia.

In certain embodiments, a method of treating a VMAT2-mediated disorder comprises administering to the subject a therapeutically effective amount of a compound as disclosed herein, or a pharmaceutically acceptable salt, solvate, or prodrug thereof, so as to affect: (1) decreased inter-individual variation in plasma levels of the compound or a metabolite thereof; (2) increased average plasma levels of the compound or decreased average plasma levels of at least one metabolite of the compound per dosage unit; (3) decreased inhibition of, and/or metabolism by at least one cytochrome P₄₅₀ or monoamine oxidase isoform in the subject; (4) decreased metabolism via at least one polymorphically-expressed cytochrome P₄₅₀ isoform in the subject; (5) at least one statistically-significantly improved disorder-control and/or disorder-eradication endpoint; (6) an improved clinical effect during the treatment of the disorder, (7) prevention of recurrence, or delay of decline or appearance, of abnormal alimentary or hepatic parameters as the primary clinical benefit, or (8) reduction or elimination of deleterious changes in any diagnostic hepatobiliary function endpoints, as compared to the corresponding non-isotopically enriched compound.

In certain embodiments, inter-individual variation in plasma levels of the compounds as disclosed herein, or metabolites thereof, is decreased; average plasma levels of the compound as disclosed herein are increased; average plasma levels of a metabolite of the compound as disclosed herein are decreased; inhibition of a cytochrome P₄₅₀ or monoamine oxidase isoform by a compound as disclosed herein is decreased; or metabolism of the compound as disclosed herein by at least one polymorphically-expressed cytochrome P₄₅₀ isoform is decreased; by greater than about 5%, greater than about 10%, greater than about 20%, greater than about 30%, greater than about 40%, or by greater than about 50% as compared to the corresponding non-isotopically enriched compound.

Plasma levels of the compound as disclosed herein, or metabolites thereof, may be measured using the methods described by Li et al. Rapid Communications in Mass Spectrometry 2005, 19, 1943-1950; Jindal, et al., Journal of Chromatography, Biomedical Applications 1989, 493(2), 392-7; Schwartz, et al., Biochemical Pharmacology 1966, 15(5), 645-55; Mehvar, et al., Drug Metabolism and Disposition 1987, 15(2), 250-5; Roberts et al., Journal of Chromatography, Biomedical Applications 1981, 226(1), 175-82; and any references cited therein or any modifications made thereof.

Examples of cytochrome P₄₅₀ isoforms in a mammalian subject include, but are not limited to, CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, and CYP51.

Examples of monoamine oxidase isoforms in a mammalian subject include, but are not limited to, MAO_{A}, and MAO_{B}.

The inhibition of the cytochrome P₄₅₀ isoform is measured by the method of Ko et al. (British Journal of Clinical Pharmacology, 2000, 49, 343-351). The inhibition of the MAO_{A} isoform is measured by the method of Weyler et al. (J. Biol Chem. 1985, 260, 13199-13207). The inhibition of the MAO_{B} isoform is measured by the method of Uebelhack et al. (Pharmacopsychiatry, 1998, 31, 187-192).

Examples of polymorphically-expressed cytochrome P₄₅₀ isoforms in a mammalian subject include, but are not limited to, CYP2C8, CYP2C9, CYP2C19, and CYP2D6.

The metabolic activities of liver microsomes, cytochrome P₄₅₀ isoforms, and monoamine oxidase isoforms are measured by the methods described herein.

Examples of improved disorder-control and/or disorder-eradication endpoints, or improved clinical effects include, but are not limited to:
a. improved Unified Parkinson's Disease Rating Scale scores;
b. improved Abnormal Involuntary Movement Scale scores;
c. improved Goetz Dyskinesia Rating Scale scores;
d. improved Unified Dyskinesia Rating Scale scores;
e. improved PDQ-39 Parkinson's Disease Questionnaire scores; and
f. improved Global Primate Dyskinesia Rating Scale scores.

Examples of diagnostic hepatobiliary function endpoints include, but are not limited to, alanine aminotransferase ("ALT"), serum glutamic-pyruvic transaminase ("SGPT"), aspartate aminotransferase ("AST" or "SGOT"), ALT/AST ratios, serum aldolase, alkaline phosphatase ("ALP"), ammonia levels, bilirubin, gamma-glutamyl transpeptidase ("GGTP," "γ-GTP," or "GGT"), leucine aminopeptidase ("LAP"), liver biopsy, liver ultrasonography, liver nuclear scan, 5'-nucleotidase, and blood protein. Hepatobiliary endpoints are compared to the stated normal levels as given in "Diagnostic and Laboratory Test Reference", 4th edition, Mosby, 1999**.** These assays are run by accredited laboratories according to standard protocol.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

### Combination Therapy

The compounds disclosed herein may also be combined or used in combination with other agents useful in the treatment of VMAT2-mediated disorders. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used therefor, simultaneously or sequentially with a compound as disclosed herein. When a compound as disclosed herein is used contemporaneously with one or more other drugs, a pharmaceutical composition containing such other drugs in addition to the compound disclosed herein may be utilized, but is not required.

In certain embodiments, the compounds disclosed herein can be combined with one or more dopamine precursors, including, but not limited to, levodopa.

In certain embodiments, the compounds disclosed herein can be combined with one or more DOPA decarboxylase inhibitors, including, but not limited to, carbidopa.

In certain embodiments, the compounds disclosed herein can be combined with one or more catechol-O-methyl transferase (COMT) inhibitors, including, but not limited to, entacapone and tolcapone.

In certain embodiments, the compounds disclosed herein can be combined with one or more dopamine receptor agonists, including, but not limited to, apomorphine, bromocriptine, ropinirole, and pramipexole.

In certain embodiments, the compounds disclosed herein can be combined with one or more neuroprotective agents, including, but not limited to, selegeline and riluzole.

In certain embodiments, the compounds disclosed herein can be combined with one or more NMDA antagonists, including, but not limited to, amantidine.

In certain embodiments, the compounds disclosed herein can be combined with one or more anti-psychotics, including, but not limited to, chlorpromazine, levomepromazine, promazine, acepromazine, triflupromazine, cyamemazine, chlorproethazine, dixyrazine, fluphenazine, perphenazine, prochlorperazine, thiopropazate, trifluoperazine, acetophenazine, thioproperazine, butaperazine, perazine, periciazine, thioridazine, mesoridazine, pipotiazine, haloperidol, trifluperidol, melperone, moperone, pipamperone, bromperidol, benperidol, droperidol, fluanisone, oxypertine, molindone, sertindole, ziprasidone, flupentixol, clopenthixol, chlorprothixene, thiothixene, zuclopenthixol, fluspirilene, pimozide, penfluridol, loxapine, clozapine, olanzapine, quetiapine, tetrabenazine, sulpiride, sultopride, tiapride, remoxipride, amisulpride, veralipride, levosulpiride, lithium, prothipendyl, risperidone, clotiapine, mosapramine, zotepine, pripiprazole, and paliperidone.

In certain embodiments, the compounds disclosed herein can be combined with one or more benzodiazepines ("minor tranquilizers"), including, but not limited to alprazolam, adinazolam, bromazepam, camazepam, clobazam, clonazepam, clotiazepam, cloxazolam, diazepam, ethyl loflazepate, estizolam, fludiazepam, flunitrazepam, halazepam, ketazolam, lorazepam, medazepam, dazolam, nitrazepam, nordazepam, oxazepam, potassium clorazepate, pinazepam, prazepam, tofisopam, triazolam, temazepam, and chlordiazepoxide.

In certain embodiments, the compounds disclosed herein can be combined with olanzapine or pimozide.

The compounds disclosed herein can also be administered in combination with other classes of compounds, including, but not limited to, anti-retroviral agents; CYP3A inhibitors; CYP3A inducers; protease inhibitors; adrenergic agonists; anti-cholinergics; mast cell stabilizers; xanthines; leukotriene antagonists; glucocorticoids treatments; local or general anesthetics; non-steroidal anti-inflammatory agents (NSAIDs), such as naproxen; antibacterial agents, such as amoxicillin; cholesteryl ester transfer protein (CETP) inhibitors, such as anacetrapib; anti-fungal agents, such as isoconazole; sepsis treatments, such as drotrecogin-α; steroidals, such as hydrocortisone; local or general anesthetics, such as ketamine; norepinephrine reuptake inhibitors (NRIs) such as atomoxetine; dopamine reuptake inhibitors (DARIs), such as methylphenidate; serotonin-norepinephrine reuptake inhibitors (SNRIs), such as milnacipran; sedatives, such as diazepham; norepinephrine-dopamine reuptake inhibitor (NDRIs), such as bupropion; serotonin-norepinephrine-dopamine-reuptake-inhibitors (SNDRIs), such as venlafaxine; monoamine oxidase inhibitors, such as selegiline; hypothalamic phospholipids; endothelin converting enzyme (ECE) inhibitors, such as phosphoramidon; opioids, such as tramadol; thromboxane receptor antagonists, such as ifetroban; potassium channel openers; thrombin inhibitors, such as hirudin; hypothalamic phospholipids; growth factor inhibitors, such as modulators of PDGF activity; platelet activating factor (PAF) antagonists; anti-platelet agents, such as GPIIb/IIIa blockers (e.g., abdximab, eptifibatide, and tirofiban), P2Y(AC) antagonists (e.g., clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, or atavastatin or visastatin); squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-muscarinic agents; beta-muscarinic agents, such as carvedilol and metoprolol; antiarrhythmic agents; diuretics, such as chlorothlazide, hydrochiorothiazide, flumethiazide, hydroflumethiazide, bendroflumethiazide, methylchlorothiazide, trichioromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prourokinase, and anisoylated plasminogen streptokinase activator complex (APSAC); anti-diabetic agents, such as biguanides (e.g. metformin), glucosidase inhibitors (e.g., acarbose), insulins, meglitinides (e.g., repaglinide), sulfonylureas (e.g., glimepiride, glyburide, and glipizide), thiozolidinediones (e.g. troglitazone, rosiglitazone and pioglitazone), and PPAR-gamma agonists; mineralocorticoid receptor antagonists, such as spironolactone and eplerenone; growth hormone secretagogues; aP2 inhibitors; phosphodiesterase inhibitors, such as PDE III inhibitors (e.g., cilostazol) and PDE V inhibitors (e.g., sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

Thus, in another aspect, certain embodiments provide methods for treating VMAT2-mediated disorders in a subject in need of such treatment comprising administering to said subject an amount of a compound disclosed herein effective to reduce or prevent said disorder in the subject, in combination with at least one additional agent for the treatment of said disorder. In a related aspect, certain embodiments provide therapeutic compositions comprising at least one compound disclosed herein in combination with one or more additional agents for the treatment of VMAT2-mediated disorders.

### General Synthetic Methods for Preparing Compounds

Isotopic hydrogen can be introduced into a compound as disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. Exchange techniques, on the other hand, may yield lower tritium or deuterium incorporation, often with the isotope being distributed over many sites on the molecule.

The compounds as disclosed herein can be prepared by methods known to one of skill in the art and routine modifications thereof, and/or following procedures similar to those described in the Example section herein and routine modifications thereof, and/or procedures found in WO 2005077946; WO 2008/058261; EP 1716145; Lee et al., J. Med. Chem., 1996, (39), 191-196; Kilbourn et al., Chirality, 1997, (9), 59-62; Boldt et al., Synth. Commun., 2009, (39), 3574-3585; Rishel et al., J. Org. Chem., 2009, (74), 4001-4004; DaSilva et al., Appl. Radiat. Isot., 1993, 44(4), 673-676; Popp et al., J. Pharm. Sci., 1978, 67(6), 871-873; Ivanov et al., Heterocycles 2001, 55(8), 1569-1572; US 2,830,993; US 3,045,021; WO 2007130365; WO 2008058261, which are hereby incorporated in their entirety, and references cited therein and routine modifications thereof. Compounds as disclosed herein can also be prepared as shown in any of the following schemes and routine modifications thereof.

The following schemes can be used to practice the present invention. Any position shown as hydrogen may optionally be replaced with deuterium.

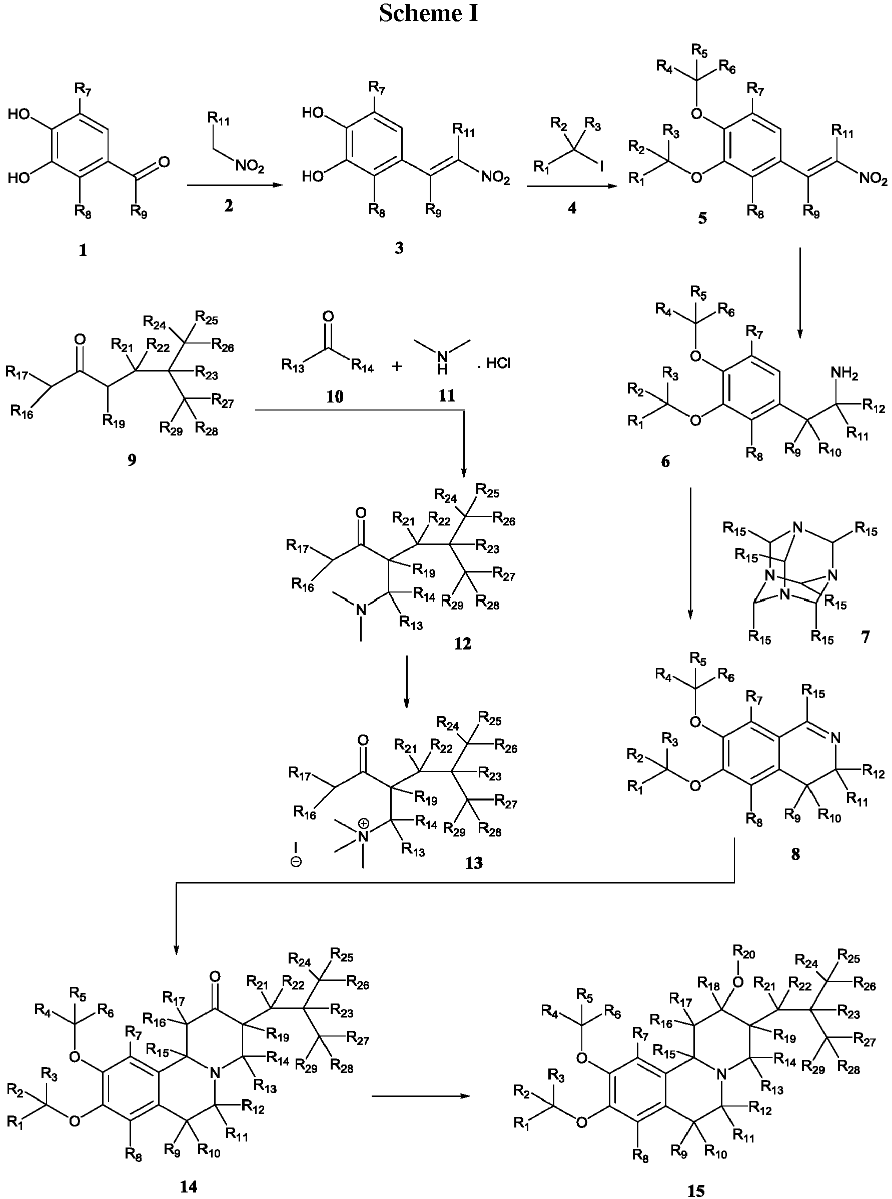

Compound **1** is reacted with compound **2** in an appropriate solvent, such as nitromethane, in the presence of an appropriate acid, such as ammonium acetate, at an elevated temperature to give compound **3.** Compound **3** is reacted with compound **4** in the presence of an appropriate base, such as potassium carbonate, in an appropriate solvent, such as *N*,*N*-dimethylformamide, at an elevated temperature to afford compound **5.** Compound **5** is reacted with an appropriate reducing reagent, such as lithium aluminum hydride, in an appropriate solvent, such as tetrahyrdofuran, at an elevated temperature to give compound **6.** Compound **6** is reacted with compound **7** in the presence of an appropriate acid, such as trifluoroacetic acid, in an appropriate solvent, such as acetic acid, at an elevated temperature to give compound **8.** Compound **9** is reacted with compound **10** and compound **11,** in an appropriate solvent, such as methanol, at an elevated temperature to afford compound **12.** Compound **12** is reacted with an appropriate methylating agent, such as methyl iodide, in an appropriate solvent, such as ethyl acetate, to give compound **13.** Compound **8** is reacted with compound **13** in an appropriate solvent, such as ethanol, at an elevated temperature to give compound **14.** Compound **14** is reacted with an appropriate reducing agent, such as sodium borohydride, in an appropriate solvent, such as methanol, to give compound **15** of Formula I.

Deuterium can be incorporated to different positions synthetically, according to the synthetic procedures as shown in Scheme I, by using appropriate deuterated intermediates. For example, to introduce deuterium at one or more positions of R₁-R₆, compound **4** with the corresponding deuterium substitutions can be used. To introduce deuterium at one or more positions of R₇-R₉, compound **1** with the corresponding deuterium substitutions can be used. To introduce deuterium at one or more positions of R₁₀ and R₁₂, lithium aluminum deuteride can be used. To introduce deuterium at R₁₁, compound **2** with the corresponding deuterium substitution can be used. To introduce deuterium at one or more positions of R₁₃-R₁₄, compound **10** with the corresponding deuterium substitutions can be used. To introduce deuterium at R₁₅, compound **7** with the corresponding deuterium substitution can be used. To introduce deuterium at one or more positions of R₁₆-R₁₇, R₁₉, and R₂₁-R₂₉, compound **9** with the corresponding deuterium substitutions can be used. To indroduce deuterium at R18, sodium borodeuteride can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

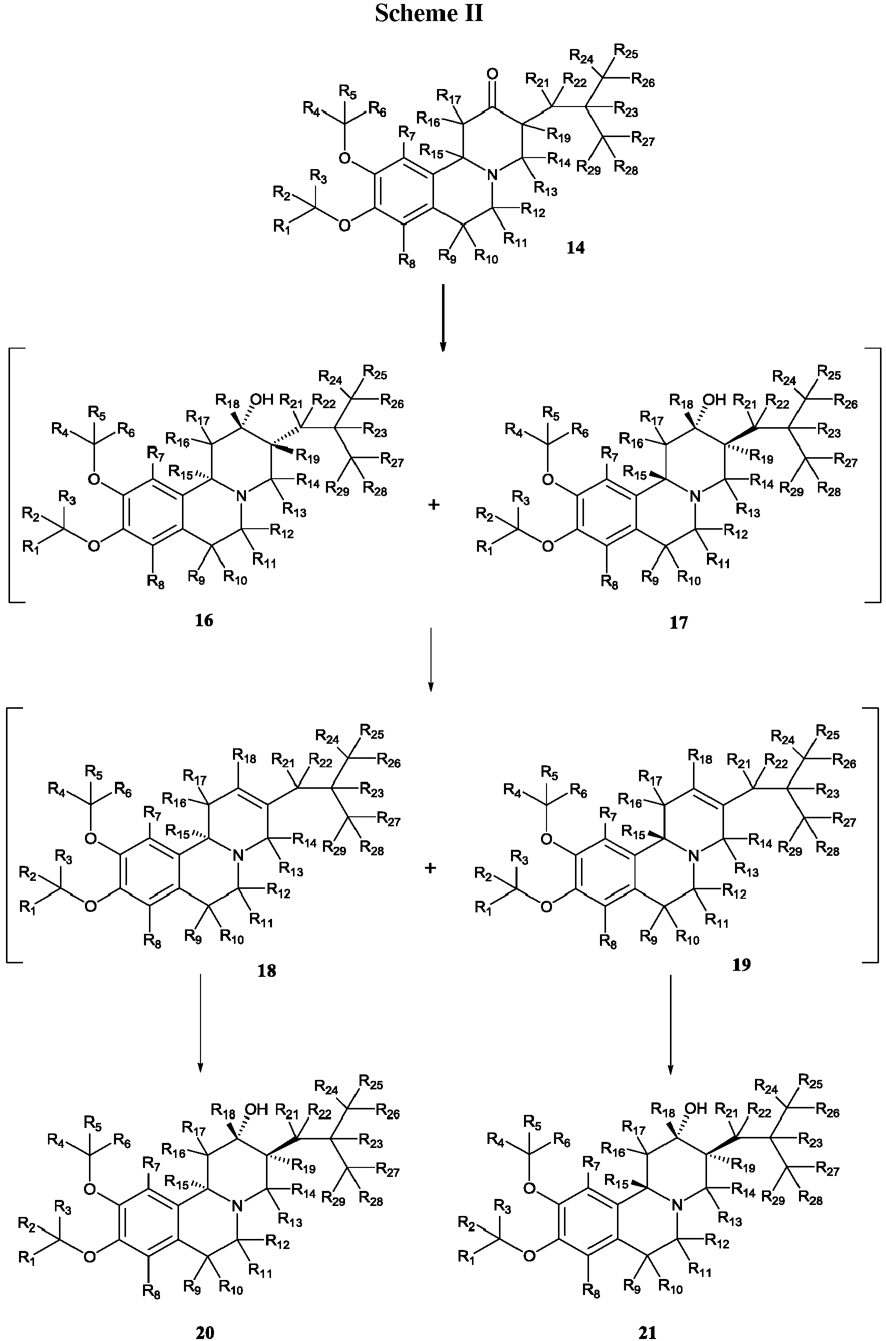

Compound **14** is reacted with an appropriate reducing agent, such as lithium tri-sec-butyl borohydride, in an appropriate solvent, such as ethanol, to give a mixture of compounds **16** and **17** of Formula II. Compounds **16** and **17** are reacted with an appropriate dehydrating reagent, such as phosphorous pentachloride, in an appropriate solvent, such as dichloromethane to afford a mixture of compounds **18** and **19.** Compounds **18** and **19** are reacted with an appropriate hydroborating reagent, such as borane-tetrahydrofuran complex, in an appropriate solvent, such as tetrahyrdofuran, then oxidized with a mixture of sodium hydroxide and hydrogen peroxide, to give compounds **20** and **21** of Formula II. Mixtures of compounds **16** and **17** or **20** and **21** can be separated by chiral preparative chromatography of through the preparation of Mosher's esters (wherein the mixture is treated with R-(+)-3,3,3-trifluoro-2-methoxy-2-phenylpropanoic acid, an appropriate chlorinating agent, such as oxalyl chloride, and an appropriate base, such as 4-dimethylaminopyridine, in an appropriate solvent, such as dichloromethane, to give an epimeric mixture of R-(+)-3,3,3-trifluoro-2-methoxy-2-phenylpropanoate esters), which can be isolated via chromatography and then converted to the desired alcohol via hydrolysis (the Mosher's esters are treated with an appropriate base, such as sodium hydroxide, in an appropriate solvent, such as methanol, to give the desired compounds of Formula II).

Deuterium can be incorporated to different positions synthetically, according to the synthetic procedures as shown in Scheme II, by using appropriate deuterated intermediates. For example, to introduce deuterium at one or more positions of R₁-R₁₇ and R₂₁-R₂₉, compound **14** with the corresponding deuterium substitutions can be used. To introduce deuterium at R₁₈, lithium tri-sec-butyl borodeuteride can be used. To introduce deuterium at R₁₉, trideuteroborane can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

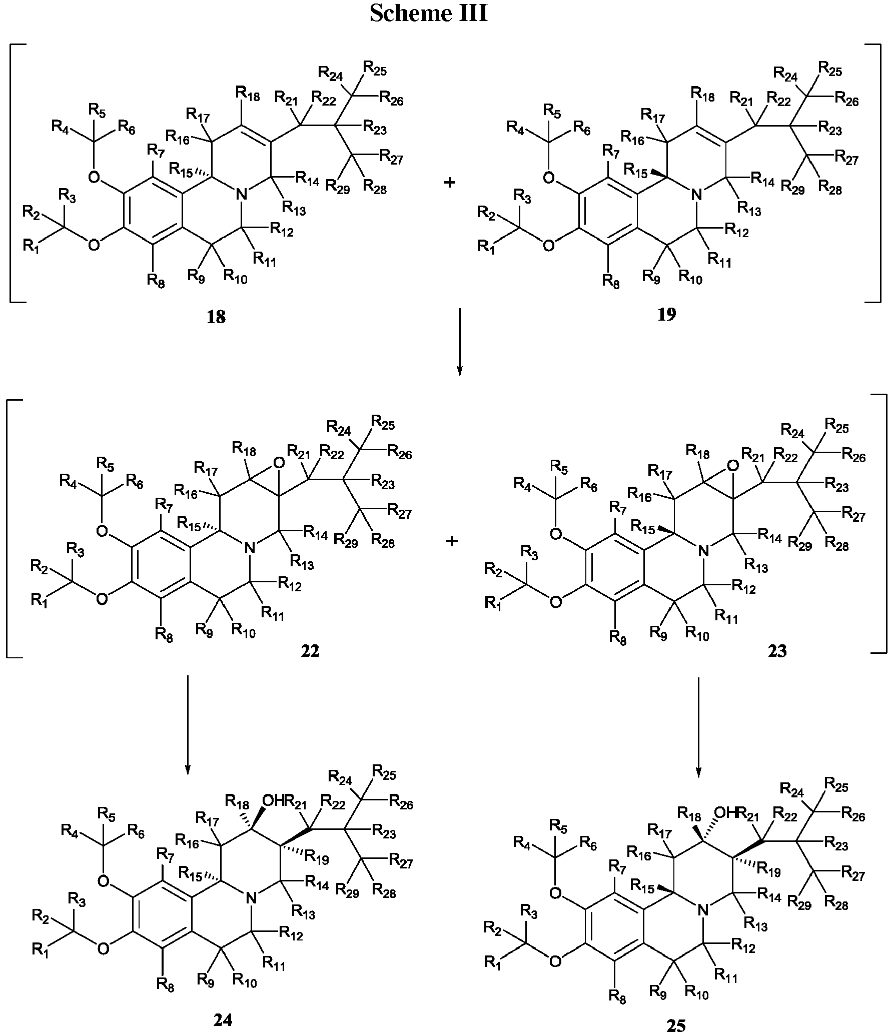

Compounds **18** and **19** (prepared as shown in Scheme II) are reacted with an appropriate peroxidizing agent, such as m-chloroperbenzoic acid, in the presence of an appropriate acid, such as perchloric acid, in an appropriate solvent, such as methanol, to give compounds **22** and **23.** Compounds **22** and **23** are reacted with an appropriate reducing agent, such as borane-tetrahydrofuran complex, in an appropriate solvent, such as tetrahyrdofuran, then hydrolyzed with a mixture of sodium hydroxide and hydrogen peroxide, to give compounds **24** and **25** of Formula II. Mixtures of compounds **24** and **25** can be separated by chiral preparative chromatography of through the preparation of Mosher's esters (wherein the mixture is treated with R-(+)-3,3,3-trifluoro-2-methoxy-2-phenylpropanoic acid, an appropriate chlorinating agent, such as oxalyl chloride, and an appropriate base, such as 4-dimethylaminopyridine, in an appropriate solvent, such as dichloromethane, to give an epimeric mixture of R-(+)-3,3,3-trifluoro-2-methoxy-2-phenylpropanoate esters), which can be isolated via chromatography and then converted to the desired alcohol via hydrolysis (the Mosher's esters are treated with an appropriate base, such as sodium hydroxide, in an appropriate solvent, such as methanol, to give the desired compounds of Formula II).

Deuterium can be incorporated to different positions synthetically, according to the synthetic procedures as shown in Scheme III, by using appropriate deuterated intermediates. For example, to introduce deuterium at one or more positions of R₁-R₁₈ and R₂₁-R₂₉, compounds **18** and **19** with the corresponding deuterium substitutions can be used. To introduce deuterium at R₁₉, trideuteroborane can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

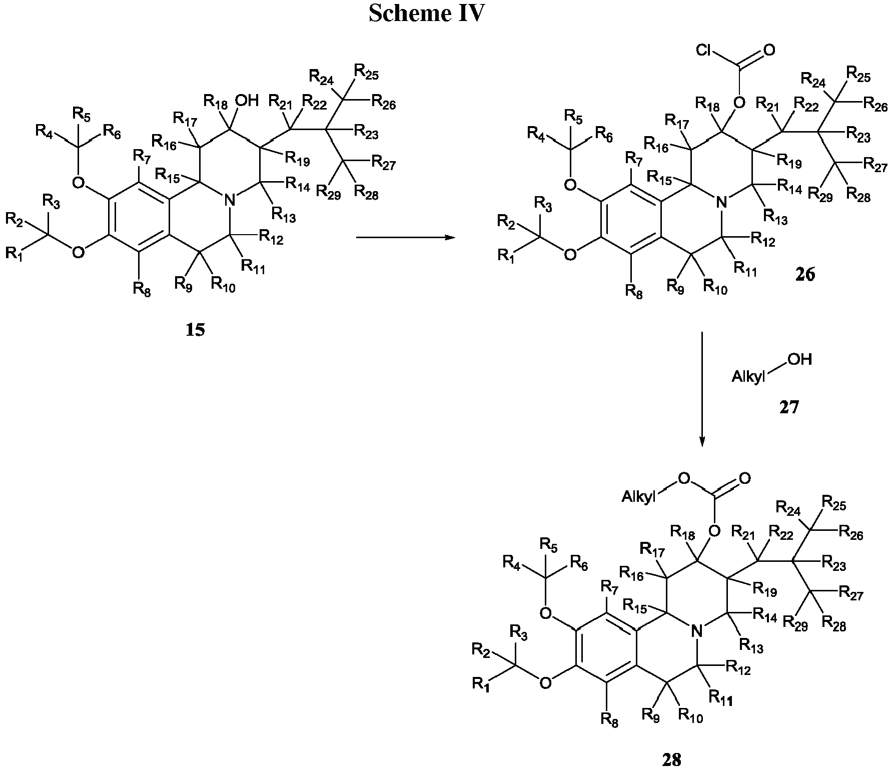

Compound **15** is reacted with an appropriate phosgene equivalent, such as triphosgene, in an appropriate solvent, such as dichloromethane, to give compound **26.** Compound **26** is reacted with an appropriate alcohol, such as compound **27,** in the presence of an appropriate base, such as 4-dimethylaminopyridine, to give compound **28** of Formula II (where R₂₂ is -C(O))-alkyl).

Deuterium can be incorporated to different positions synthetically, according to the synthetic procedures as shown in Scheme IV, by using appropriate deuterated intermediates. For example, to introduce deuterium at one or more positions of R₁-R₁₉ and R₂₁-R₂₉, compound **16** with the corresponding deuterium substitutions can be used. To introduce deuterium at R₂₀, compound **27** with the corresponding deuterium substitutions can be used.

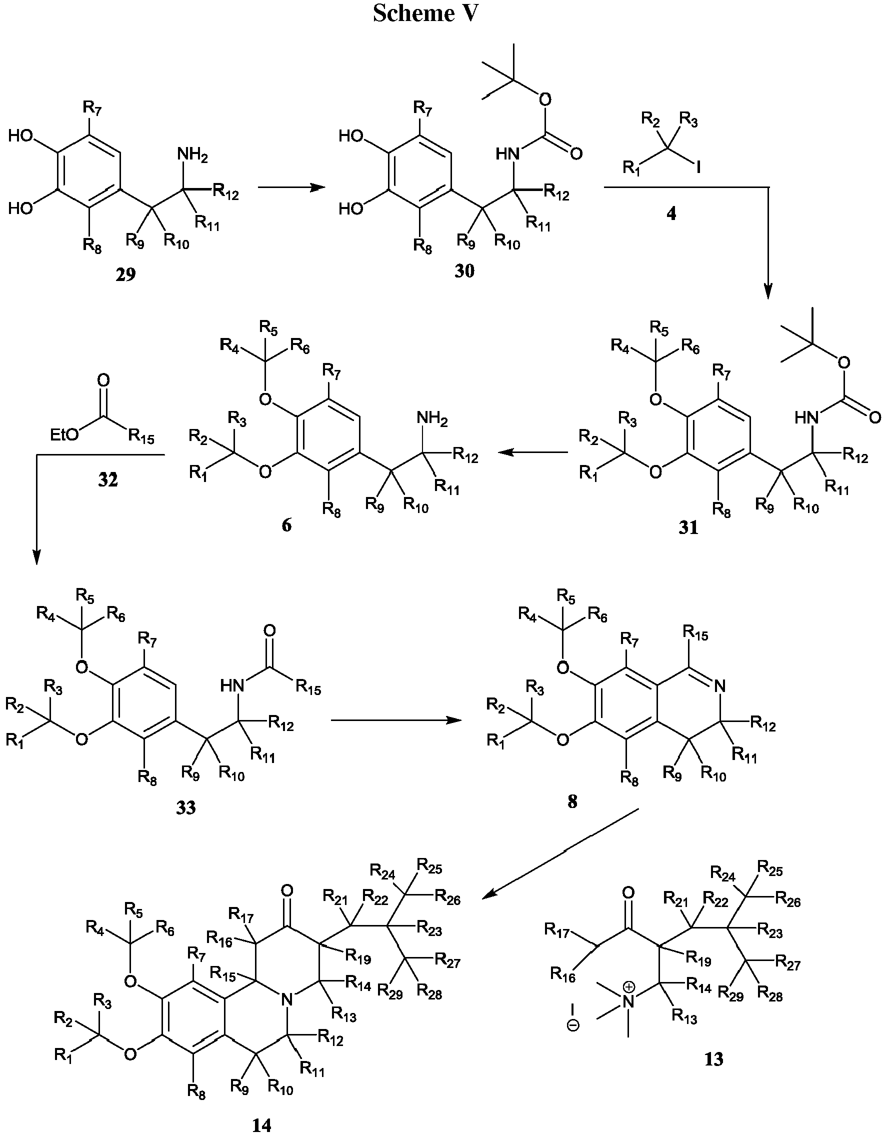

Compound **29** is reacted with an appropriate protecting agent, such as di-tert-butyl dicarbonate, in an appropriate solvent, such as a mixture of tetrathydrofuran and water, in the presence of an appropriate base, such as sodium carbonate, to give compound **30.** Compound **30** is reacted with compound **4** in the presence of an appropriate base, such as potassium carbonate, in the presence of an appropriate catalyst, such as 18-crown-6, in an appropriate solvent, such as acetone, to afford compound **31.** Compound **31** is reacted with an appropriate deprotecting agent, such as hydrogen chloride, in an appropriate solvent, such as ethyl acetate, to give compound **6.** Compound **6** is reacted with compound **32** at an elevated temperature to give compound **33.** Compound **33** is reacted with an appropriate dehydrating agent, such as phosphorous oxychloride, at an elevated temperature to afford compound **8.** Compound **8** is reacted with compound **13** in an appropriate solvent, such as methanol, at an elevated temperature to give compound **14.**

Deuterium can be incorporated to different positions synthetically, according to the synthetic procedures as shown in Scheme V, by using appropriate deuterated intermediates. For example, to introduce deuterium at one or more positions of R₁-R₆, compound **4** with the corresponding deuterium substitutions can be used. To introduce deuterium at one or more positions of R₇-R₁₂, compound **29** with the corresponding deuterium substitutions can be used. To introduce deuterium at R₁₅, compound **32** with the corresponding deuterium substitution can be used. To introduce deuterium at one or more positions of R₁₃-R₁₄, R₁₆-R₁₇, R₁₉, and R₂₁-R₂₉, compound **13** with the corresponding deuterium substitutions can be used.

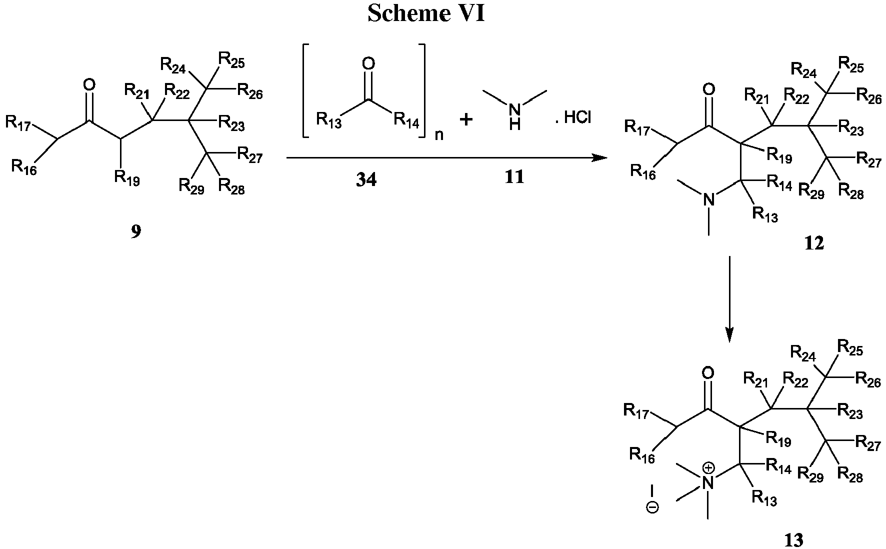

Compound **9** is reacted with compound **11** and compound **34** (paraformaldehyde and/or formaldehyde) in an appropriate solvent, such as ethanol, in the presence of an appropriate acid, such as hydrochloric acid, at an elevated temperature to give compound **12.** Compound **12** is reacted with an appropriate methylating agent, such as methyl iodide, in an appropriate solvent, such as ethyl acetate, to give compound **13.** Compound **8** is reacted with compound **13** in an appropriate solvent, such as dichloromethane, to give compound **13.**

Deuterium can be incorporated to different positions synthetically, according to the synthetic procedures as shown in Scheme VI, by using appropriate deuterated intermediates. For example, to introduce deuterium at one or more positions of R₁₃-R₁₄, compound **10** with the corresponding deuterium substitutions can be used. To introduce deuterium at one or more positions of R₁₆-R₁₇, R₁₉, and R₂₁-R₂₉, compound **9** with the corresponding deuterium substitutions can be used.

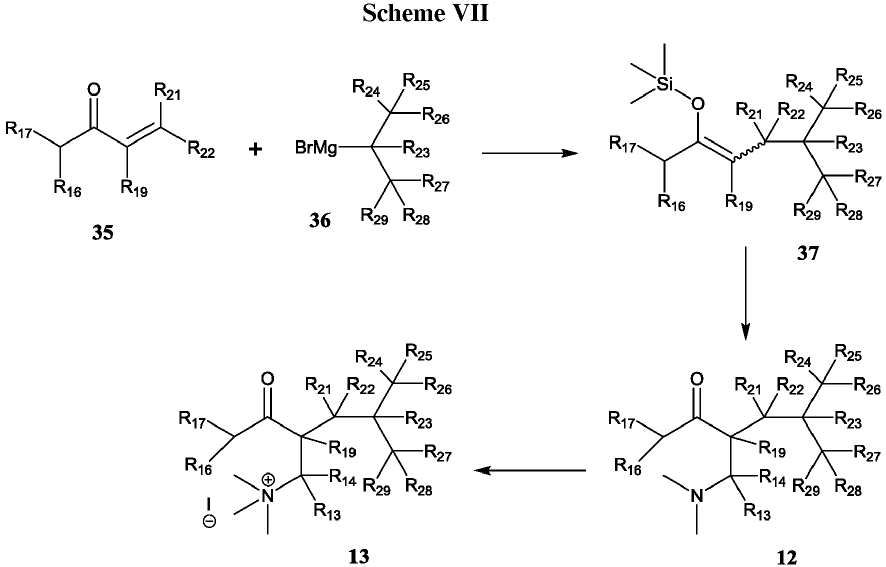

Compound **35** is reacted with compound **36** in an appropriate solvent, such as tetrahydrofuran, in the presence of an appropriate catalyst, such as cuprous iodide, and an appropriate co-solvent, such as hexamethylphosphorous triamide, then reacted with an appropriate protecting agent, such as trimethylsilyl chloride, and an appropriate base, such as triethylamine, to give compound **37.** Compound **37** is reacted with an appropriate mannich base, such as *N*-methyl-*N-*methylenemethanaminium iodide, in an appropriate solvent, such as acetonitrile, to afford compound **12.** Compound **12** is reacted with an appropriate methylating agent, such as methyl iodide, in an appropriate solvent, such as diethyl ether, to give compound **13.**

Deuterium can be incorporated to different positions synthetically, according to the synthetic procedures as shown in Scheme VII, by using appropriate deuterated intermediates. For example, to introduce deuterium at one or more positions of R₁₆-R₁₇, R₁₉, and R₂₁-R₂₂, compound **35** with the corresponding deuterium substitutions can be used. To introduce deuterium at one or more positions of R₂₃-R₂₉, compound **36** with the corresponding deuterium substitutions can be used.

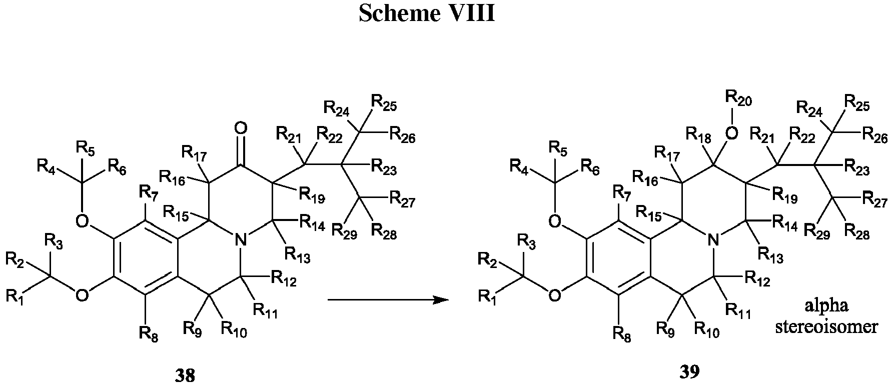

Compound **38** is reacted with an appropriate reducing agent, such as sodium borohydride, in an appropriate solvent, such as ethanol, to give compound **39** of Formula II having predominantly (∼4:1) alpha stereochemistry. The alpha stereoisomer can be further enriched by recrystalization from an appropriate solvent, such as ethanol.

Deuterium can be incorporated to different positions synthetically, according to the synthetic procedures as shown in Scheme I, by using appropriate deuterated intermediates. For example, to introduce deuterium at one or more positions of R₁-R₁₇, R₉₉, and R₂₁-R₂₉, compound **38** with the corresponding deuterium substitutions can be used. To indroduce deuterium at R₁₈, sodium borodeuteride can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

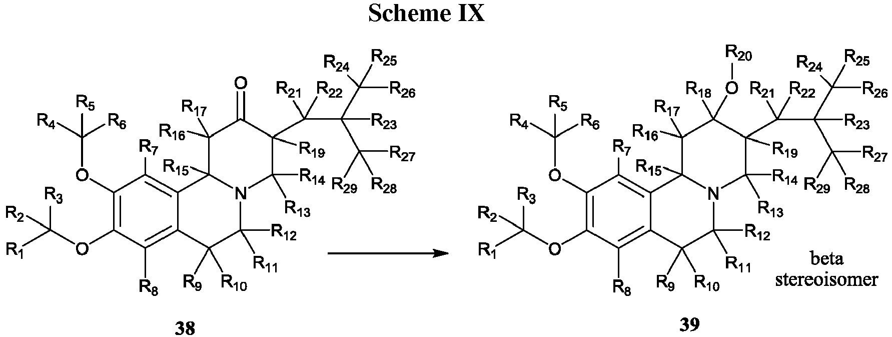

Compound **38** is reacted with an appropriate reducing agent, such as potassium tri-sec-butyl borohydride (K-selectride), in an appropriate solvent, such as tetrahydrofuran, to give compound **40** of Formula I having beta stereochemistry.

Deuterium can be incorporated to different positions synthetically, according to the synthetic procedures as shown in Scheme I, by using appropriate deuterated intermediates. For example, to introduce deuterium at one or more positions of R₁-R₁₇, R₉₉, and R₂₁-R₂₉, compound **38** with the corresponding deuterium substitutions can be used. To indroduce deuterium at R₁₈, potassium tri-sec-butyl borodeuteride can be used.

Deuterium can be incorporated to various positions having an exchangeable proton, such as the hydroxyl O-H, via proton-deuterium equilibrium exchange. For example, to introduce deuterium at R₂₀, this proton may be replaced with deuterium selectively or non-selectively through a proton-deuterium exchange method known in the art.

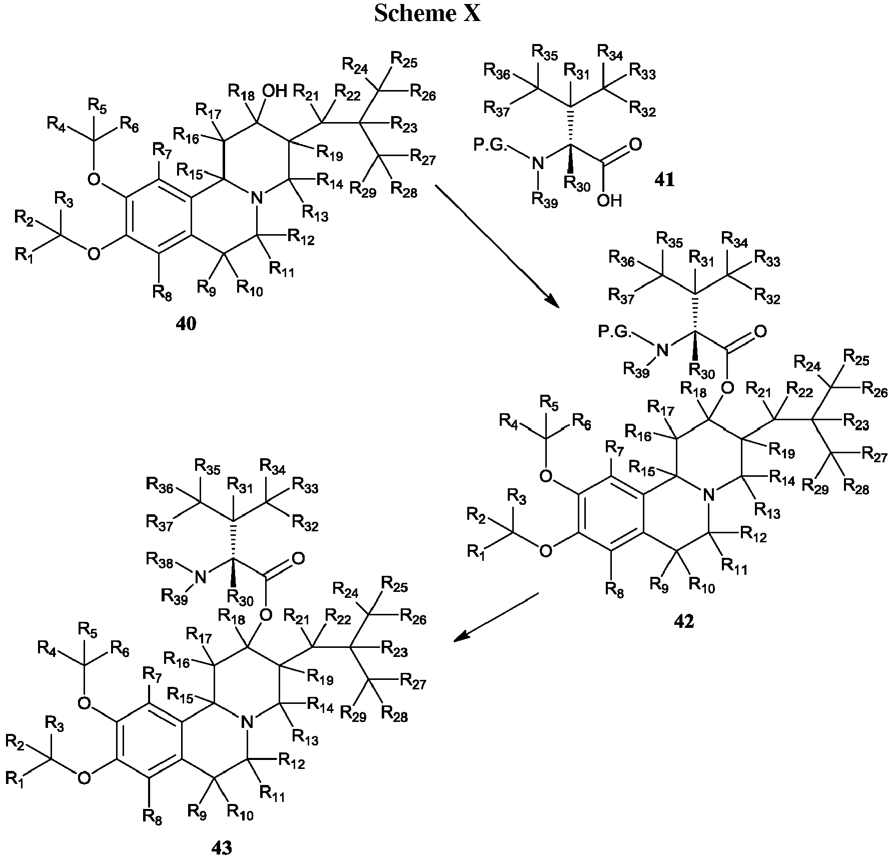

Compound **40** is reacted with compound **41** (wherein P.G. is an appropriate protecting group, such as carboxybenzoyl) in the presence of an appropriate coupling agent, such as dicyclohexylcarbodiimide (DCC), an appropiate catalyst, such as 4-dimethylaminopyridine (DMAP), in an appropriate solvent, such as dichloromethane, to give compound **42.** Compound **42** is reacted with an appropriate deprotecting agent, such as a combination of hydrogen and an appropriate catalyst, such as palladium on carbon, in an appropriate solvent, such as methanol, to give compound **43** of Formula II.

Deuterium can be incorporated to different positions synthetically, according to the synthetic procedures as shown in Scheme I, by using appropriate deuterated intermediates. For example, to introduce deuterium at one or more positions of R₁-R₁₉ and R₂₁-R₂₉, compound **40** with the corresponding deuterium substitutions can be used. To indroduce deuterium at one or more positions of R₃₀-R₃₇, compound **41** with the corresponding deuterium substitutions can be used.

Deuterium can be incorporated to various positions having an exchangeable proton, such as the hydroxyl O-H or amine N-Hs, via proton-deuterium equilibrium exchange. For example, to introduce deuterium at R₂₀ and R₃₈-R₃₉, these protons may be replaced with deuterium selectively or non-selectively through a proton-deuterium exchange method known in the art.

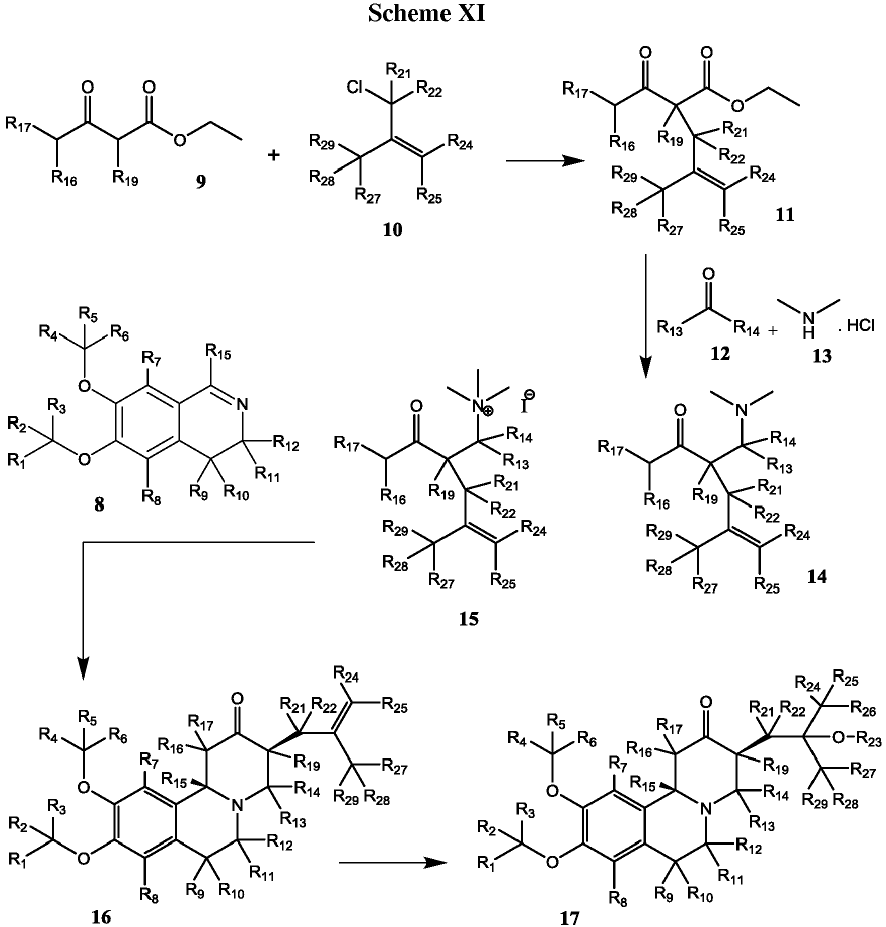

Compound **44** is reacted with compound **45** in the presence of an appropriate base, such as potassium carbonate, in the presence of an appropriate phase transfer catalyst, such as a combination of potassium iodide and tetrabutylammonium bromide, in an appropriate solvent, such as *N,N-*dimethylformamide, at an elevated temperature to afford compound **46.** Compound **46** is reacted with an appropriate base, such as potassium hydroxide, then reacted with compound **47** and compound **48** in the presence of an appropriate acid, such as hydrochloric acid, and an appropriate phase transfer catalyst, such as tetrabutylammonium bromide, in an appropriate solvent, such as water, to afford compound **49.** Compound **49** is reacted with an appropriate methylating agent, such as methyl iodide, in an appropriate solvent, such as methyl tert-butyl ether, to give compound **50.** Compound **8** is reacted with compound **50** in an appropriate solvent, such as a mixture of methanol and water, at an elevated temperature to give compound **51.** Compound **51** is reacted with an appropriate acid, such as sulfuric acid, in an appropriate solvent, such as water, to give compound **52** of Formula III.

Deuterium can be incorporated to different positions synthetically, according to the synthetic procedures as shown in Scheme I, by using appropriate deuterated intermediates. For example, to introduce deuterium at one or more positions of R₁-R₁₂ and R₁₅, compound **8** with the corresponding deuterium substitutions can be used. To introduce deuterium at one or more positions of R₁₃-R₁₄, compound **47** with the corresponding deuterium substitutions can be used. To introduce deuterium at one or more positions of R₁₆-R₁₇ and R₁₉, compound **44** with the corresponding deuterium substitutions can be used. To introduce deuterium at one or more positions of R₂₁-R₂₂, R₂₄-R₂₅, and R₂₇-R₂₉, compound **45** with the corresponding deuterium substitutions can be used. To introduce deuterium at one or more positions of R₂₃ and R₂₆, D₂SO₄ and/or D₂O can be used.

Deuterium can be incorporated to various positions having an exchangeable proton, such as the hydroxyl O-H, via proton-deuterium equilibrium exchange. For example, to introduce deuterium at R₂₃, this proton may be replaced with deuterium selectively or non-selectively through a proton-deuterium exchange method known in the art.

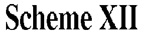

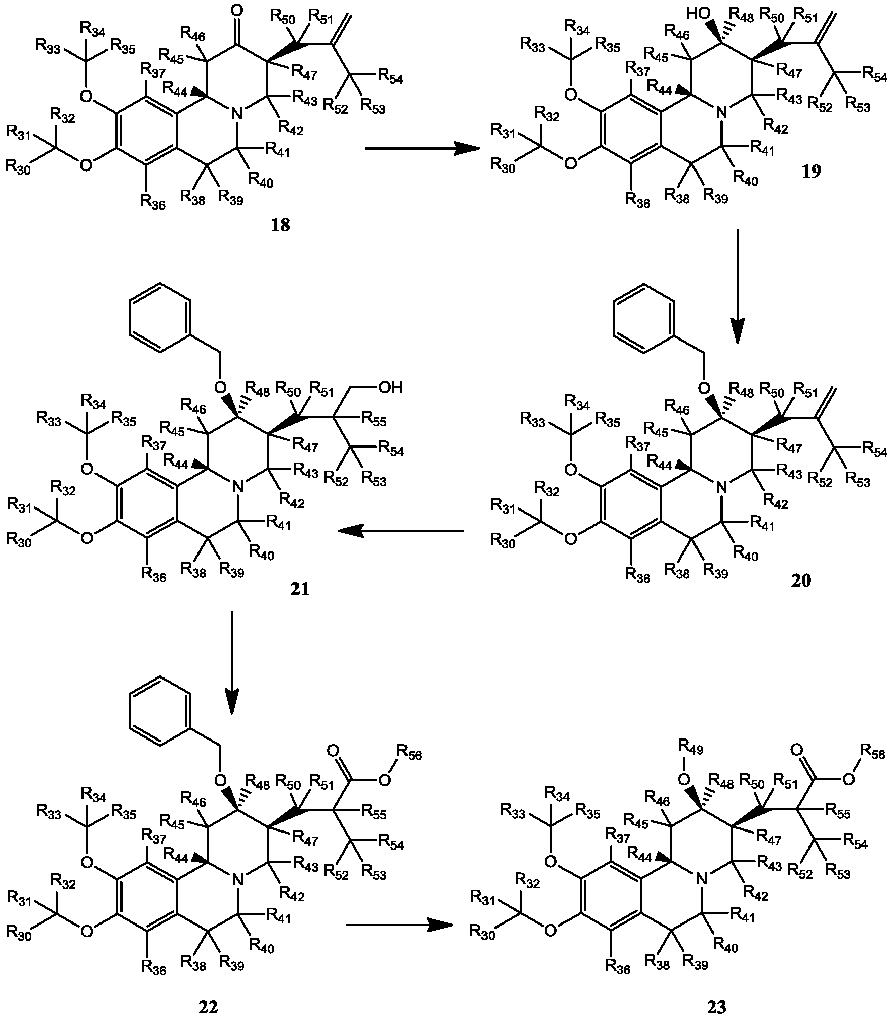

Compound **53** is reacted with an appropriate reducing agent, such as lithium tri-sec-butyl borohydride, in an appropriate solvent, such as tetrahydrofuran, to give compound **54.** Compound **54** is reacted with an appropriate protecting agent, such as benzyl bromide, in the presence of an appropriate base, such as sodium hydride, in an appropriate solvent, such as tetrahydrofuran to give compound **55.** Compound **55** is reacted with an appropriate hydroborating reagent, such as borane-dimethylsulfide complex, in an appropriate solvent, such as tetrahyrdofuran, then reacted with an appropriate base, such as aqueous sodium hydroxide, to give compound **56.** Compound **56** is reacted with an appropriate oxidizing agent, such as Jones reagent (an aqueous solution of chromium trioxide and sulfuric acid), in an appropriate solvent, such as acetone, to give compound **57.** Compound **57** is reacted with an appropriate deprotecting agent, such as a mixute of palladium on carbon and hydrogen gas, in an appropriate solvent, such as methanol, to give compound **58** of Formula IV.

Deuterium can be incorporated to different positions synthetically, according to the synthetic procedures as shown in Scheme II, by using appropriate deuterated intermediates. For example, to introduce deuterium at one or more positions of R₃₀-R₄₇ and R₅₀-R₅₄, compound **53** with the corresponding deuterium substitutions can be used. To introduce deuterium at R₄₈, lithium tri-sec-butyl borodeuteride can be used. To introduce deuterium at R₅₅, trideuteroborane can be used.

Deuterium can be incorporated to various positions having an exchangeable proton, such as the hydroxyl O-H or carboxyl O-H, via proton-deuterium equilibrium exchange. For example, to introduce deuterium at R₄₉ and/or R₅₆, these protons may be replaced with deuterium selectively or non-selectively through a proton-deuterium exchange method known in the art.

The invention is further illustrated by the following examples. All IUPAC names were generated using CambridgeSoft's ChemDraw 10.0.

### EXAMPLE 1

### D₆-(±)-3-Isobutyl-9,10-dimethoxy-3,4,6,7-tetrahydro-1H-pyrido[2,1-a]isoquinolin-2(11bH)-one((±)-Tetrabenazine-d₆)

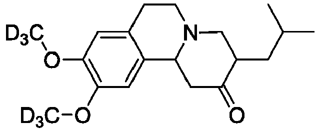

### Step 1

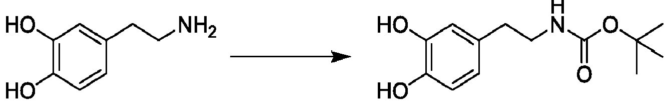

***Tert*-butyl 3,4-dihydroxyphenethylcarbamate**: A solution of dopamine hydrochloride (209 g, 1.11 mol, 1.00 equiv), sodium carbonate (231 g, 2.75 mol, 2.50 equiv) and di-tert-butyl dicarbonate (263 g, 1.21 mol, 1.10) in 2.4 L tetrahydrofuran / water (5:1) was stirred at 20°C for 2.5 h. After the starting material was consumed completedly, the reaction was diluted with ethyl acetate (2 L) and washed with water (2x600 mL). The organic phase was dried over sodium sulfate, filtered and concentrated under reduced pressure until two volumes of solvent was left. The precipitated solid was isolated by filtration and dried under vacuum to give 254 g (91%) of *tert*-butyl 3,4-dihydroxyphenethylcarbamate as white solid. ¹H-NMR (300 MHz, CDCl₃) *δ* 8.72 (s, 1H), 8.62 (s, 1H), 6.79 (m, 1H), 6.62 (m, 1H), 6.51 (m, 1H), 6.40 (m, 1H), 3.03 (m, 2H), 2.50 (m, 2H), 1.37 (s, 1H). LC-MS: *m*/*z* = 254 (MH)⁺.

### Step 2

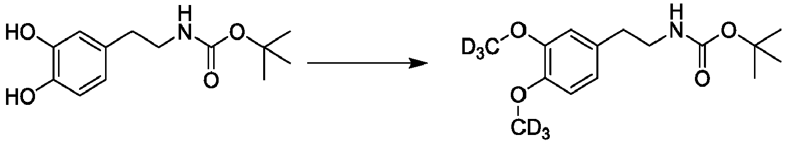

**D₆-*tert*-butyl 3,4-dimethoxyphenethylcarbamate:** A solution of *tert-*butyl 3,4-dihydroxyphenethylcarbamate (127 g, 397 mmol, 1.00 equiv), potassium carbonate (359.3 g, 2.604 mmol, 3.00 equiv) and 18-crown-6 (1,4,7,10,13,16-hexaoxacyclooctadecane) (68.64 g, 0.26 mmol, 0.03 equiv) in acetone (800 mL) was stirred at 38°C. After 30 min., CD₃I (362 g, 2.604 mmol, 3.00 equiv) was added to the reaction, and the mixture was stirred at 38°C for 12 h. Then an additional CD₃I (120 g, 0.868 mmol, 1.00 equiv) was added to the solution and the solution was stirred for 5 h. Then the mixture was cooled to room temperature and the solid was filtered. The filtrate was concentrated under vacuum. The resultant solid was dissolved in H₂O (300 mL) and extracted with EA (3x300 mL), the organic layers was combined and concentrated under vacuum to give 114 g (79%) of d₆-*tert*-butyl 3,4-dimethoxyphenethylcarbamate as white solid. ¹H-NMR (300 MHz, CDCl₃) *δ* 7.39 (m, 5H), 6.82 (m, 1H), 6.73 (m, 2H), 5.12 (s, 1H), 3.45 (m, 2H), 2.77 (m, 2H). LC-MS: *m*/*z* = 288 (MH)⁺.

### Step 3

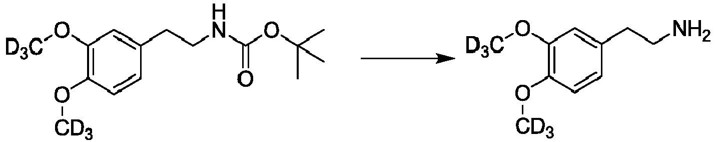

**D₆-2-(3,4-dimethoxyphenyl)ethanamine:** A solution of d₆-*tert*-butyl 3,4-dimethoxyphenethylcarbamate (128 g, 455.26 mmol, 1.00 equiv) in ethyl acetate (1.5 L) was stirred at room temperature. Then HCl gas was introduced into the reaction mixture for 2h. The precipitated solid was isolated by filtration. The solid was dissolved in 300 mL of water. The pH value of the solution was adjusted to 12 with sodium hydroxide (solid). The resulting solution was stirred for 1 h at 5-10°C. The resulting solution was extracted with 6x800 mL of ethyl acetate and the organic layers combined, dried over sodium sulfate, and concentrated under vacuum to give 64 g (78%) of d₆-2-(3,4-dimethoxyphenyl)ethanamine as yellow oil. ¹H-NMR (300 MHz, CDCl₃) *δ* 6.77 (m, 3H), 3.89 (s, 3H), 3.87 (s, 3H), 2.96 (m, 2H), 2.71 (m, 2H), 1.29 (s, 2H). LC-MS: *m*/*z* = 182 (MH)⁺.

### Step 4

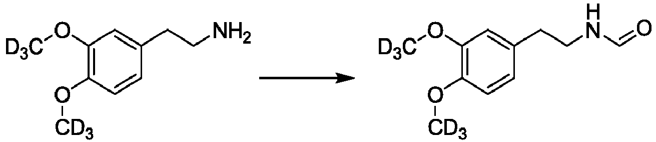

### D₆-N-[2-(3,4-dimethoxy-phenyl)ethyl]formamide:

A solution of d₆-2-(3,4-dimethoxyphenyl)ethanamine (69 g, 368 mmol, 1.00 equiv) in ethyl formate(250 mL) was heated under reflux overnight. The solution was concentrated under vacuum to give 71 g (91%) of d₆-*N*-[2-(3,4-dimethoxyphenyl)ethyl]formamide as yellow solid. The crude solid was used in next step without purification. ¹H-NMR (300 MHz, CDCl₃) *δ* 8.17 (s, 1H), 6.81 (m, 3H), 5.53 (br, 1H).3.59 (m, 2H), 2.81 (t, 2H, *J* = 6.9 Hz). LC-MS: *m*/*z* = 216 (MH)⁺.

### Step 5

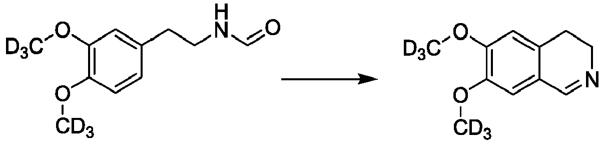

### D₆-6,7-dimethoxy-3,4-dihydroisoquinoline:

A solution of d₆-*N*-[2-(3,4-dimethoxy-phenyl)ethyl]formamide (71 g, 329 mmol, 1.00 equiv) in phosphorus oxychloride (100 mL) was stirred at 105°C for 1 h. Then the solution was concentrated under vacuum to remove phosphorus oxychloride. The residual oil was dissolved in ice / water. The solution was made basic with potassium carbonate with cooling. The basic aqqueous solution was extracted with dichloromethane. The collected organic phase was dried using sodium sulfate and then filtered. The dichloromethane was removed by concentration under vacuum to give an orange oil. Purification by silica gel (ethyl acetate:petroleum ether = 1:1 ∼ ethyl acetate) to give 43 g (66%) of d₆-6,7-dimethoxy-3,4-dihydroisoquinoline as orange solid (yield 66%). ¹H-NMR (300 MHz, CDCl₃) *δ* 8.24 (s, 1H), 6.82 (s, 1H), 6.68 (s, 1H), 3.74 (m, 2H), 2.69 (t, 2H, *J* = 7.2 Hz). LC-MS: *m*/*z* = 198 (MH)⁺.

### Step 6

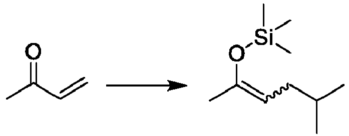

**Trimethyl(5-methylhex-2-en-2-yloxy)silane:** To a cold (-78°C), stirred solution of i-PrMgBr (500 mL of 2 M solution in tetrahydrofuran, 1 mol, 1.00 equiv) in anhydrous tetrahydrofuran (1 L) was added CuI (19.02 g, 0.1 mol, 0.10 equiv) and the resultant mixture was stirred for 15 min at -78°C. Anhydrous hexamethylphosphorous triamide (358.4 g, 2 mmol, 2 equiv) was added and after 20 min, a solution of methyl vinyl ketone (70 g, 0.1 mol, 1.00 equiv), trimethylsilyl chloride (217 g, 0.2 mol, 2.00 equiv), in tetrahydrofuran (200 mL) was added dropwise over 30 min. After the reaction mixture was stirred at -78 °C for 1h, triethylamine (20.2g, 200 mmol, 2.00 equiv) was added and the resulting mixture stirred for 10 min at 0 °C. To this was added *tert*-butyl methyl ether (2 L), and the solution was washed with 5% ammonia solution (6x300 mL). Then the organic phase was dried over sodium sulfate and concentrated under vacuum at 25°C to give 155 g crude product as yellow liquid. The liquid was purified by distilling (64-68°C/40 mmHg) to provide 118 g (63.3%) of trimethyl(5-methylhex-2-en-2-yloxy)silane (E:Z = 56 : 44) as a colorless oil. ¹H-NMR (300 MHz, *d₆*-DMSO) *δ* 4.58 (m, 0.56H), 4.43 (m, 0.44H), 1.73 (s, 1.69H), 1.66 (s, 1.32H), 1.53 (m, 1H), 0.84 (m, 6 H), 0.15(m, 9H).

### Step 7

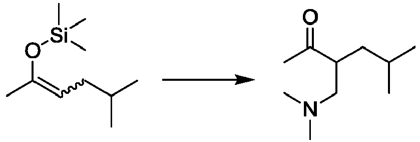

**3-[(Dimethylamino)methyl]-5-methylhexan-2-one:** To a stirred solution of trimethyl(5-methylhex-2-en-2-yloxy)silane (118 g, 633 mmol, 1.00 equiv) in anhydrous acetonitrile (800 mL) was added N-methyl-N-methylenemethanaminium iodide (128.8 g, 696.3 mmol, 1.10 equiv) in several batches and the resultant mixture was stirred at 20°C overnight. Then the solution was concentrated under vacuum to remove the solvent. The residue was dissolved in 400 mL 1 N HCl (aq.) and extracted with *tert-*butyl methyl ether. Then the water phase was basiced with 2 N aq. NaOH and extracted with *tert-*butyl methyl ether. The organic phase was dried and concentrated under vacuum. The liquid was purified by distilling (80°C/0.5 mmHg) to provide 50 g (46%) of 3-[(dimethylamino)methyl]-5-methylhexan-2-one as a colorless oil. ¹H-NMR (300 MHz, *d₆*-DMSO) *δ* 0.92 (d, 3H), 0.98 (d, 3H), 1.11-1.23 (m, 1H), 1.23-1.38 (m, 1H), 1.54-1.70 (m, 1H), 2.30 (s, 3H), 3.01 (s, 9H), 3.10-3.32 (m, 2H), 3.81-3.88 (m, 1H).

### Step 8

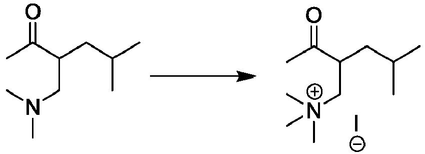

**2-Acetyl-*N,N*,*N*,4-tetramethylpentan-1-aminium iodide:** A solution of 3-[(dimethylamino)methyl]-5-methylhexan-2-one (50 g, 15.00 mmol, 1.00 equiv) and methyl iodide (4.26 g, 30.00 mmol, 2.00 equiv) in 50 mL diethyl ether was stirred overnight at room temperature. The precipitated solid was isolated by filtration and dried under vacuum to give 79 g (86%) of 2-acetyl-*N*,*N*,*N*,4-tetramethylpentan-1-aminium iodide as white solid. ¹H-NMR (300 MHz, *d*₆-DMSO) *δ* 0.89-0.98 (m, 6H), 1.11-1.20 (m, 1H), 1.40 (m, 1H), 1.66 (m, 1H), 2.30 (s, 3H), 3.01(s, 9H), 3.21 (m, 2H), 3.85 (m, 1H).

### Step 9

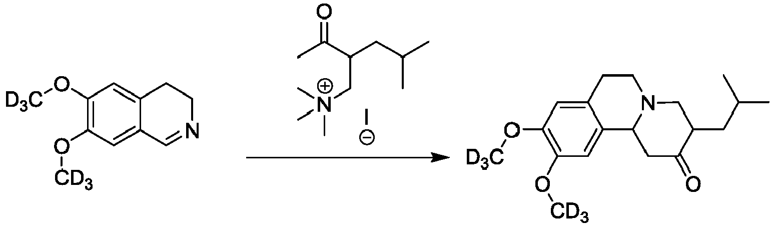

**D₆-(±)-tetrabenazine:** A solution of *d*₆-6,7-dimethoxy-3,4-dihydroisoquinoline (33.4 g, 169 mmol, 1.10 equiv) and 2-acetyl-*N*,*N*,*N*,4-tetramethylpentan-1-aminium iodide (48 g, 153 mmol, 1.00 equiv) in 300ml of methanol was heated under reflux for 48 h. Then 150 mL water was added. The solution was cooled to room temperature. The precipitated solid was isolated by filtration and dried under vacuum to give 38 g of crude d₆-tetrabenazine as yellow solid. The crude tetrabenazine was dissolved in *tert-*butyl methyl ether (15 volumes), the mixture was heated until the solid was almost dissolved. The yellow solid which was unsolvable was filtered. The filtrate was concentrated under vacuum until 2 volumes *tert-*butyl methyl ether was left. The solid was filtered and collected. The above solid was dissolved in ethanol (4 volumes), then the mixture was heated until the solid was dissolved. The solution was stirred and cooled to room temperature at the rate of 20°C/h. Then the mixture was stirred at 0°C for 1h. The precipitated solid was isolated by filtration and dried under vacuum to give 25 g (50.4%) of tetrabenazine-*d*₆ as white solid. ¹H-NMR (300 MHz, CD₂Cl₂) *δ* 6.61 (s, 1H), 6.55 (s, 1H), 3.84 (s, 3H), 3.82 (s, 3H), 3.50 (d, 1H, *J* = 12 Hz), 3.27 (dd, 1H, *J* = 11.4Hz, *J* = 6.3 Hz), 3.11 (m, 2H), 2.84 (dd, 1H, *J* = 10.5 Hz, *J* = 3 Hz), 2.74 (m, 2H), 2.56 (m, 2H), 2.31 (t, 1H, *J* = 12 Hz), 1.76 (m, 1H), 1.63 (m, 1H), 0.98 (m, 1H), 0.89 (m, 6H). LC-MS: *m*/*z* = 324 (MH)⁺.

### EXAMPLE 2

### D₆-(±)-alpha-3-Isobutyl-9,10-dimethoxy-2,3,4,6,7,11b-hexahydro-1H-pyrido[2,1-a]isoquinolin-2-ol ((±)-alpha-dihydrotetrabenazine-d₆)

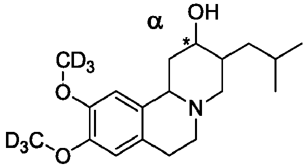

### Step 1

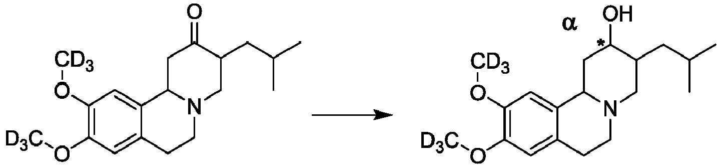

**D₆-(±)-alpha-dihydrotetrabenazine:** To d₆-(±)-tetrabenazine (2 g, 6.18 mmol, 1.00 equiv) in 20 mL of ethanol at 0 °C, was added NaBH₄ (470 mg, 12.36 mmol, 2.00 equiv) in several batches at 0 °C. The reaction mixture was allowed to stir for 60 min at room temperature. The excess solvent was carefully removed under vacuum, and the residue was dissolved in 50 mL dichloromethane and washed with three portions of saturated aqueous brine. The combined organic extracts were dried over sodium sulfate, filtered, and concentrated under reduced pressure to provide a white solid. The solid was further purified by recrystallization from ethanol to afford 610 mg of *d*₆-(±)-alpha-dihydrotetrabenazine (30%) as a white solid. ¹H-NMR (300 MHz, CDCl₃) *δ* 6.68 (s, 1H), 6.59 (s, 1H), 3.42 (m, 1H), 3.42 (m, 4H), 2.63 (m, 2H), 2.49 (m, 1H), 2.01 (t, 1H, *J* = 11.4 Hz), 1.75 (m, 2H), 1.56 (m, 3H), 1.05 (dd, 1H, *J* = 9.9 Hz, *J* = 13.8 Hz), 0.95 (m, 6H). MS: *m*/*z* = 326 [M+H]⁺.

### EXAMPLE 3

### D₆-(±)-beta-3-Isobutyl-9,10-dimethoxy-2,3,4,6,7,11b-hexahydro-1H-pyrido[2,1-a]isoquinolin-2-ol ((±)-beta-dihydrotetrabenazine-d₆)

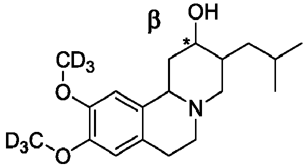

### Step 1

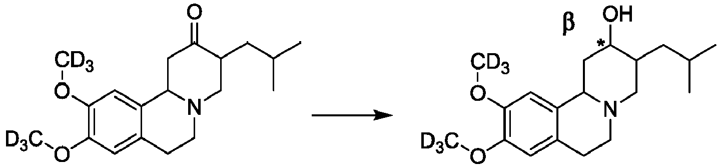

**D₆-(±)-beta-dihydrotetrabenazine:** To d₆-(±)-tetrabenazine (1 g, 3.1 mmol, 1.00 equiv) in 20 mL of tetrahydrofuran at 0°C, was added dropwise potassium tri-sec-butyl borohydride (K-selectride) (1 M in tetrahydrofuran) (6.2 mL, 1.00 equiv) at 0°C. The reaction mixture was allowed to stir for 60 min at 0°C. HPLC showed that the reaction was completed. Then the mixture was poured into ice/water (30 mL). The solution was concentrated under vacuum to remove tetrahydrofuran and then extracted with dichloromethane. The combined organic extracts were dried over sodium sulfate, filtered, and concentrated under reduced pressure to provide white solid. The solid was purified by Prep-HPLC to afford 640 mg *d*₆-(±)-beta-dihydrotetrabenazine (63%) as white solid. ¹H-NMR (300 MHz, CDCl₃) *δ* 6.69 (s, 1H), 6.60 (s, 1H), 4.10 (s, 1H), 3.54 (m, 1H), 3.21 (m, 1H), 2.99 (m, 1H), 2.65 (m, 3H), 2.51 (m, 2H), 2.02 (m, 1H), 1.73 (m, 2H), 1.52 (m, 1H), 1.23 (m, 2H). MS: *m*/*z* = 326 [M+H]⁺.

### EXAMPLE 4

### 2-Acetyl-N,N,N,4-tetramethylpentan-1-aminium iodide

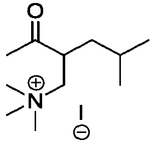

### Step 1

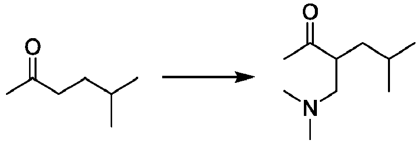

**3-[(Dimethylamino)methyl]-5-methylhexan-2-one:** A mixture of dimethylamine hydrochloride (3.78 kg, 46.22 mol, 1.30 equiv), paraformaldehyde (1.45 kg, 48.35 mol, 1.36 equiv), 5-methyl-2-hexanone (4.06 kg, 35.55 mol, 1.00 equiv) and conc. HCl (284 mL) in 95% ethanol (14.6 L) was refluxed for 24 hours under N₂. Then ethanol was removed under reduced pressure. The orange-yellow residue was diluted with 5 L water and extracted with *tert-*butyl methyl ether (2x5.2 L). The pH value of aqueous layers was adjusted to 9 with 20% NaOH. The resulting solution was extracted with ethyl acetate (2x4 L).The organic layers was combined and concentrated under vacuum to give 1150 g of crude product as a yellow liquid (GC showed that 7% of the undesired isomer was contained). This was marked as product A. The pH value of above aqueous layers was adjusted to 9 with 20% NaOH again. The resulting solution was extracted with ethyl acetate (2x4 L).The organic layers was combined and concentrated under vacuum to give 1350 g of crude product as a yellow liquid (GC showed that 15% of of the undesired isomer was contained). This was marked as product B. The product A was diluted with 3 L ethyl acetate, and 50 g toluenesulfonic acid was added, then the solution was stirred overnight at rt. The precipitated solid was removed. The filtrate was washed with water (2x400 mL) and 5% aqueous NaOH (200 mL). The product B was diluted with 3.5 L ethyl acetate, and 200 g toluenesulfonic acid was added, then the solution was stirred overnight at rt. The precipitated solid was removed and the filtrate was washed with water (2x400 mL) and 5% aqueous NaOH (200 mL). The two parts of above organic phase was dried over sdium sulfate and concentrated under vacuum to give 2.2 kg of 3-[(dimethylamino)methyl]-5-methylhexan-2-one (36%) as yellow liquid. (2% of the undesired isomer was found by GC). ¹H-NMR (300 MHz, *d*₆-DMSO) *δ* 0.92 (d, 3H), 0.98 (d, 3H), 1.11-1.23 (m, 1H), 1.23-1.38 (m, 1H), 1.54-1.70 (m, 1H), 2.30 (s, 3H), 3.01 (s, 9H), 3.10-3.32 (m, 2H), 3.81-3.88 (m, 1H). MS: *m*/*z* = 172 [M+H]⁺.

### Step 2

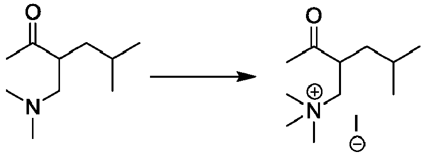

**2-Acetyl-*N*,*N*,*N*,4-tetramethylpentan-1-aminium iodide:** A solution of 3-[(dimethylamino)methyl]-5-methylhexan-2-one (2.2 kg, 12.84 mol, 1.00 equiv) in dichloromethane (10 L) was dropwised a solution of methyl iodide (2 kg, 14.12 mol, 1.1 equiv) in dichloromethane (2 L) at 5∼10°C. Then the solution was stirred overnight at rt. The reaction was monitored by LCMS until completion of reaction (3-[(dimethylamino)methyl]-5-methylhexan-2-one < 5.0%). The precipitated solid was isolated by filtration and dried under vacuum to give 3.5 kg (87%) of 2-Acetyl-*N*,*N*,*N*,4-tetramethylpentan-1-aminium iodide as white solid. ¹H-NMR (300 MHz, *d*₆-DMSO) *δ* 0.89-0.98 (m, 6H), 1.11-1.20 (m, 1H), 1.40 (m, 1H), 1.66 (m, 1H), 2.30 (s, 3H), 3.01(s, 9H), 3.21 (m, 2H), 3.85 (m, 1H). MS: *m*/*z* =186 [M+H]⁺.

### EXAMPLE 5

### d₆-3-(2-hydroxy-2-methylpropyl)-9,10-dimethoxy-3,4,6,7-tetrahydro-1H-pyrido[2,1-a]isoquinolin-2(11bH)-one (racemic mixture of -(3S,11bS) and-(3R,11bR) enantiomers)

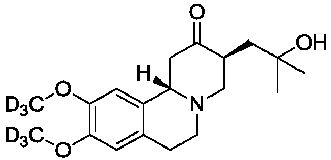

### Step 1

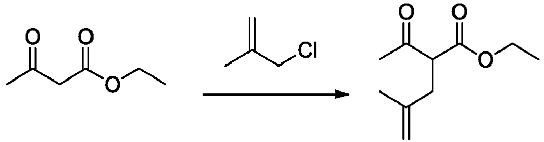

**Ethyl 2-acetyl-4-methylpent-4-enoate:** To a solution of ethyl acetoacetate (500 g, 3.84 mol, 1.00 eq), potassium iodide (63.8 g, 0.384 mol, 0.10 eq), tetrabutylammonium bromide (136.2 g, 0.422 mol, 0.11 eq), and K₂CO₃ (631.9 g, 4.57 mol, 1.19 eq) in dimethylformamide (1.5 L) was heated to 40-50 °C. At this temperature 3-chloro-2-methyl-1-propene (382.6 g, 4.22 mol, 1.10 eq) was added. The reaction mixture was heated to 65-75°C and stirred for 6 hrs. Then the reaction mixture was cool to 25-35 °C and quenched with water (5.00 L). The product was extracted with toluene (2x2.00 L), and the combined toluene layers were washed with water (2x1.5 L) and concentrated under vacuum at 50-55 °C to give 707 g of ethyl 2-acetyl-4-methylpent-4-enoate (quantitative yield) as a brown liquid.

### Step 2

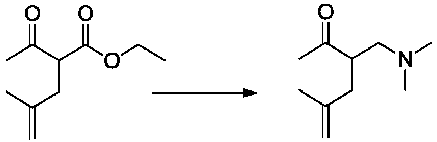

**3-((Dimethylamino)methyl)-5-methylhex-5-en-2-one:** To a solution of potassium hydroxide (234.5 g, 4.18 mol, 1.10 eq) in water (4.2 L) was added ethyl 2-acetyl-4-methylpent-4-enoate (700 g, 3.80 mol, 1.0 eq) and stirred at 25-35 °C for 4 hrs. The reaction mixture was washed with methyl tert-butyl ether (2x2.80 L). The pH of the aqueous layer was adjusted to 6.8-7.2 using concentrated hydrochloric acid. Then dimethylamine hydrochloride (464.8 g, 5.70 mol, 1.5 eq), 37% formaldehyde solution (474 mL, 6.36 mol, 1.675 eq) and tetrabutylammonium bromide (122.5 g, 0.38 mol, 0.10 eq) were added. Concentrated hydrochloric acid was added to the reaction mixture at 25-35 °C for 60-90 minutes until the pH of the reaction mixture was <1. Then the reaction mixture was stirred at 25-35 °C for 15 hrs. The reaction mixture was washed with methyl tert-butyl ether (2x2.8 L). The pH of the aqueous layer was adjusted to 9-10 by using 20% potassium hydroxide solution. Then the product was extracted with ethyl acetate (3x2.8 L). The ethyl acetate layer was washed with water (2x2.1 L), followed by 10% ammonium chloride solution (2x3.5L). Then the ethyl acetate layer was treated with activated carbon (5% w/w), filtered through a bed of celite which was washed with ethyl acetate (350 mL). The filtrate was dried over sodium sulfate and distilled under vacuum at 40-45 °C to give 122 g of 3-((dimethylamino)methyl)-5-methylhex-5-en-2-one as a brown liquid (19% yield).

### Step 3

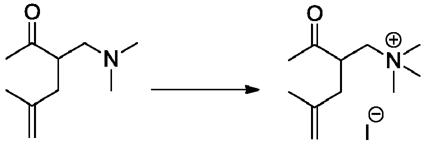

**2-Acetyl-N,N,N,4-tetramethylpent-4-en-1-aminium iodide:** To a solution of 3-((dimethylamino)methyl)-5-methylhex-5-en-2-one (40 g, 0.236 mol, 1.00 eq) in methyl tert-butyl ether (600 L) was added methyl iodide (77.25 g, 0.544 mol, 2.30 eq) at 0-10 °C for 1-2 hrs. Then the reaction mixture was stirred at 25-35 °C for 15 hrs and at 40-42 °C for 6 hrs. The reaction mixture was cooled to 25-35 °C, filtered, and washed with methyl tert-butyl ether (400 L) to give 54 g of 2-acetyl-N,N,N,4-tetramethylpent-4-en-1-aminium iodide as off white solid (73.3% yield).

### Step 4

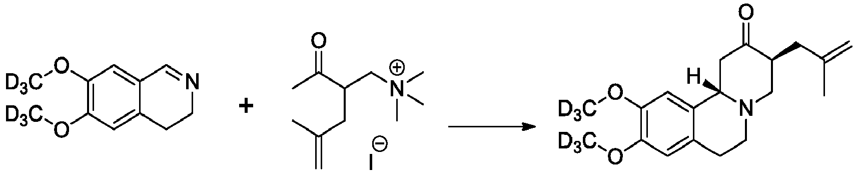

**D₆**-**9,10-dimethoxy-3-(2-methylallyl)-3,4,6,7-tetrahydro-1H-pyrido[2,1-a]isoquinolin-2(11bH)-one (racemic mixture of -(3S,11bS) and-(3R,11bR) enantiomers):** To a solution of d₆-6,7-dimethoxy-3,4-dihydroisoquinoline (35 g, 0.149 mol, 1.00 eq) and 2-acetyl-N,N,N,4-tetramethylpent-4-en-1-aminium iodide (50.34 g, 0.161 mol, 1.08 eq) in 3:1 methanol water (210 mL) was added K₂CO₃ (20.71 g, 0.149 mol, 1.00 eq). The reaction mixture was heated to 40-45 °C for 30 hrs. Then the reaction mixture was cooled to room temperature (25-35 °C) and water was added (105 mL). The reaction mixture was stirred for 30 minutes. The precipitated solid was filtered, washed with water (105 mL), and dried to give 42 g of crude d₆-(3S,11bS)-9,10-dimethoxy-3-(2-methylallyl)-3,4,6,7-tetrahydro-1H-pyrido[2,1-a]isoquinolin-2(11bH)-one as a yellow solid. The crude product upon recrystallization using ethanol (3 volumes) gave 38 g d₆-(3S,11bS)-9,10-dimethoxy-3-(2-methylallyl)-3,4,6,7-tetrahydro-1H-pyrido[2,1-a]isoquinolin-2(11bH)-one (36% yield) as an off-white solid.

### Step 5

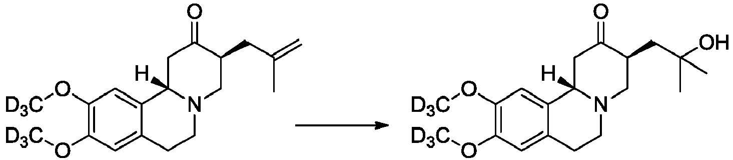

**D₆-3-(2-hydroxy-2-methylpropyl)-9,10-dimethoxy-3,4,6,7-tetrahydro-1H-pyrido[2,1-a]isoquinolin-2(11bH)-one (racemic mixture of-(3S,11bS) and -(3R,11bR) enantiomers):** d₆-(3S,11bS)-9,10-dimethoxy-3-(2-methylallyl)-3,4,6,7-tetrahydro-1H-pyrido[2,1-a]isoquinolin-2(11bH)-one (2 g, 0.0062 mol, 1.00 eq) was taken up in aqueous sulfuric acid (3.6 M, 40 mL) and stirred for 18 hrs at 25-35 °C. The reaction mixture was cooled to 0-5 °C and adjusted to pH to 9-10 by using 5% NaOH solution. The product was extracted with ethyl acetate (2x75 mL). The ethyl acetate layer was washed with water (2x25 mL). The ethyl acetate layer was dried with sodium sulfate and distilled under vacuum at 40-45 °C to give 2 g of crude d₆-(3S,11bS)-3-(2-hydroxy-2-methylpropyl)-9,10-dimethoxy-3,4,6,7-tetrahydro-1H-pyrido[2,1-a]isoquinolin-2(11bH)-one (94.7%) as an off white solid. This crude compound was purified by recrystallization from ethanol (12 mL) to give 0.78 g of pure d₆-(3S,11bS)-3-(2-hydroxy-2-methylpropyl)-9,10-dimethoxy-3,4,6,7-tetrahydro-1H-pyrido[2,1-a]isoquinolin-2(11bH)-one as an white solid (36.9% yield).

### EXAMPLE 6

### d₆-3-(2-hydroxy-2-methylpropyl)-9,10-dimethoxy-3,4,6,7-tetrahydro-1H-pyrido[2,1-a]isoquinolin-2(11bH)-one (mixture of diastereomers)

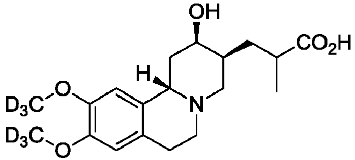

### Step 1

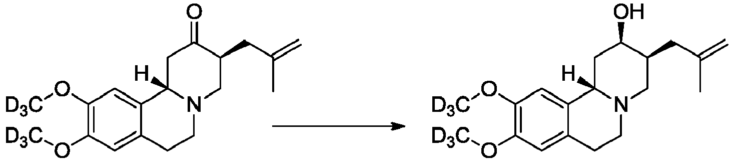

**D₆-9,10-dimethoxy-3-(2-methylallyl)-2,3,4,6,7,11b-hexahydro-1H-pyrido[2,1-a]isoquinolin-2-ol (mixture of diastereomers):** To a solution of (3S,11bS)-9,10-dimethoxy-3-(2-methylallyl)-3,4,6,7-tetrahydro-1H-pyrido[2,1-a]isoquinolin-2(llbH)-one (20 g, 0.0623 mol, 1.00 eq) in tetrahydrofuran (300 mL) was added potassium sec-butylborohydride (1M) (74.76 mL, 0.0747 mol, 1.2 eq) at 0-5 °C for 30 minutes and the reaction mixture was stirred for 30 minutes. Water (200 mL) was added to the reaction mixture and stirred for 15 minutes. The reaction mixture was concentrated under vacuum at 40 °C until complete removal of tetrahydrofuran. The precipitated solid was filtered and washed with water (400 mL) to give 19.6 g [00116] d₆-(2R,3S,11bS)-9,10-dimethoxy-3-(2-methylallyl)-2,3,4,6,7,11b-hexahydro-1H-pyrido[2,1-a]isoquinolin-2-ol as an orange solid (97.4% yield).

### Step 2

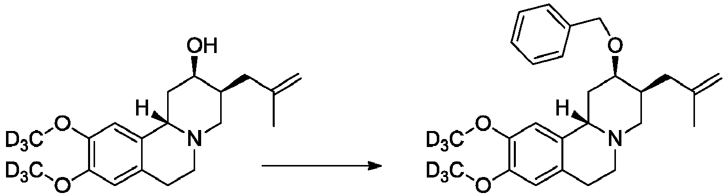

**D₆-2-(benzyloxy)-9,10-dimethoxy-3-(2-methylallyl)-2,3,4,6,7,11b-hexahydro-1H-pyrido[2,1-a]isoquinoline (mixture of diastereomers):** To a solution of d₆-(2R,3S,11bS)-9,10-dimethoxy-3-(2-methylallyl)-2,3,4,6,7,11b-hexahydro-1H-pyrido[2,1-a]isoquinolin-2-ol (22 g, 0.0681 mol, 1.00 eq) in dimethylforamide (220 mL) was added sodium hydride potion wise at 0-5 °C under a nitrogen atmosphere. The reaction mixture was slowly heated to 25-35 °C and stirred for 1 hr. Benzyl bromide (8.14 mL, 0.06811, 1.00 eq) was added to the reaction mass at 0-5 °C over 20 minutes and stirred for 30 minutes. The reaction mixture was quenched with cold water (440 mL) at 0-5 °C and the compound was extracted with ethyl acetate (2x 220 mL and 1x110 mL). The combined organic layers were washed with water (3x110 mL), dried over sodium sulfate, and distilled under vacuum at 40-45 °C to give crude d₆-(2R,3S,11bS)-2-(benzyloxy)-9,10-dimethoxy-3-(2-methylallyl)-2,3,4,6,7,11b-hexahydro-1H-pyrido[2,1-a]isoquinoline in quantitative yield as a dark brown thick liquid. Purification by chromatography (25% ethyl acetate in hexane) gave 8.62 g of d₆-(2R,3S,11bS)-2-(benzyloxy)-9,10-dimethoxy-3-(2-methylallyl)-2,3,4,6,7,11b-hexahydro-1H-pyrido[2,1-a]isoquinoline as a pale yellow solid (30.6% yield).

### Step 3

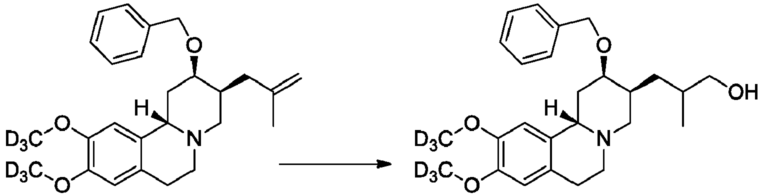

### D₆-2-(benzyloxy)-9,10-dimethoxy-2,3,4,6,7,11b-hexahydro-1H-pyrido[2,1-a]isoquinolin-3-yl)-2-methylpropan-1-ol (mixture of diastereomers):

To a solution of d₆-(2R,3S,11bS)-2-(benzyloxy)-9,10-dimethoxy-3-(2-methylallyl)-2,3,4,6,7,11b-hexahydro-1H-pyrido[2,1-a]isoquinoline (11 g, 0.0266 mol, 1.00 eq) in tetrahydrofuran (110 mL) was added borane-dimethylsulfide (4.79 mL, 0.0479 mol, 1.8 eq, 10 M solution) over 30 minutes at 0-5°C under nitrogen atmosphere. The reaction mixture was stirred overnight at 25-30 °C. The reaction mixture was quenched with 3M NaOH solution (22 mL) at 0-5 °C. The reaction mixture was concentrated under vacuum at 40 °C until complete removal of tetrahydrofuran and co-distilled twice with diethyl ether (2x110 mL). 3 M aqueous NaOH solution (55 mL) was added to the remaining residue and heated to 80-90 °C for 2 hrs. The reaction mixture was cooled to 25-30 °C and the product was extracted with ethyl acetate (3x110 mL). The combined organic layers were washed with water (3x110 mL), dried over sodium sulfate, and distilled under vacuum at 40-45 °C to give 11.74 g of crude d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropan-1-ol as a dark brown viscous liquid (quantitative yield). Purification of the crude product by chromatography (1% methanol in ethyl acetate) gave 3.26 g of d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropan-1-ol as a brown viscous liquid which solidified upon standing overnight (28.4% yield).

### Step 4

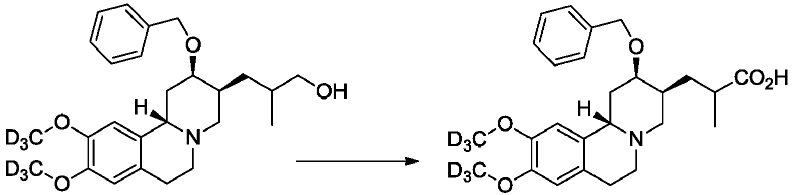

**D₆-2-(benzyloxy)-9,10-dimethoxy-2,3,4,6,7,11b-hexahydro-1H-pyrido[2,1-a]isoquinolin-3-yl)-2-methylpropanoic acid (mixture of diastereomers):** To a solution of d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropan-1-ol (3.2 g, 0.00742 mol, 1.00 eq) in acetone (64 mL) was added freshly prepared Jones reagent at 20 °C in 30 minutes. The reaction mixture was stirred at 20 °C for 30 minutes. The liquid layer was decanted and to the remaining green color gummy mass, acetone (64 mL) was added, stirred for 30 minutes, and decanted. The pH of the combined acetone layers were adjusted to 7 using saturated sodium bicarbonate solution (20 mL). The solids were filtered and washed with acetone (60 mL). The filtrate was distilled under vacuum at 35 °C until complete removal of acetone. The remaining aqueous layer was saturated with sodium chloride and extracted with ethyl acetate (5x60 mL). The combined organic layers were dried over sodium sulfate and concentrated under vacuum at 40-45 °C to give 1.5 g of crude d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropanoic acid (45.4% yield). Purification of the crude product by recrystallization from ethyl acetate (1 volume) gave 0.43 g d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropanoic acid as a pale green solid (13% yield).

**Preparation of Jones reagent:** To a solution of CrO₃ (1.11 g, 0.0111 mol, 1.5 eq) in water (2.04 mL) was added concentrated sulfuric acid (0.928 mL) at 25-30 °C. To the reaction mixture water (1 mL) was added to dissolve the remaining salts. This reagent (orange color clear liquid) was prepared afresh and used for the oxidation reaction.

### Step 5

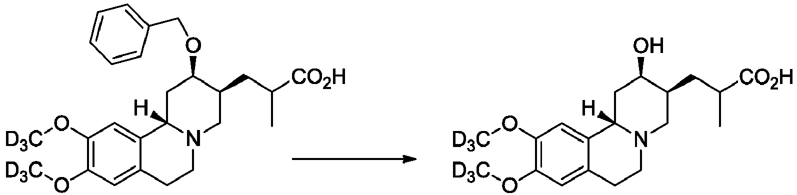

**D₆-3-(2-hydroxy-2-methylpropyl)-9,10-dimethoxy-3,4,6,7-tetrahydro-1H-pyrido[2,1-a]isoquinolin-2(11bH)-one (mixture of diastereomers):** To a solution of d₆-3-((2R,3S,11bS)-2-(benzyloxy)-9,10-dimethoxy-2,3,4,6,7,11b-hexahydro-1H-pyrido[2,1-a]isoquinolin-3-yl)-2-methylpropanoic acid (0.5 g, 0.0011 mol, 1.00 eq) in methanol (150 mL) was added 20% Pd/C (0.25 g, 50% w/w). The reaction mixture was heated to 50-55 °C for 16 hrs. The reaction mixture was cooled to room temperature (25-35 °C), filtered through a celite bed which was washed with methanol (150 mL). The filtrate was distilled under vacuum at 40-45 °C to give 0.39 g of crude d₆-3-((2R,3S,11bS)-2-hydroxy-9,10-dimethoxy-2,3,4,6,7,11b-hexahydro-1H-pyrido[2,1-a]isoquinolin-3-yl)-2-methylpropanoic acid as off-white solid (quantitative yield). This crude compound was purified by preparative HPLC to obtain 70 mg of d₆- 3-((2R,3S,11bS)-2-hydroxy-9,10-dimethoxy-2,3,4,6,7,11b-hexahydro-1H-pyrido[2,1-a]isoquinolin-3-yl)-2-methylpropanoic acid as a white solid (17.5% yield).

The following compounds can generally be made using the methods described above. It is expected that these compounds when made will have activity similar to those described in the examples above.

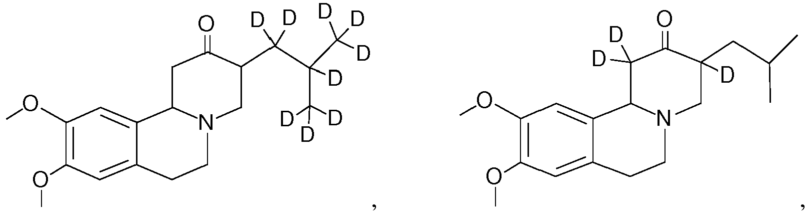

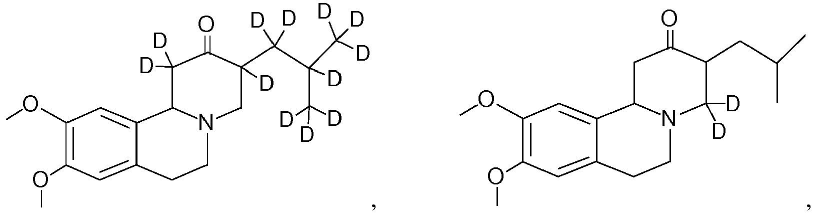

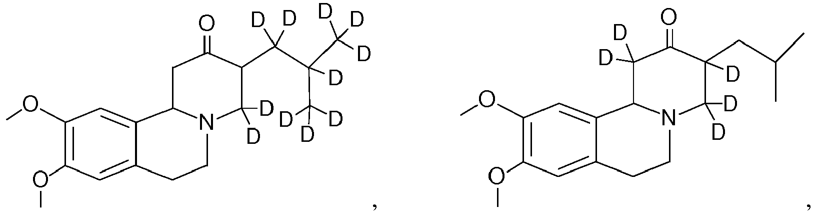

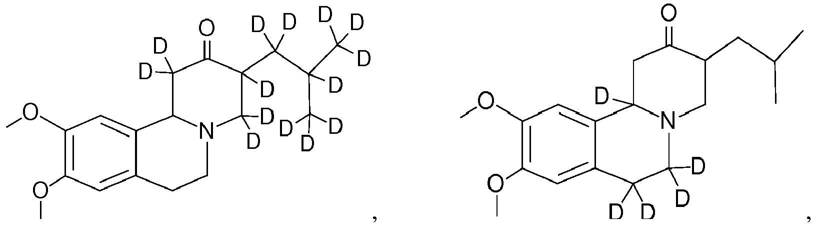

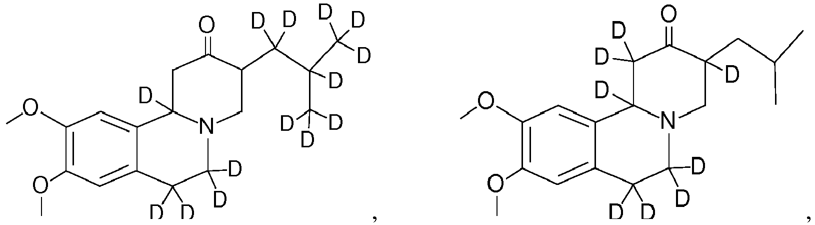

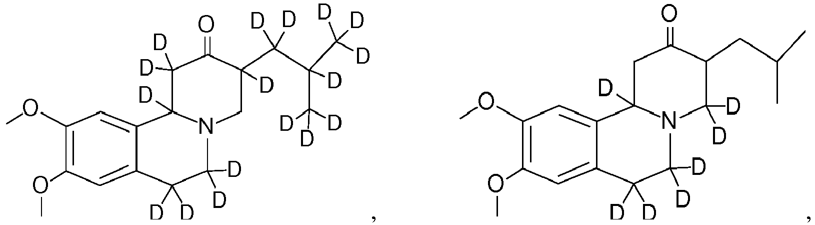

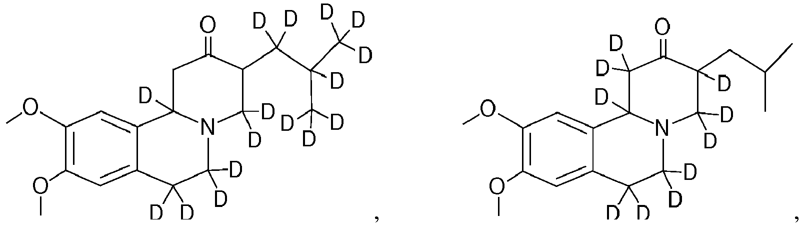

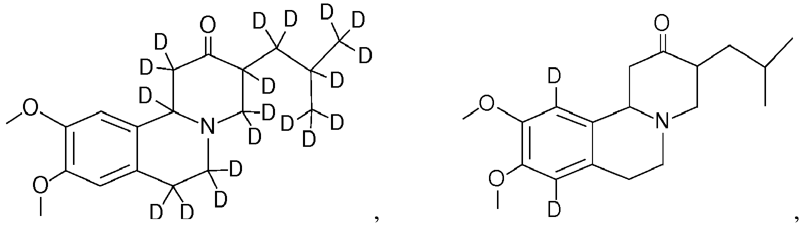

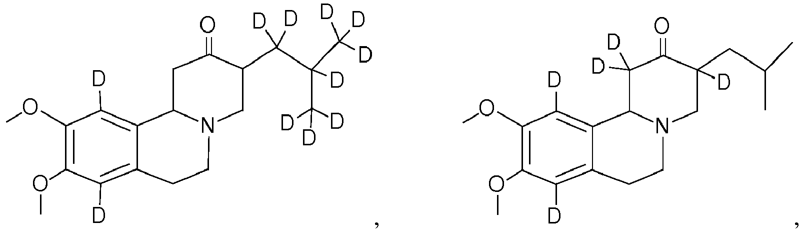

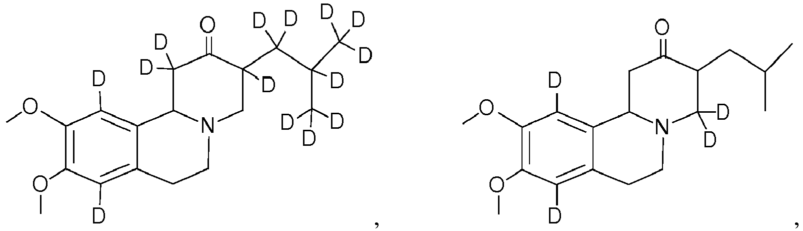

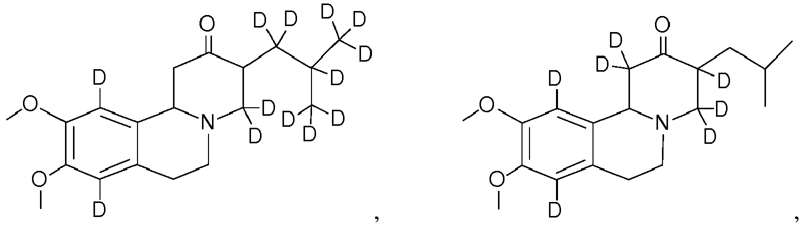

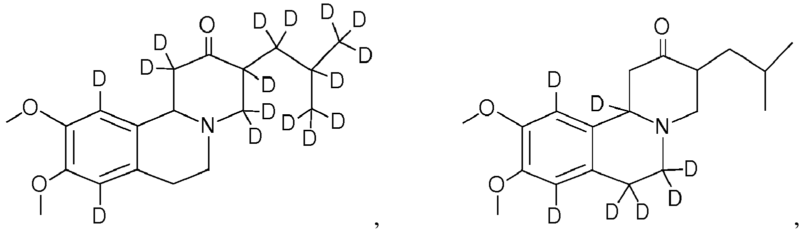

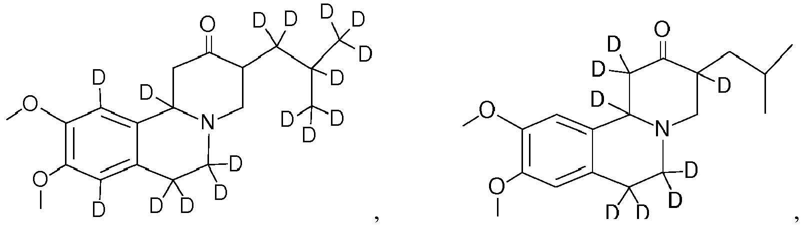

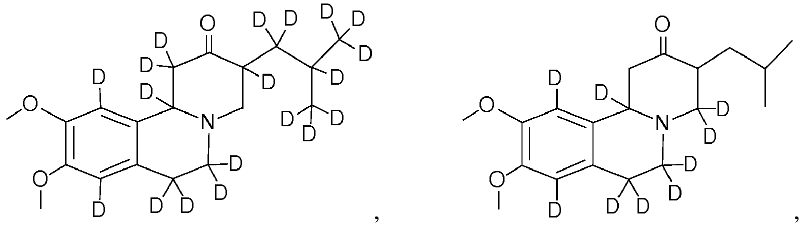

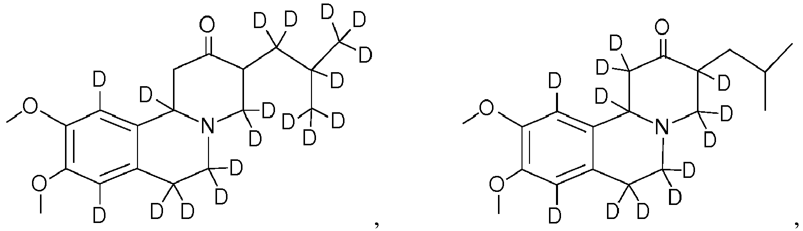

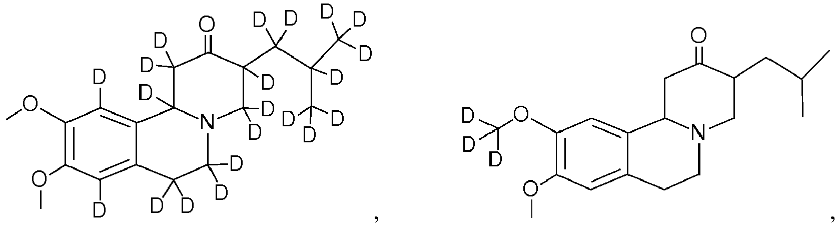

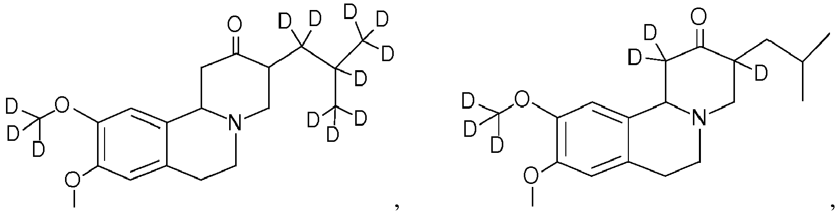

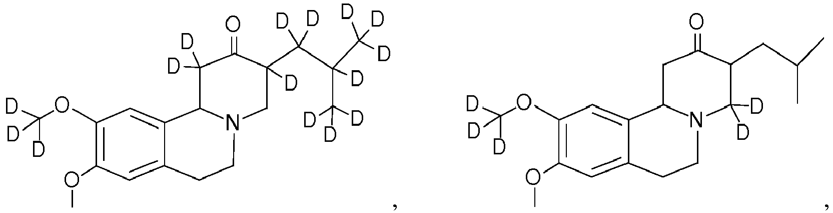

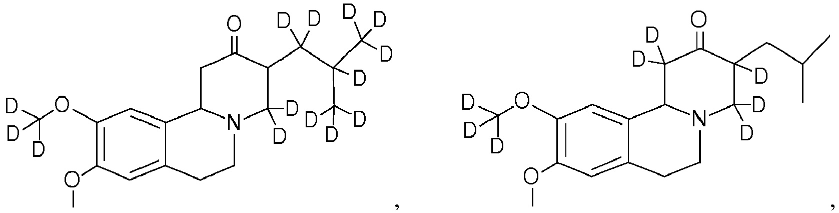

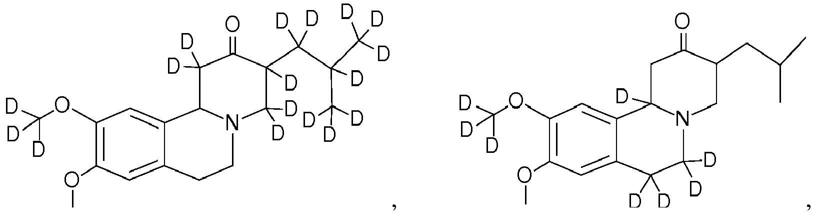

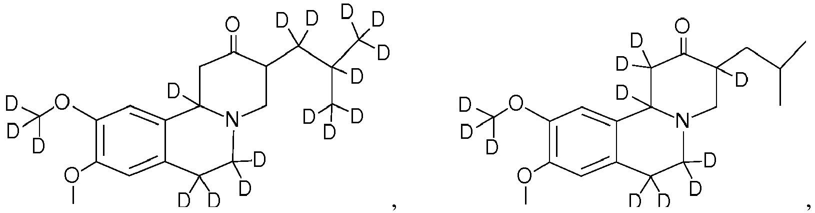

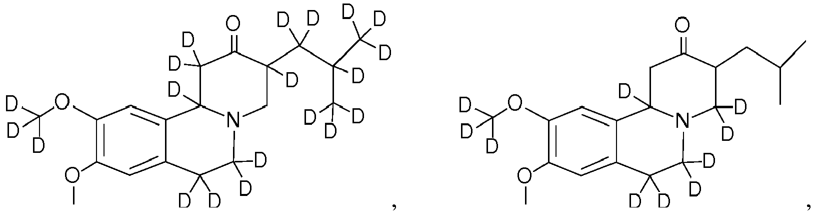

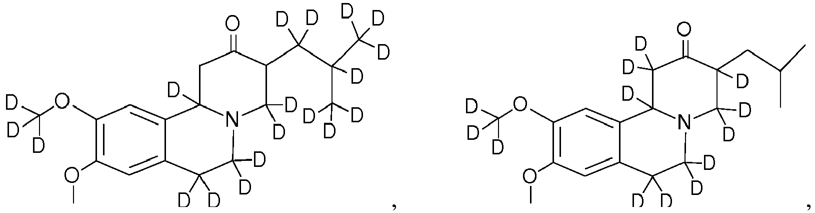

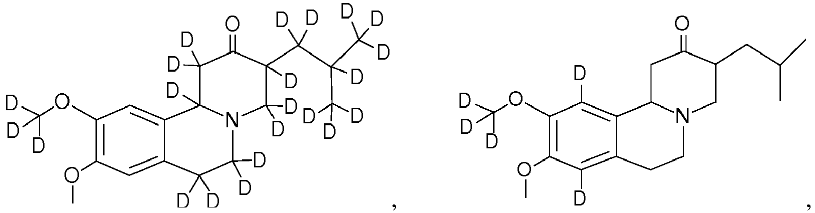

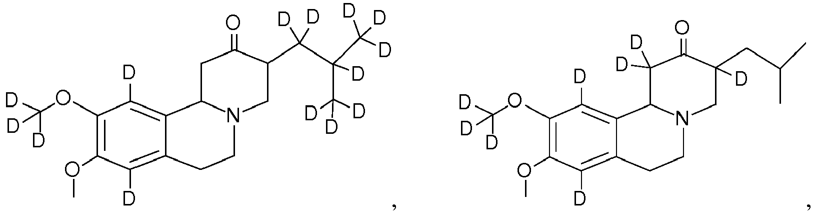

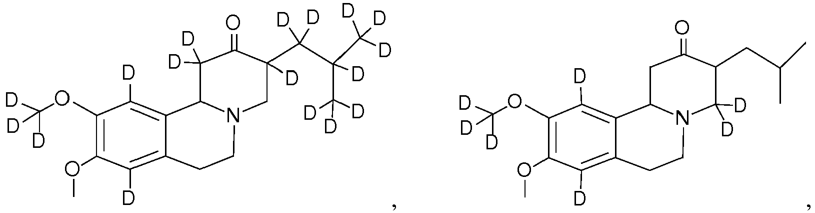

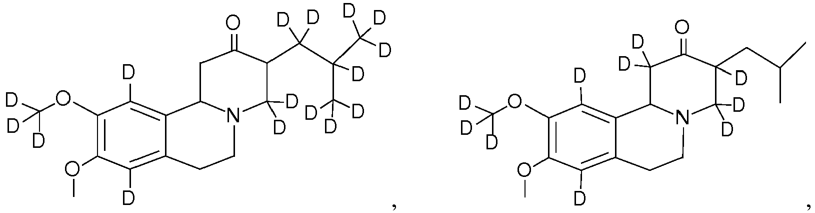

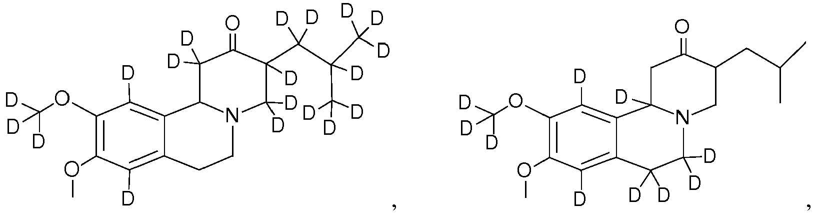

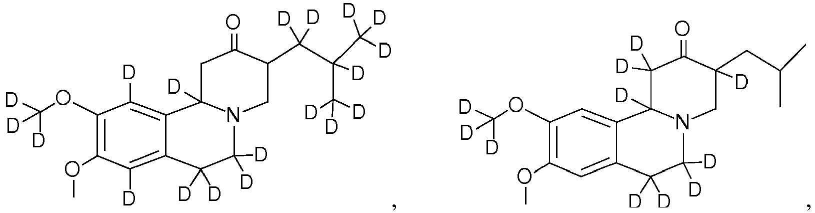

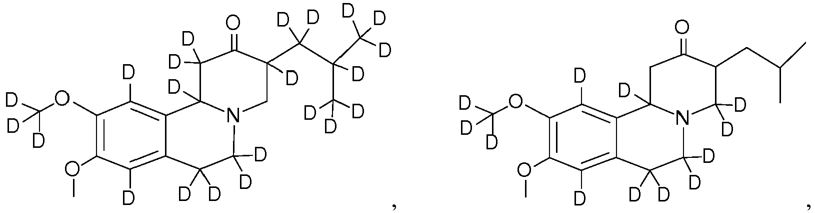

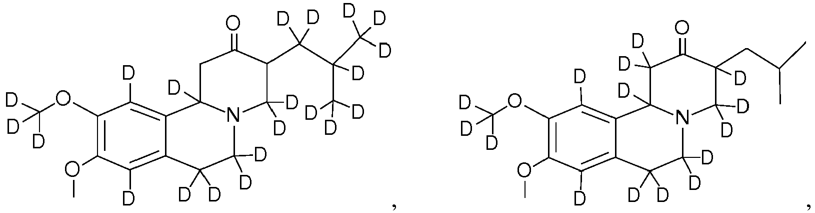

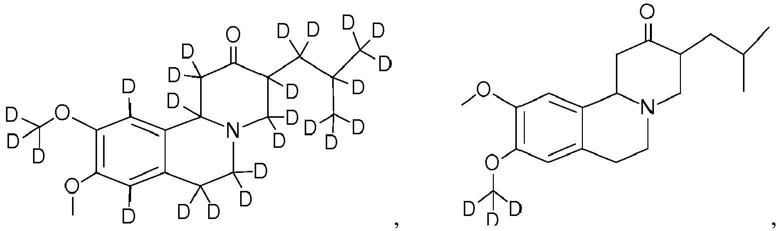

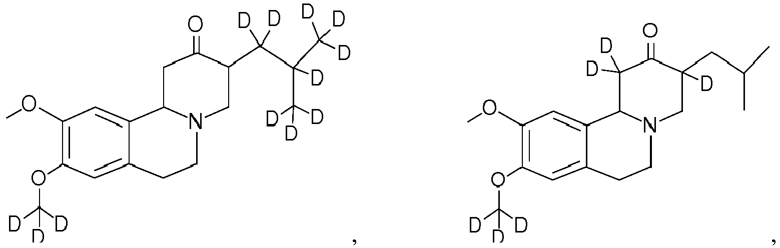

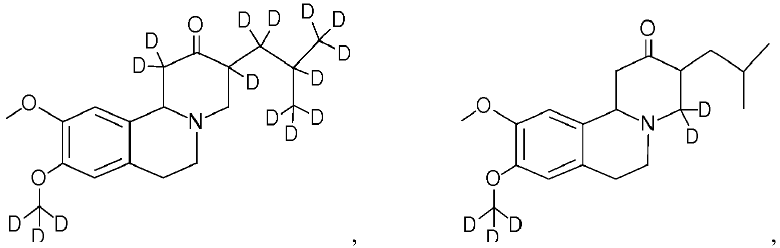

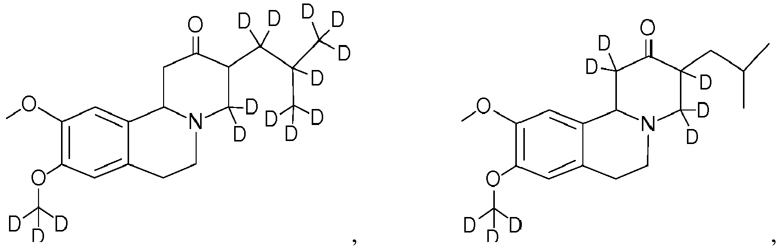

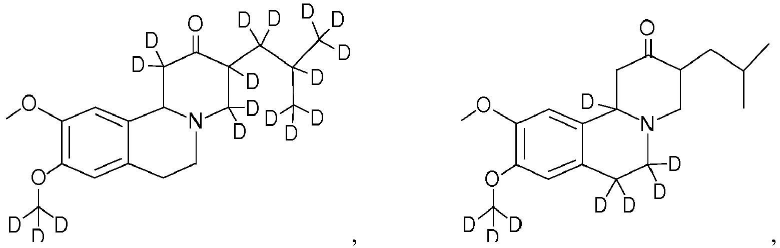

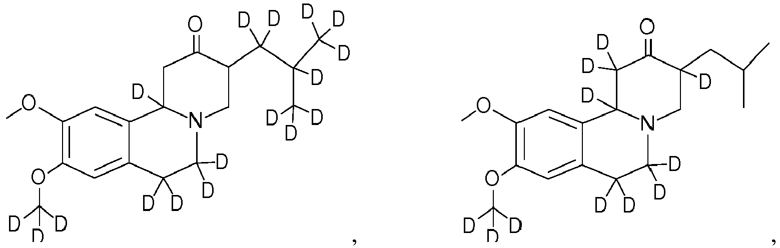

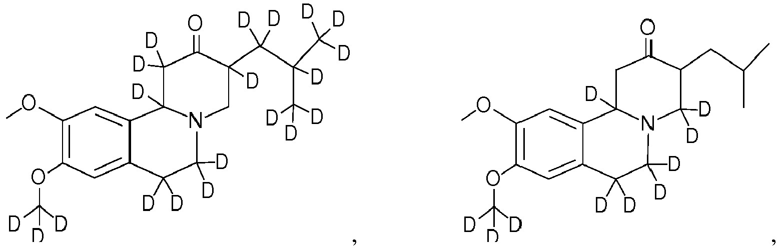

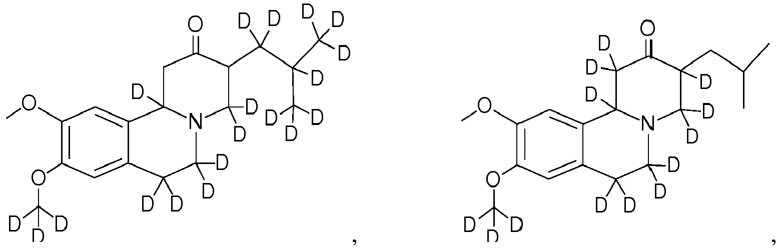

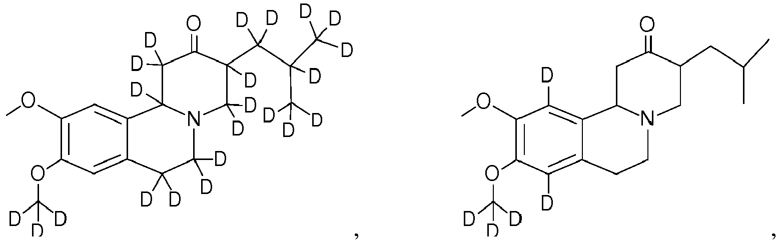

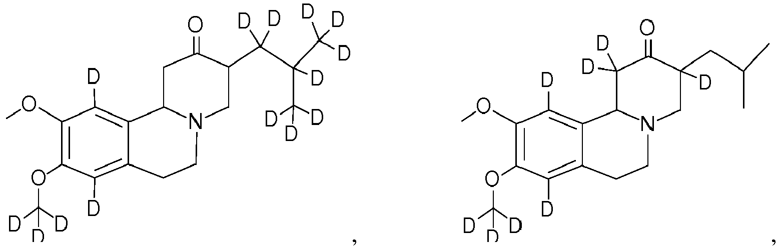

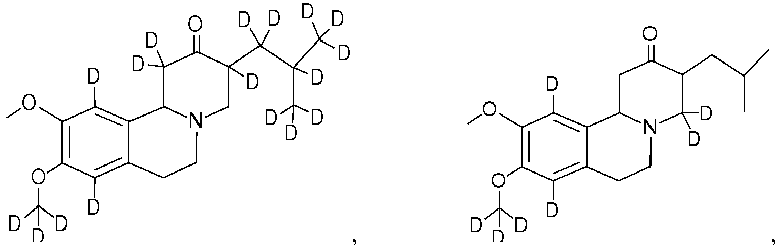

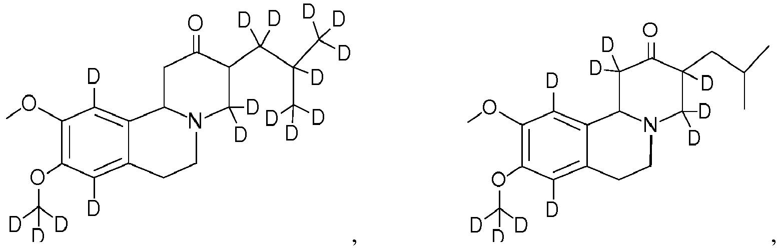

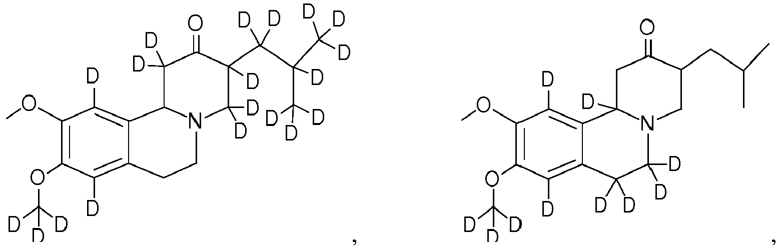

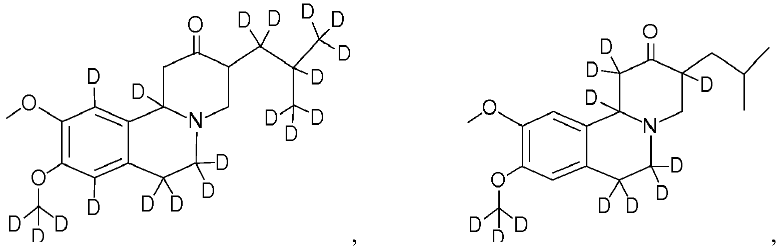

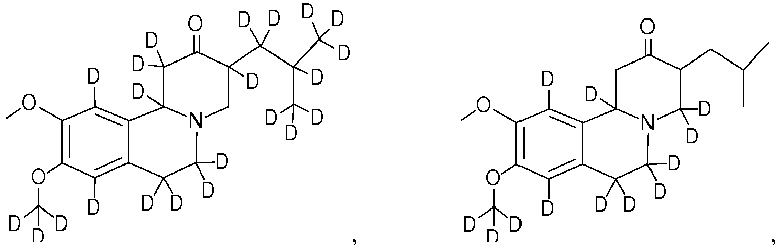

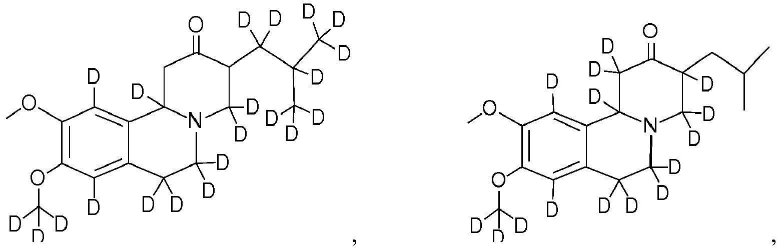

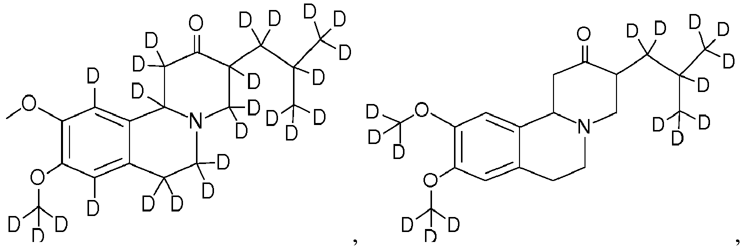

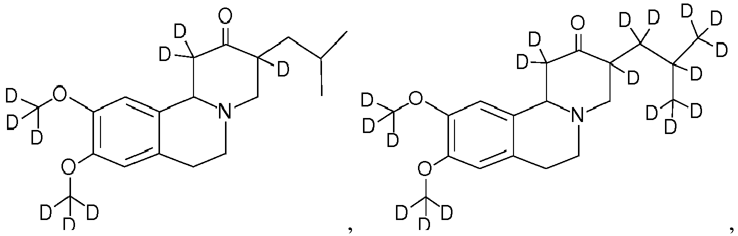

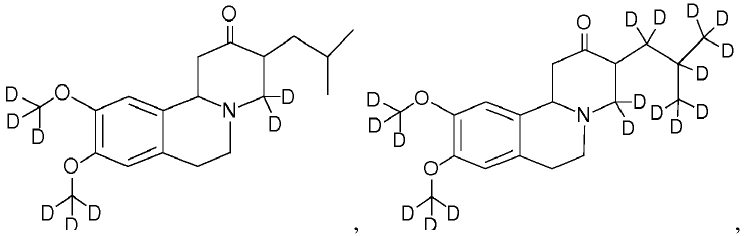

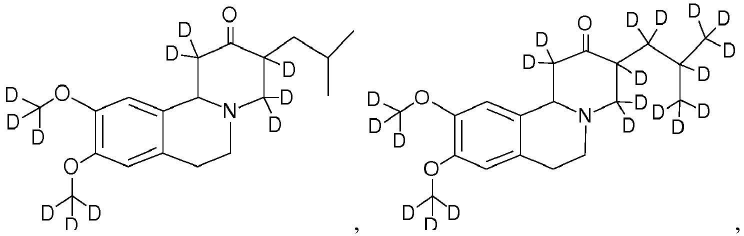

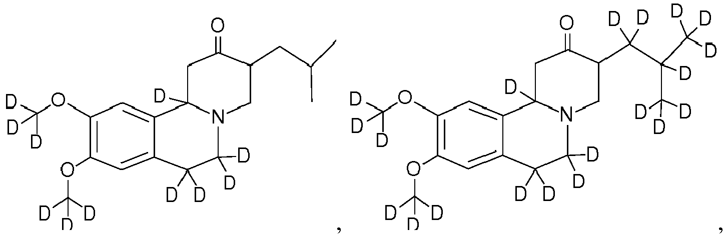

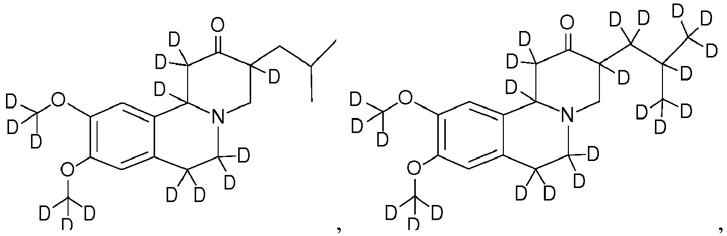

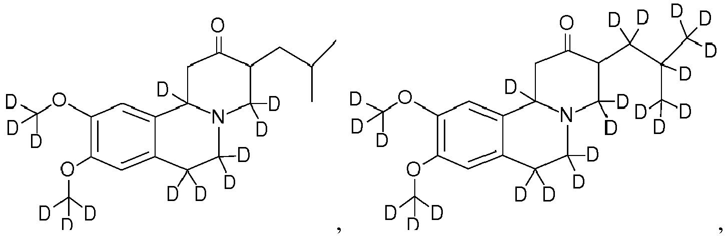

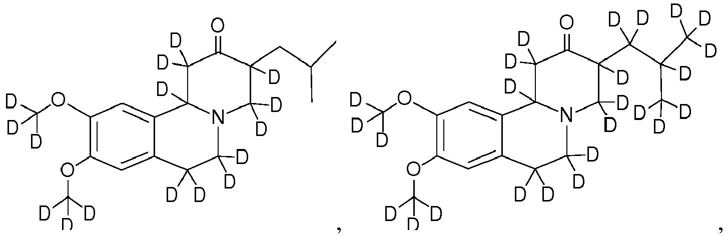

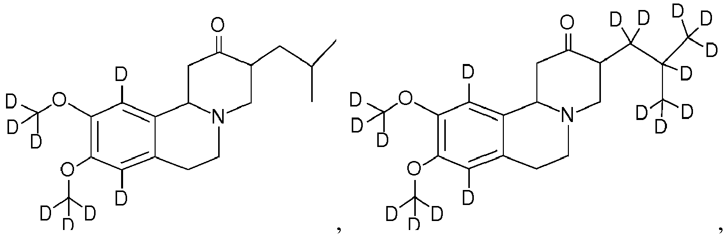

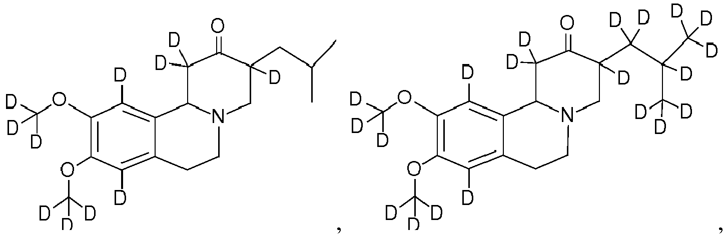

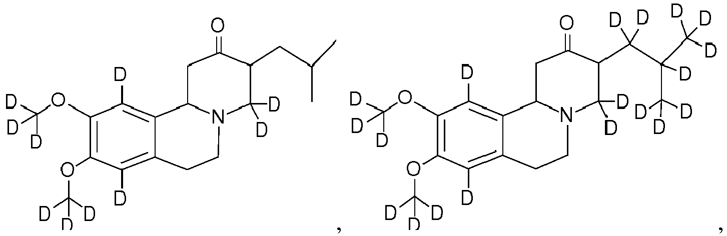

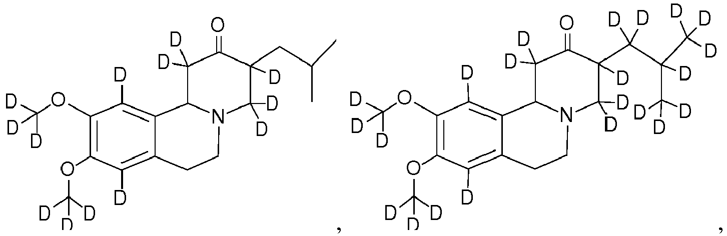

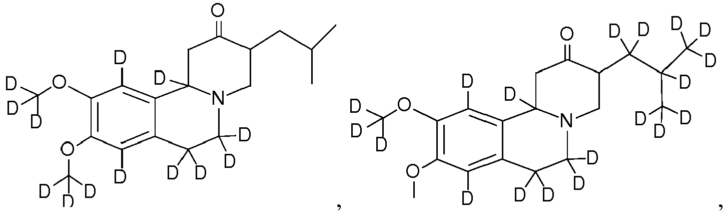

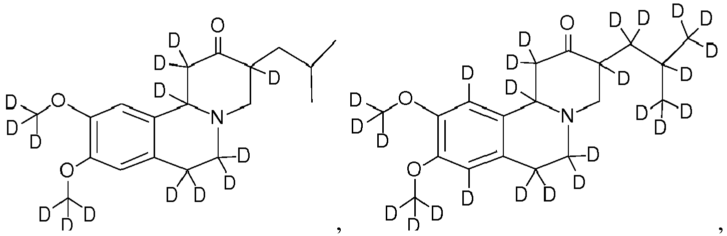

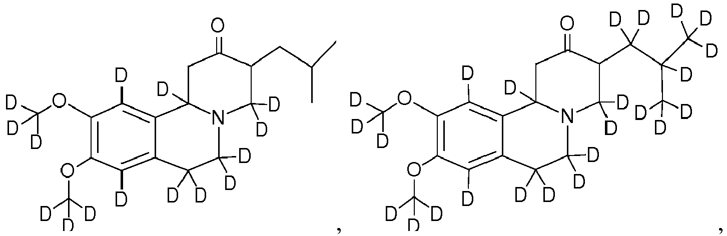

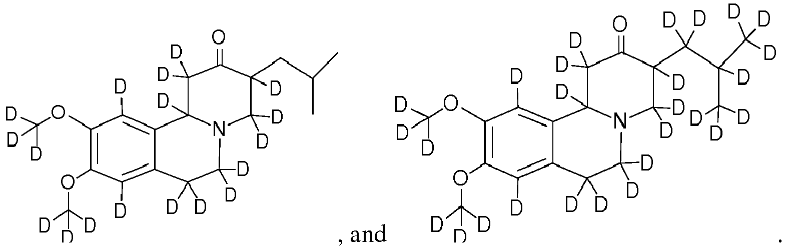

The following compounds can generally be made using the methods described above. It is expected that these compounds when made will have activity similar to those described in the examples above. 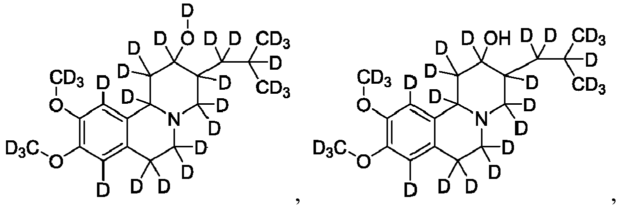 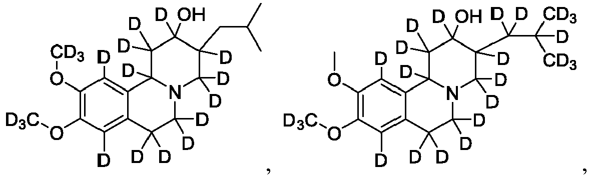 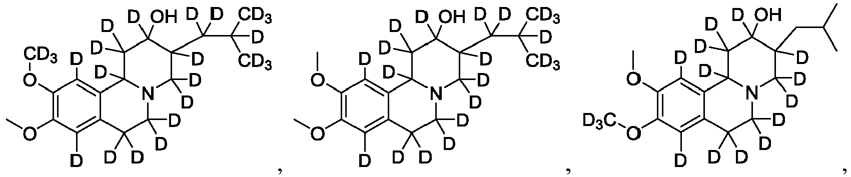 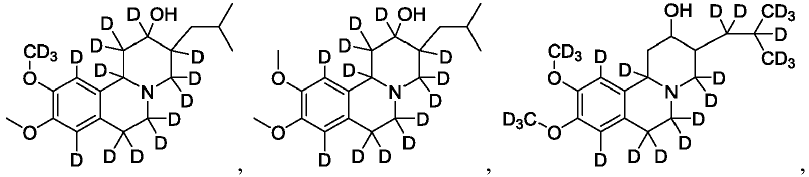 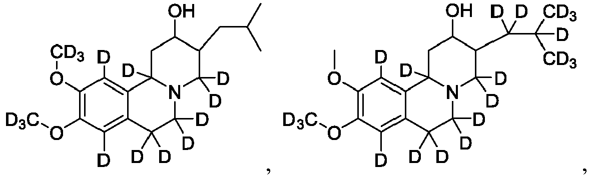 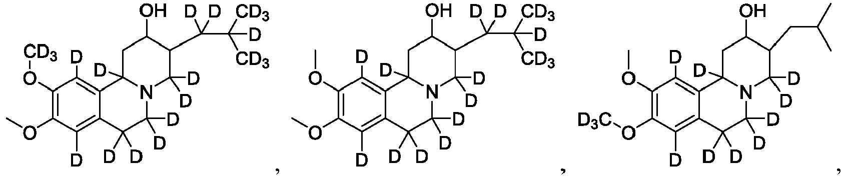 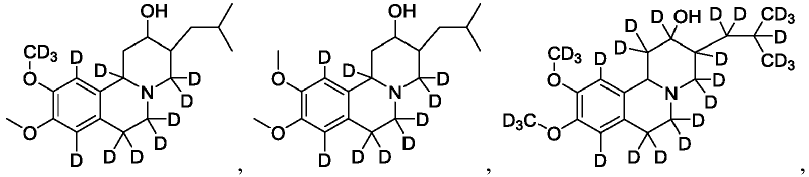 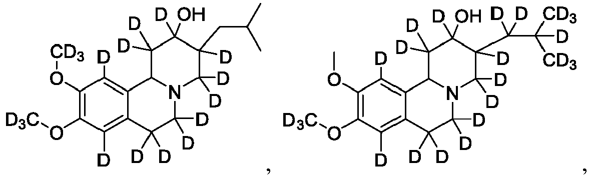 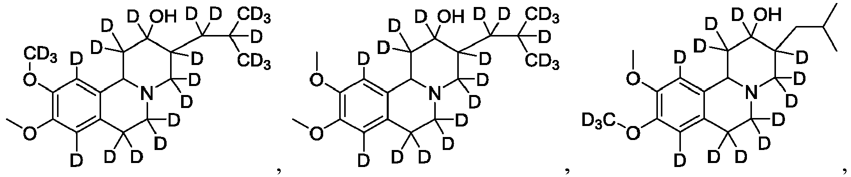 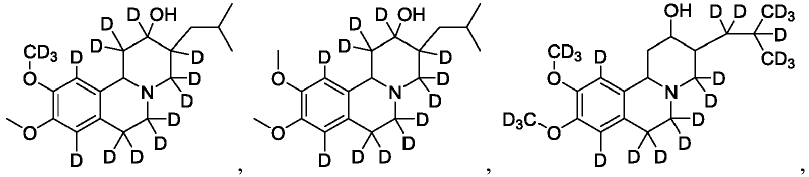 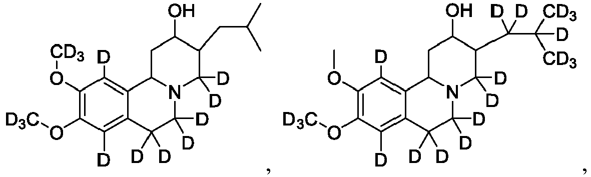 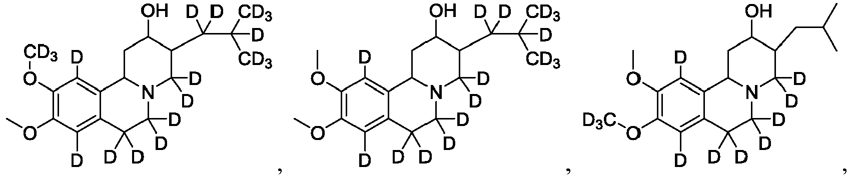 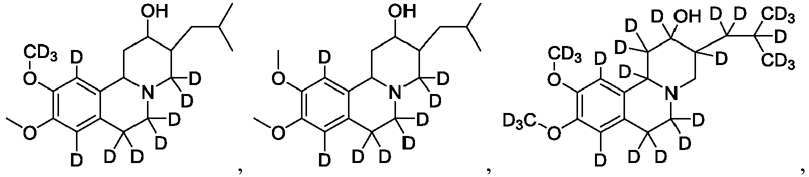 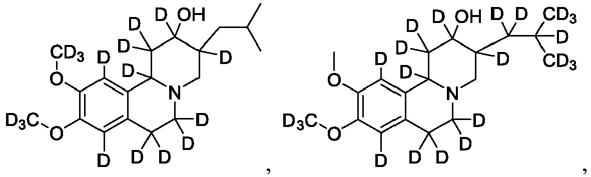 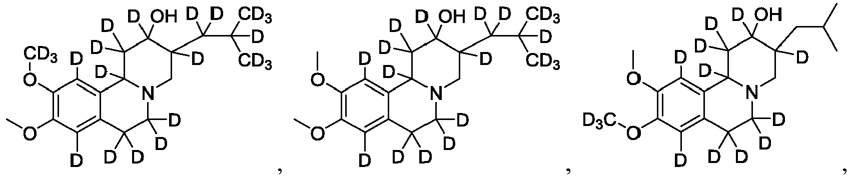 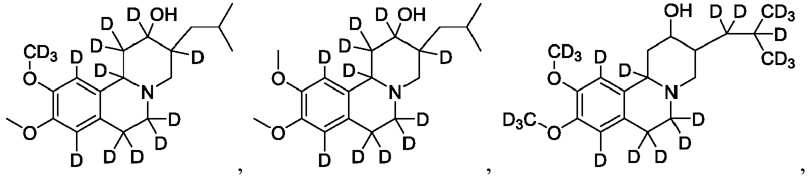 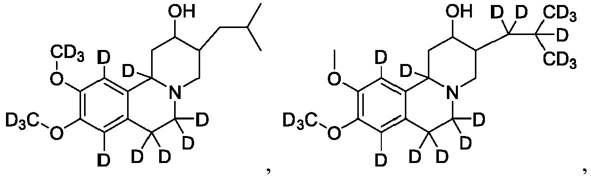 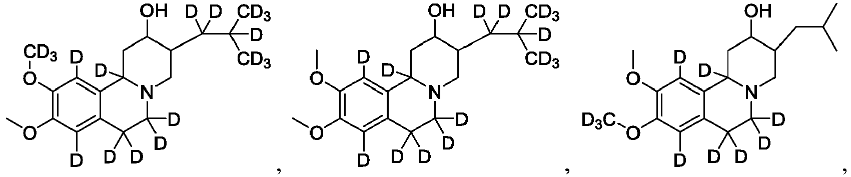 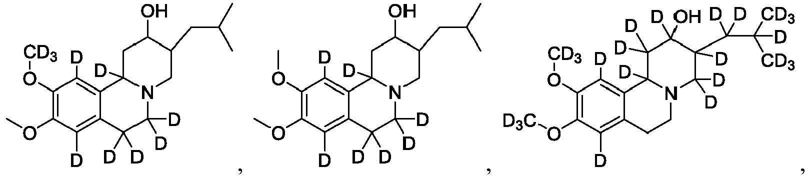 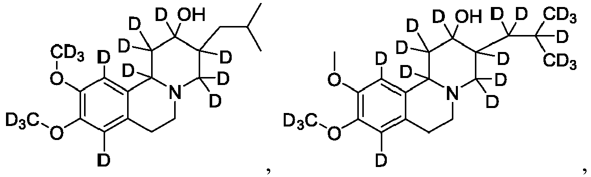 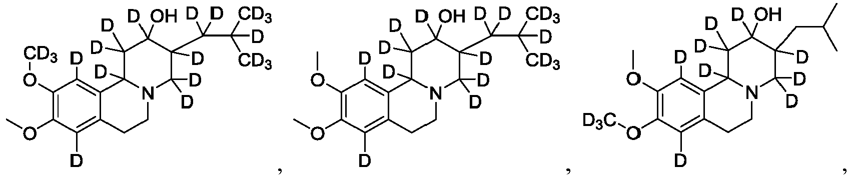 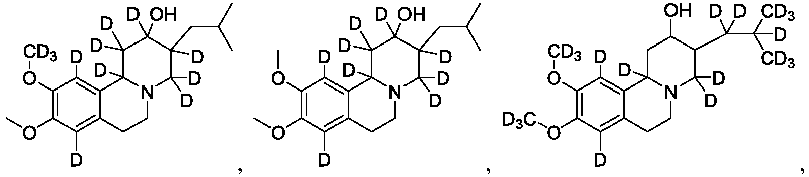 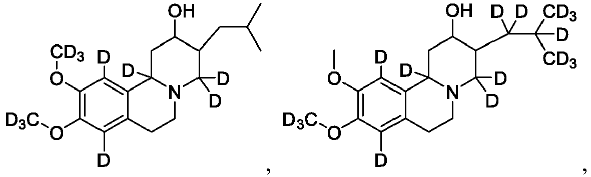 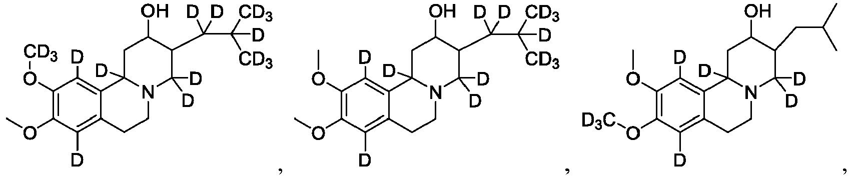 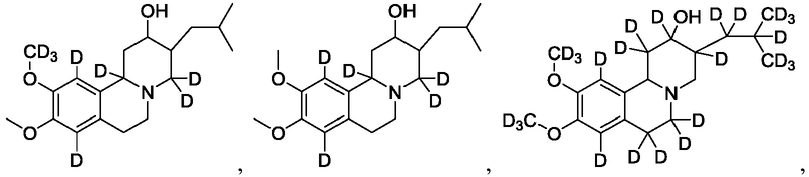 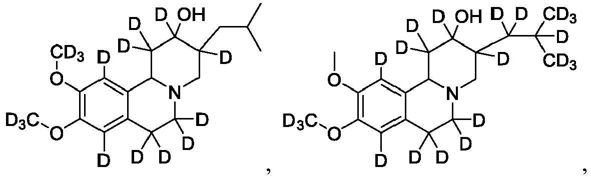 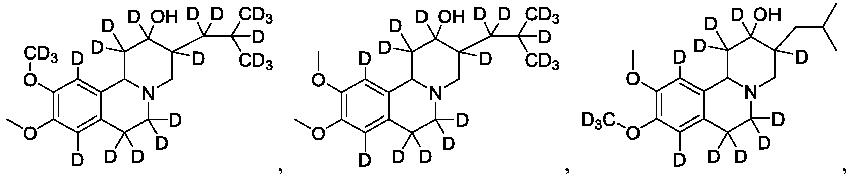 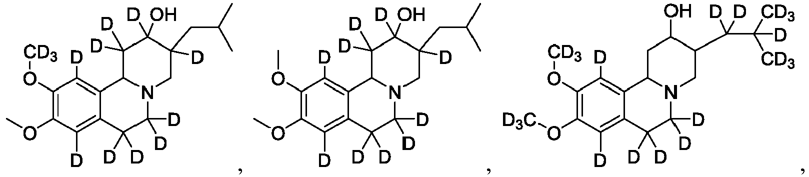 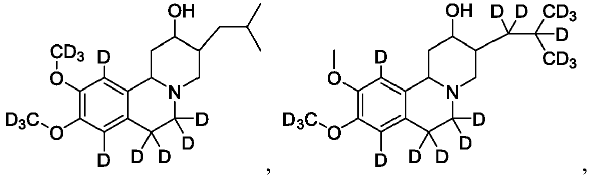 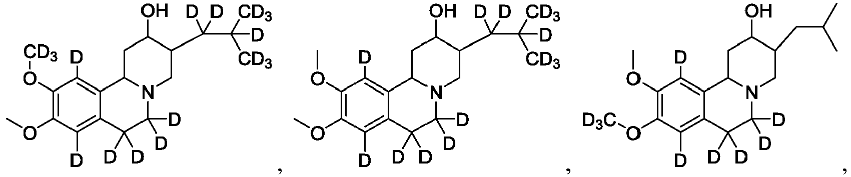 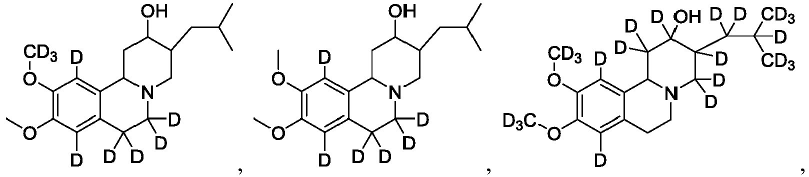 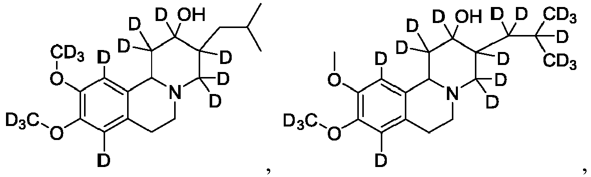 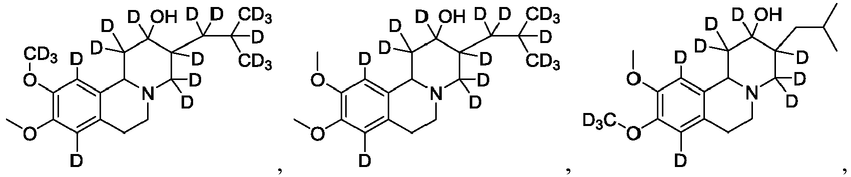 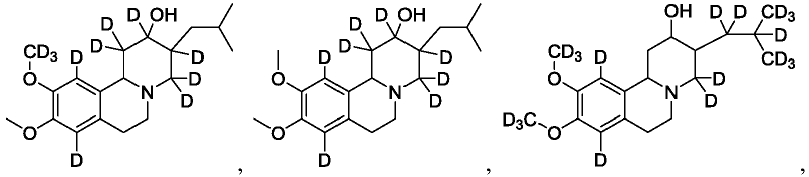 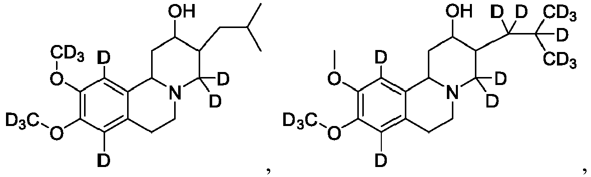 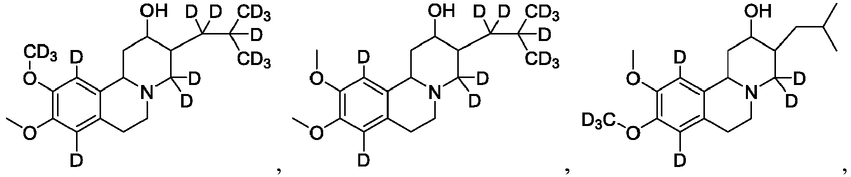 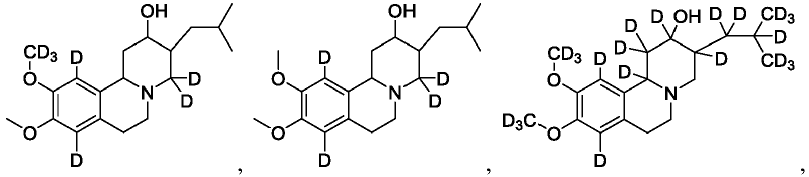 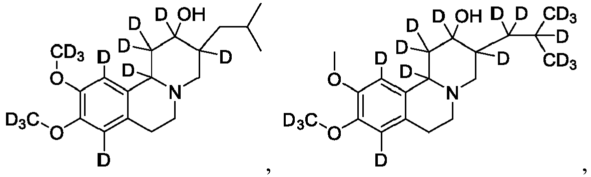 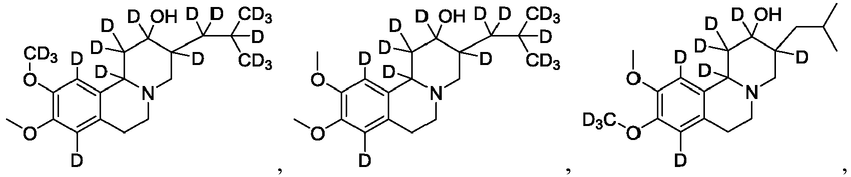 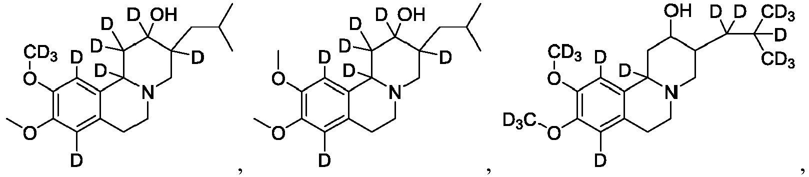 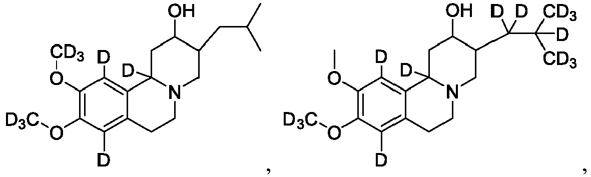 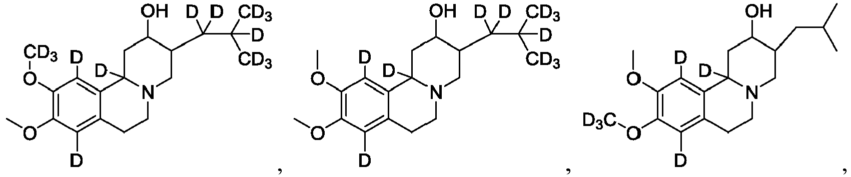 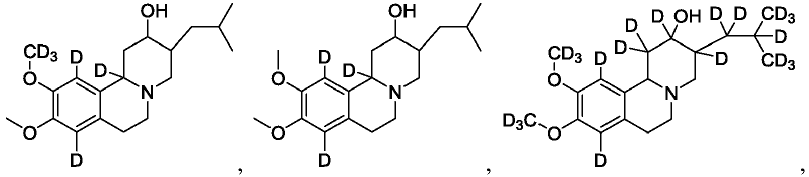 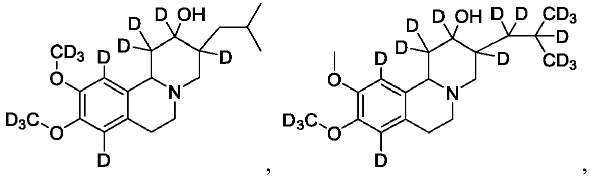 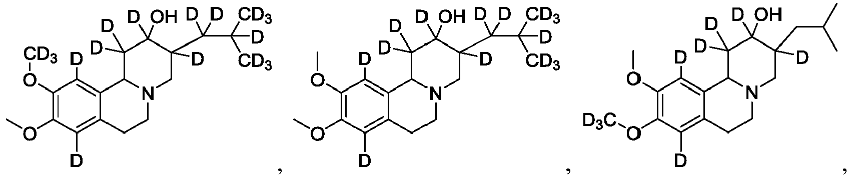 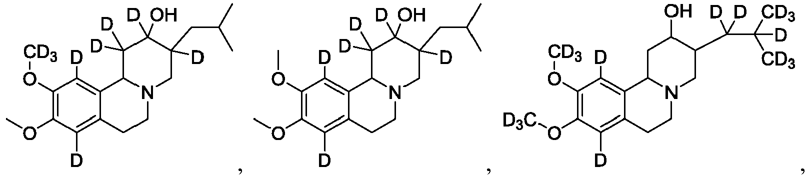 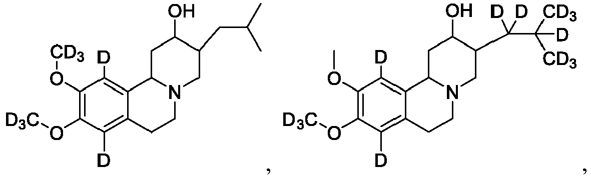 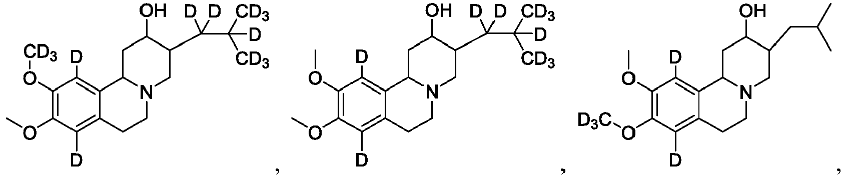 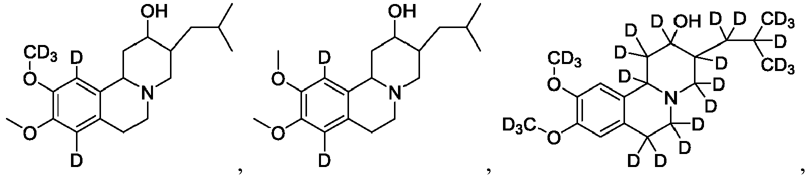 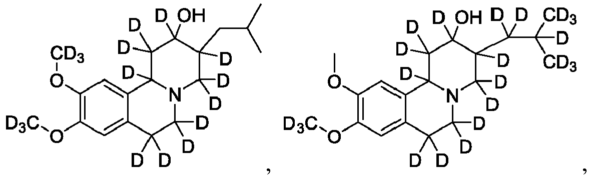 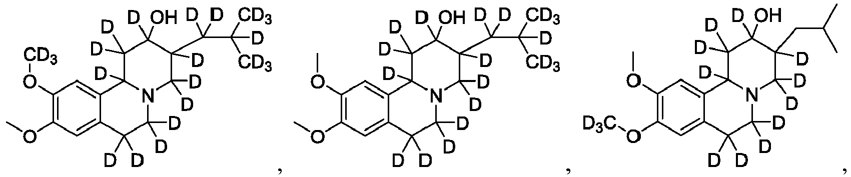 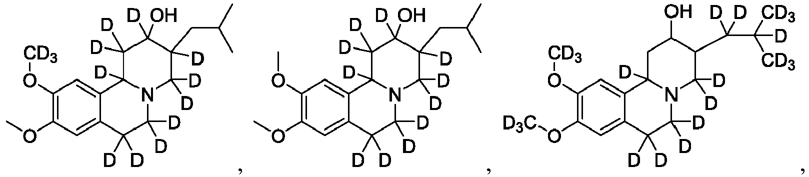 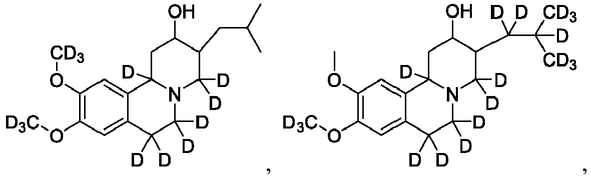 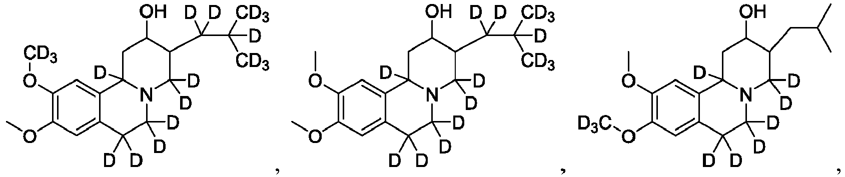 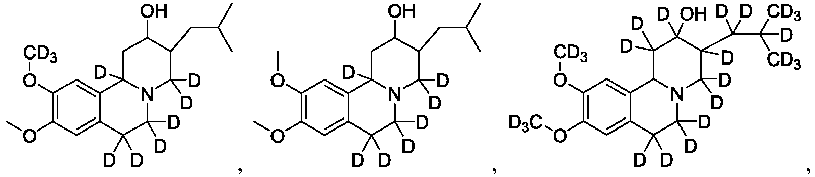 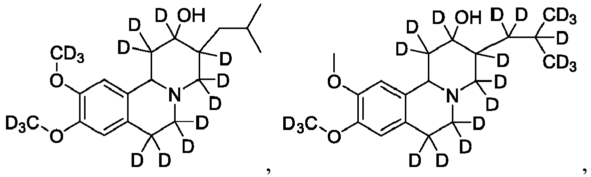 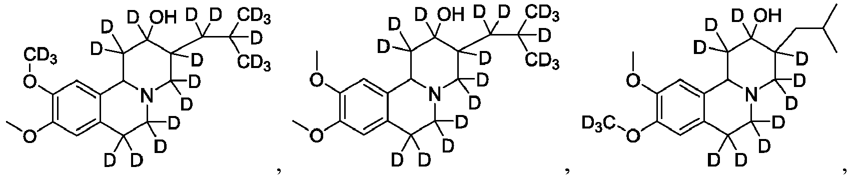 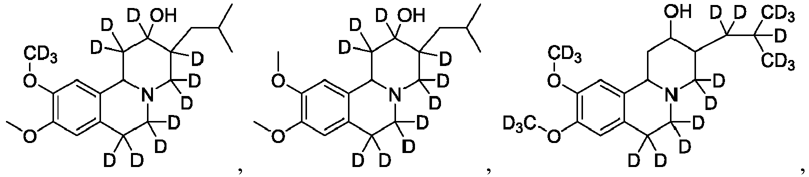 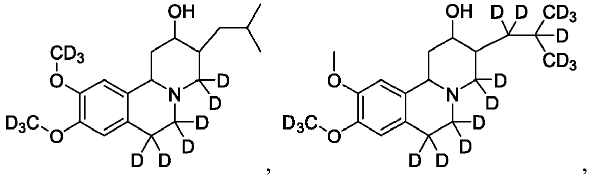 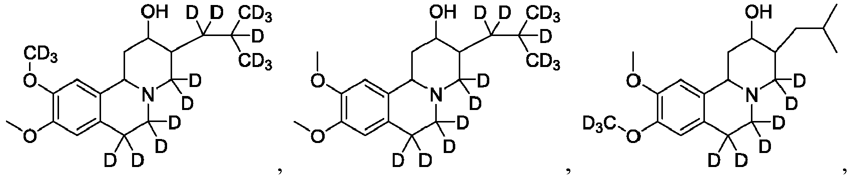 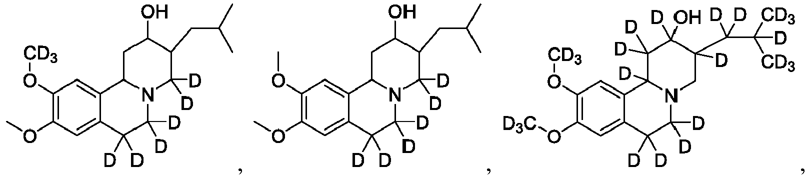 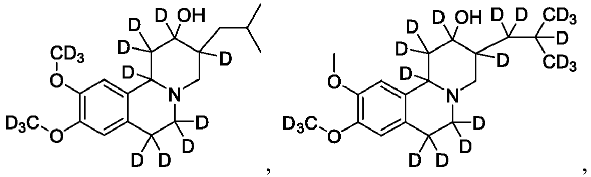 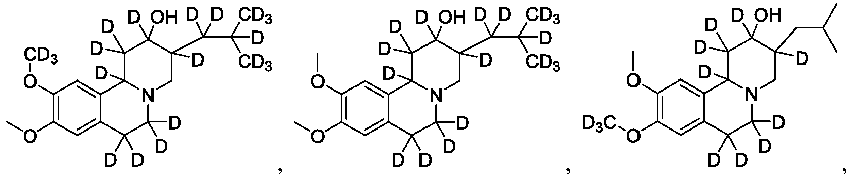 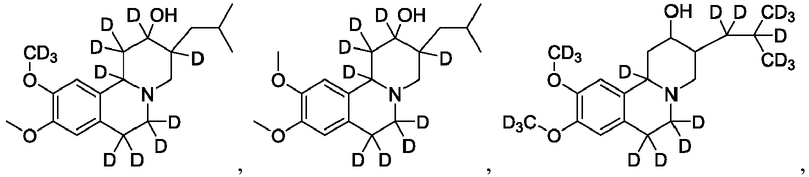 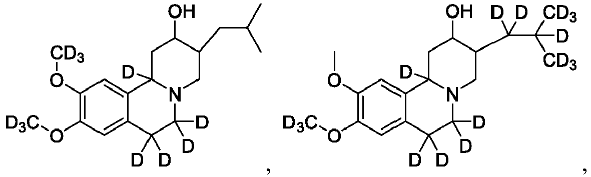 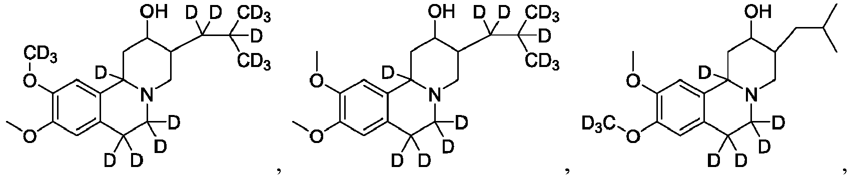 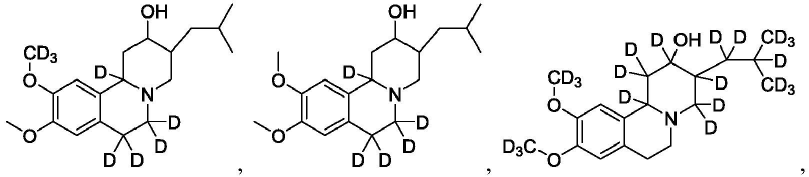 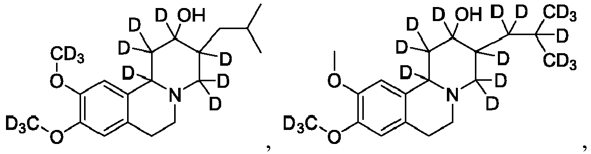 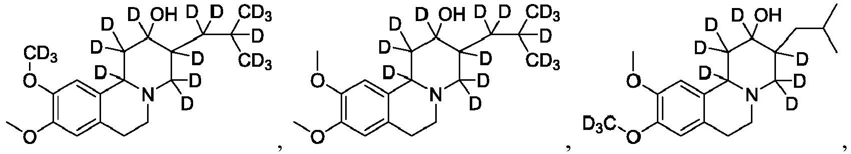 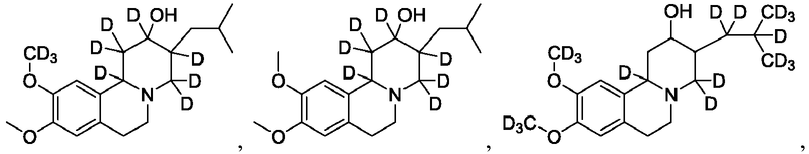 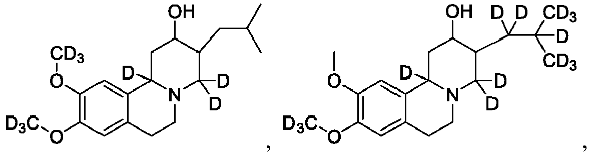 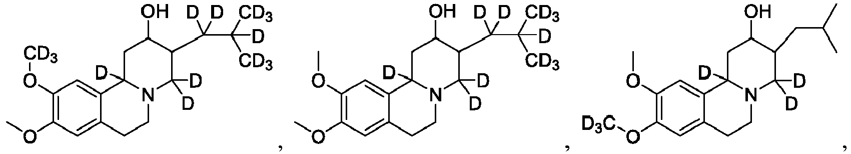 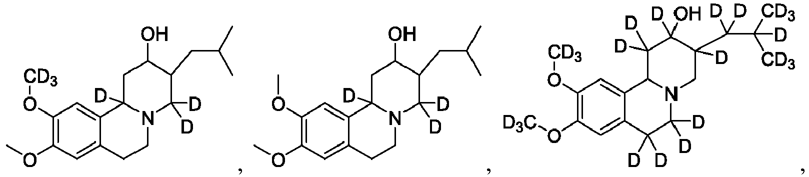 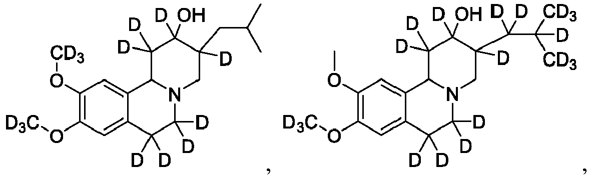 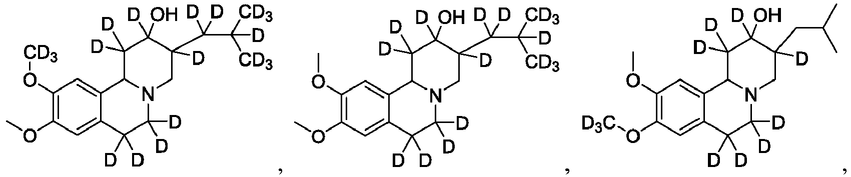 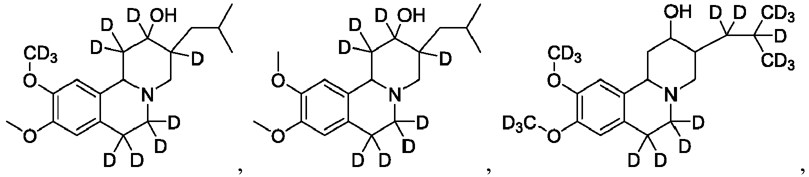 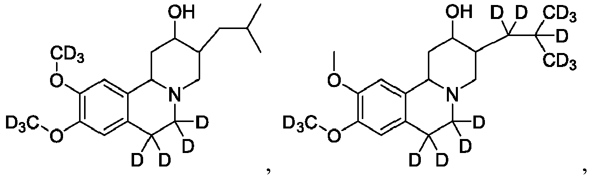 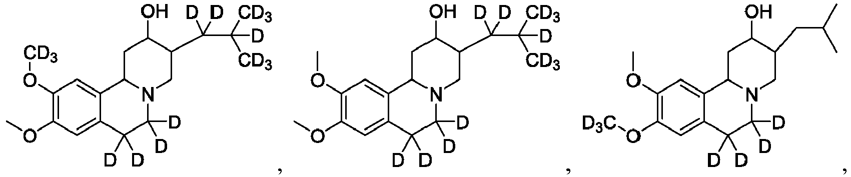 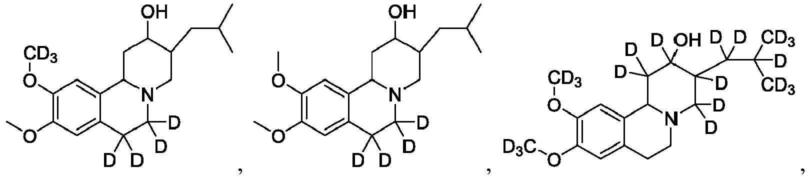 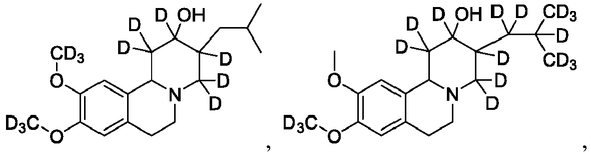 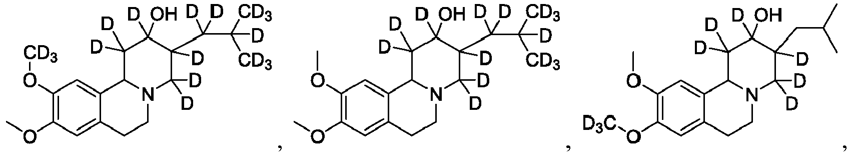 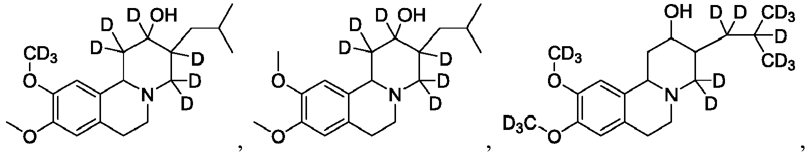 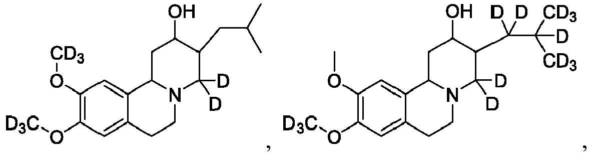 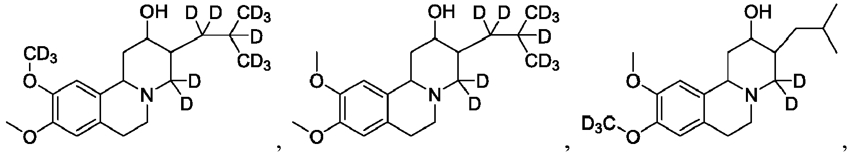 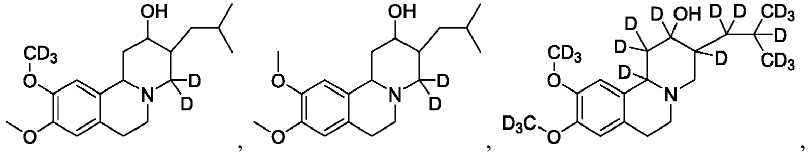 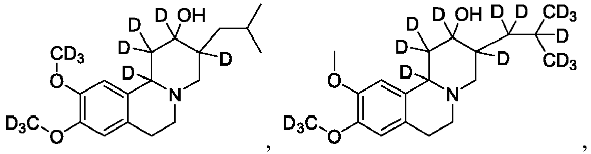 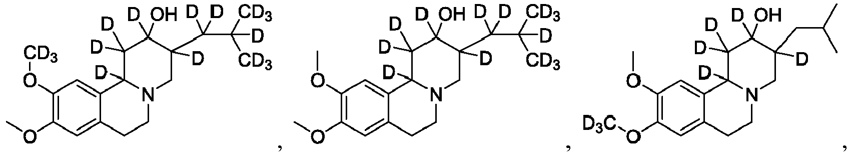 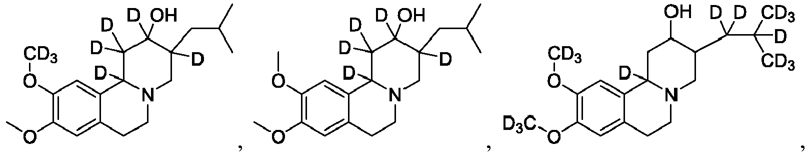 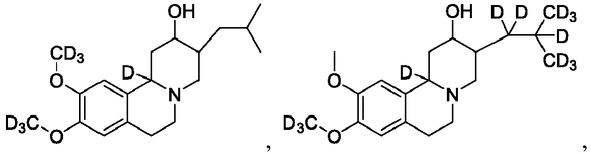 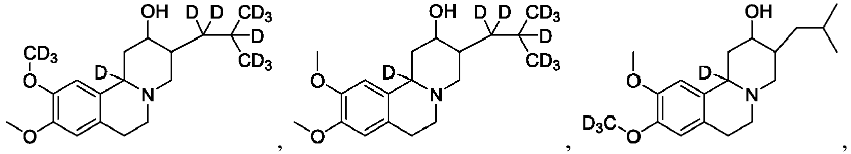 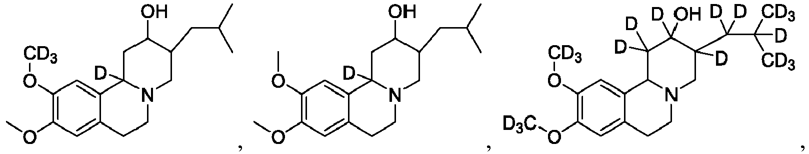 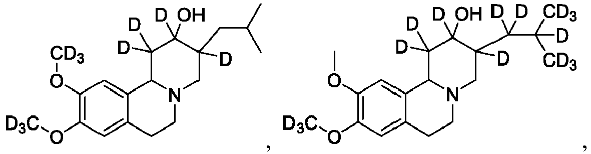 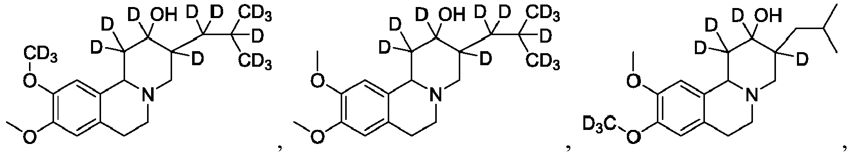 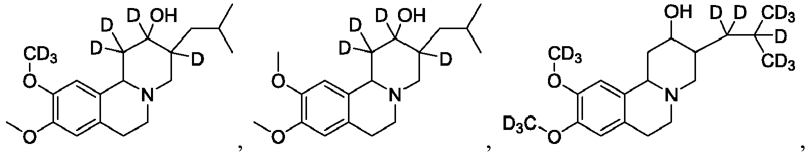 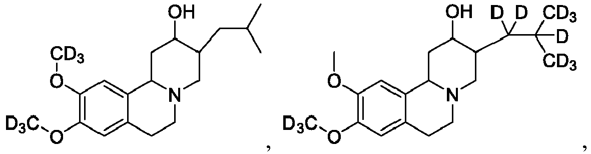 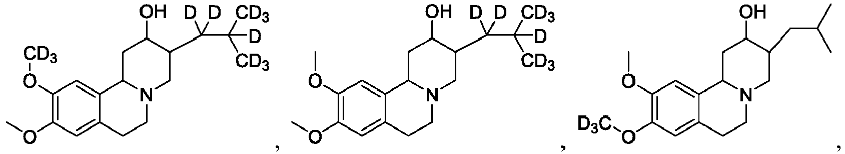 and

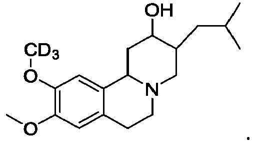

The following compounds can generally be made using the methods described above. It is expected that these compounds when made will have activity similar to those described in the examples above. 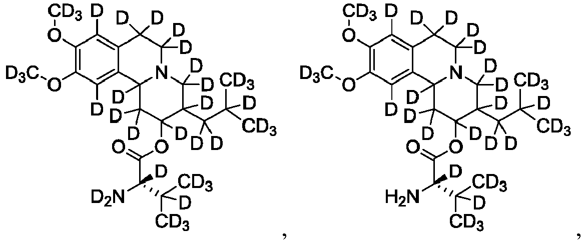 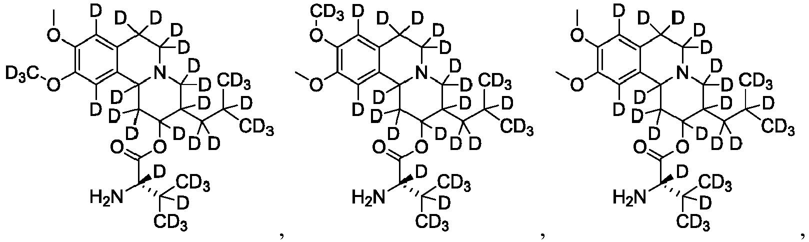 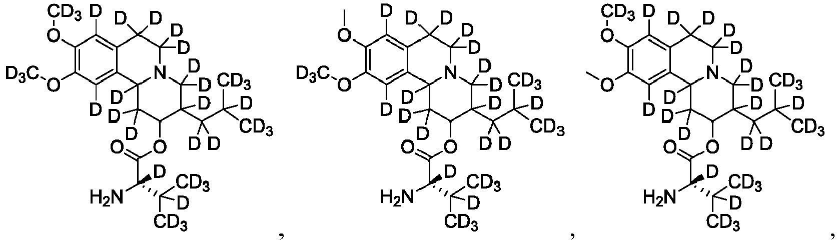 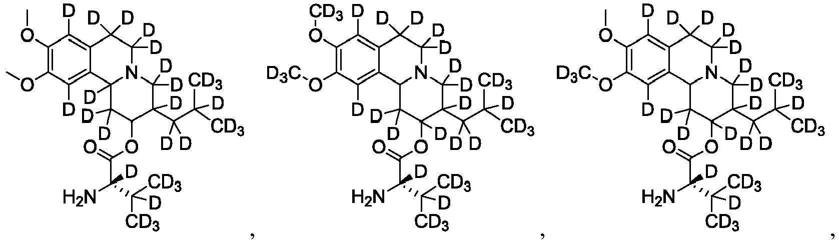 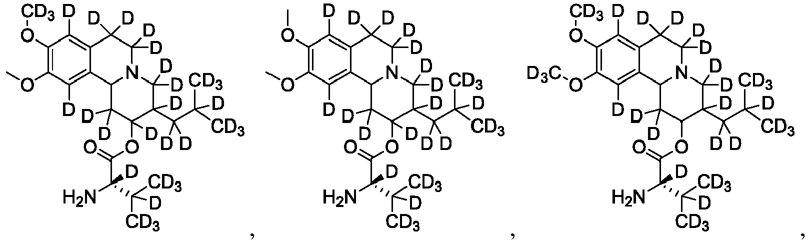 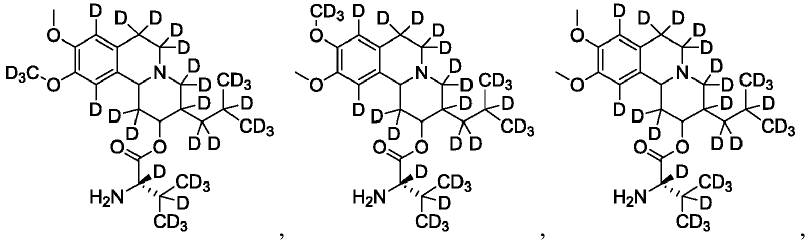 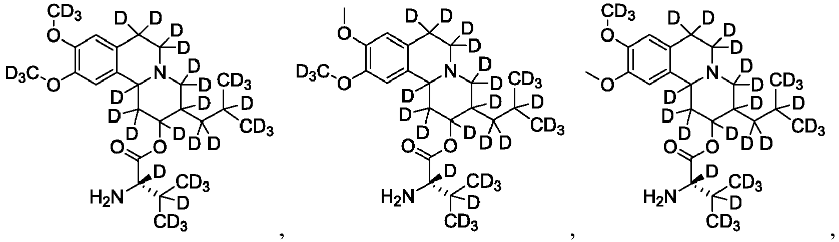 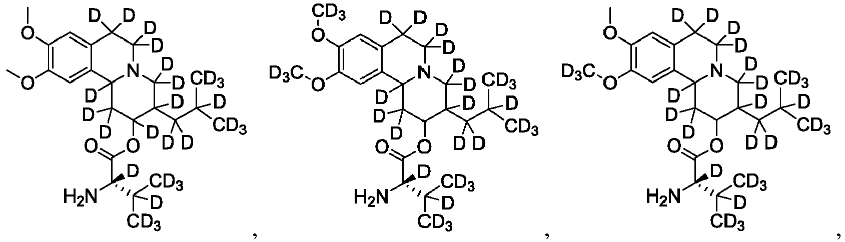 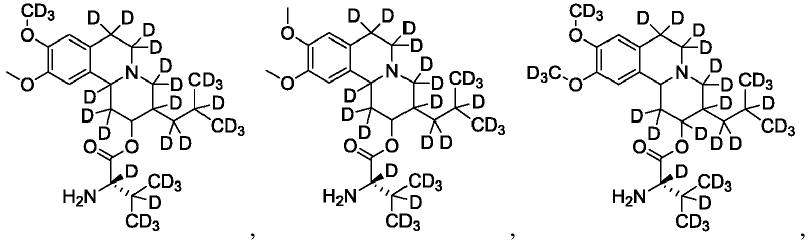 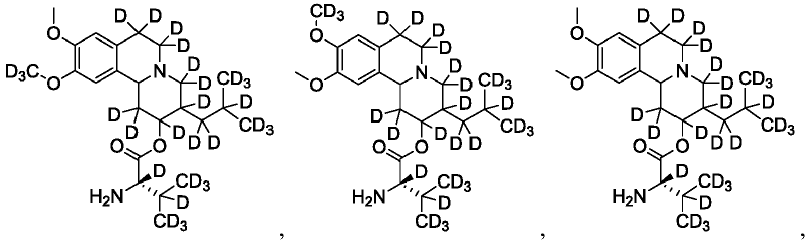 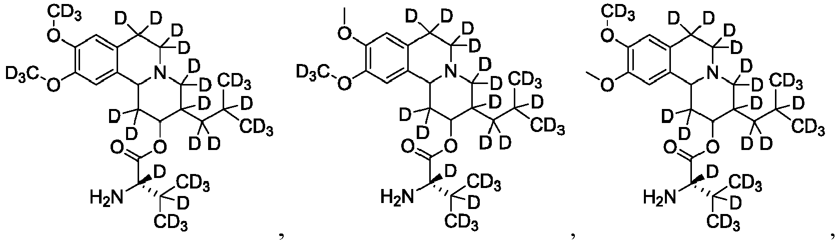 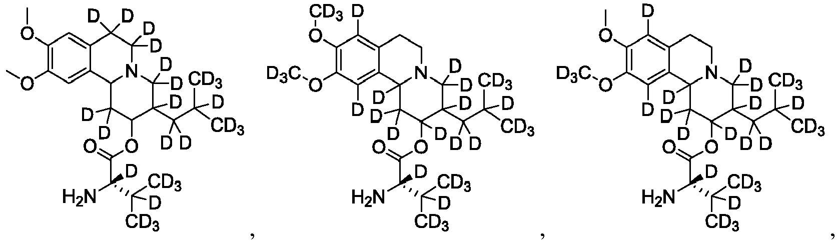 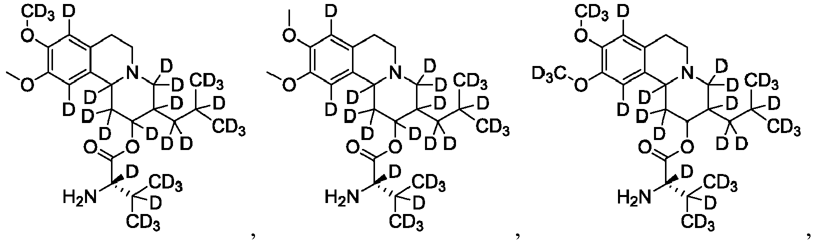 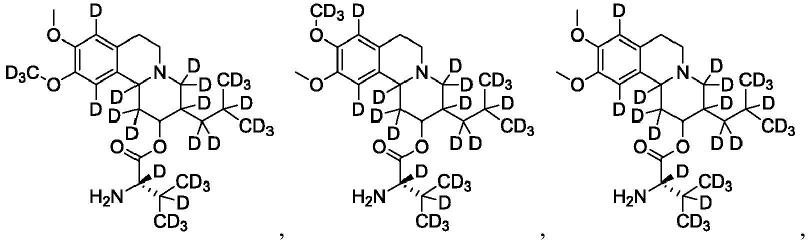 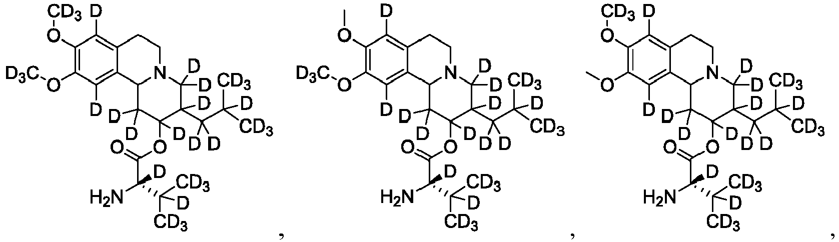 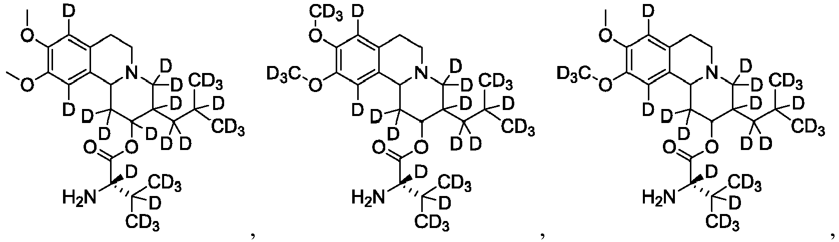 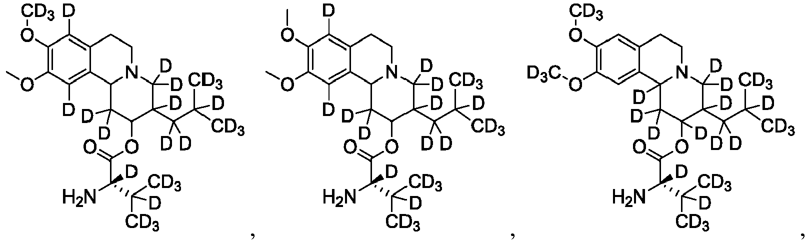 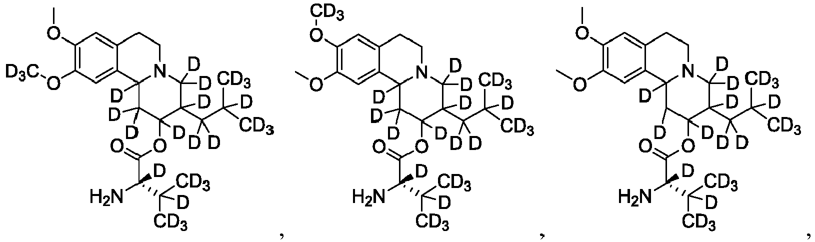 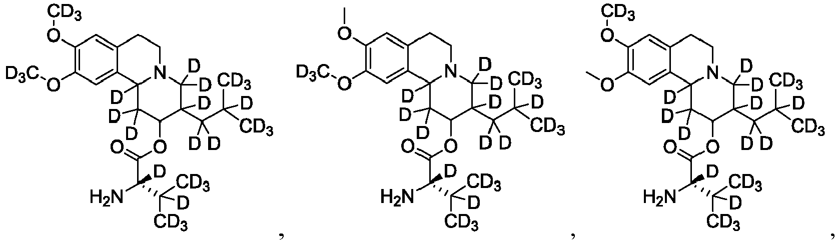 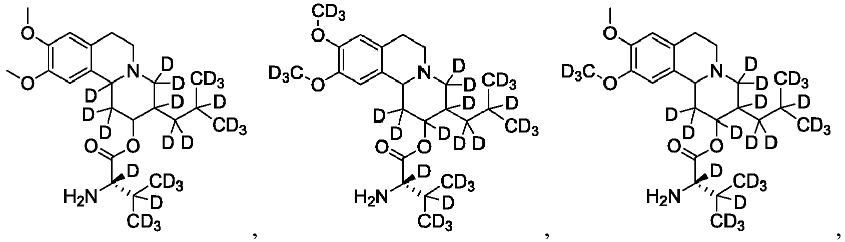 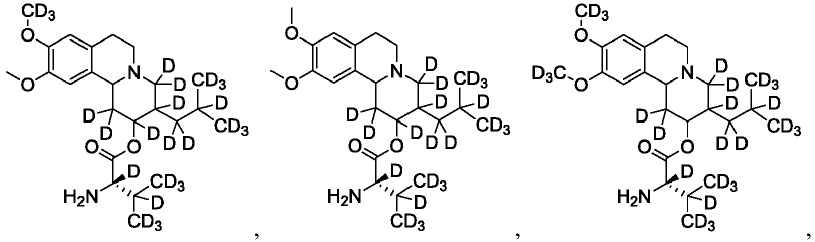 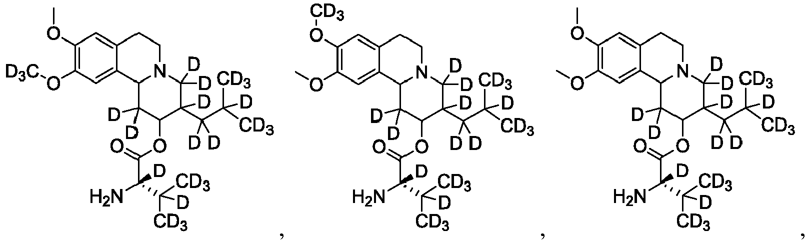 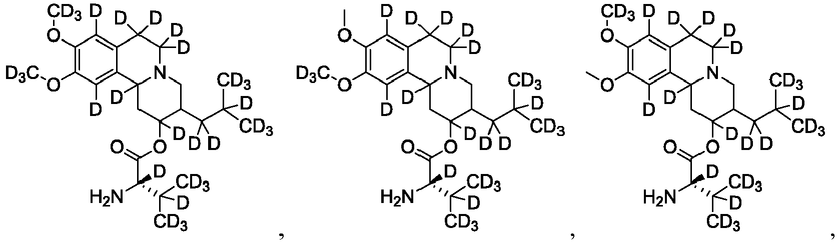 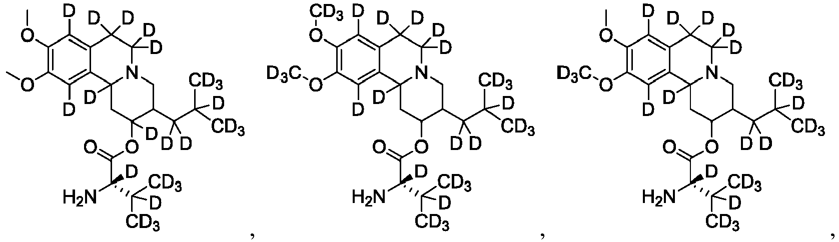 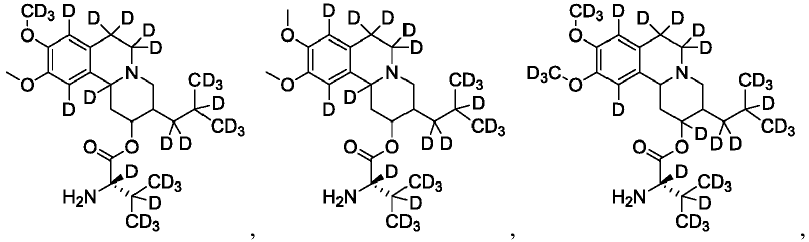 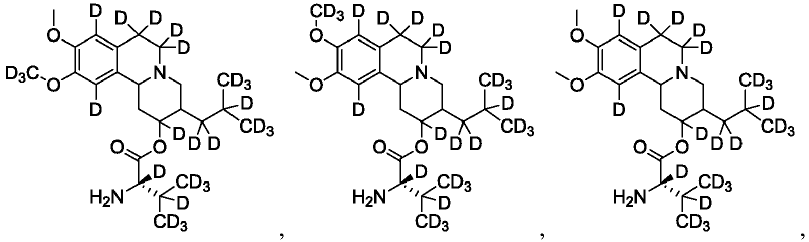 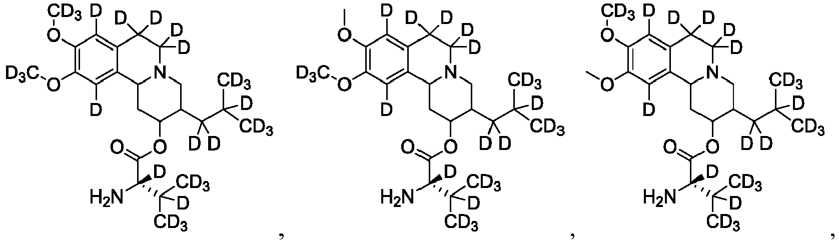 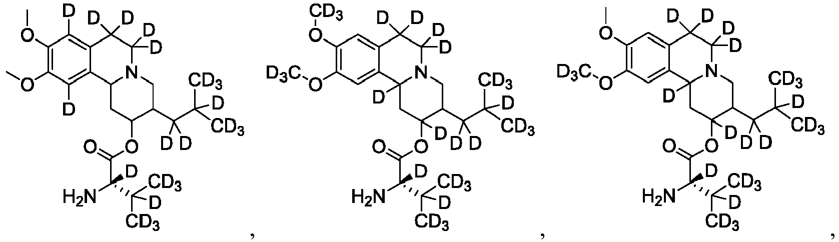 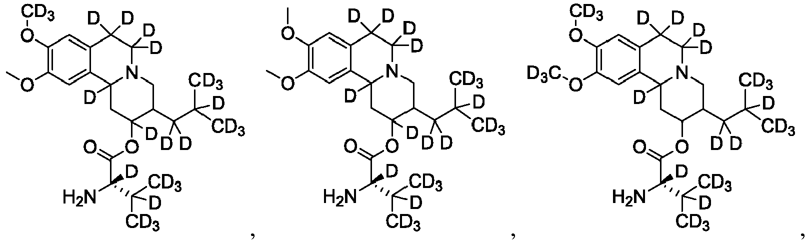 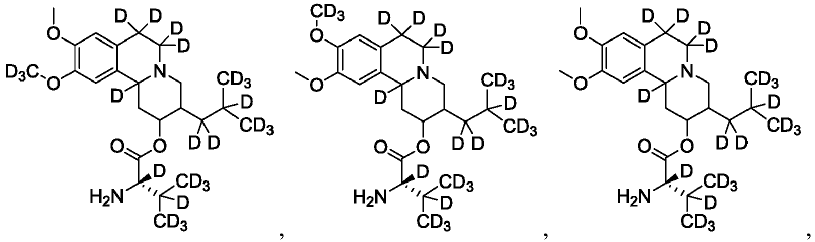 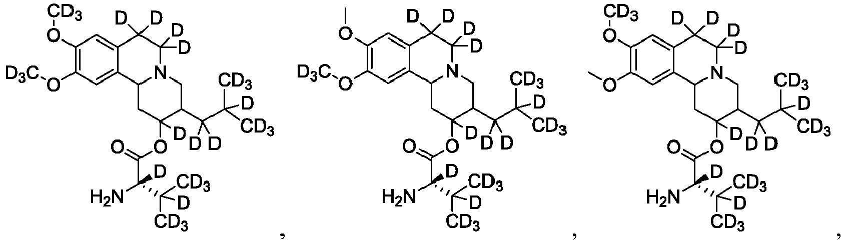 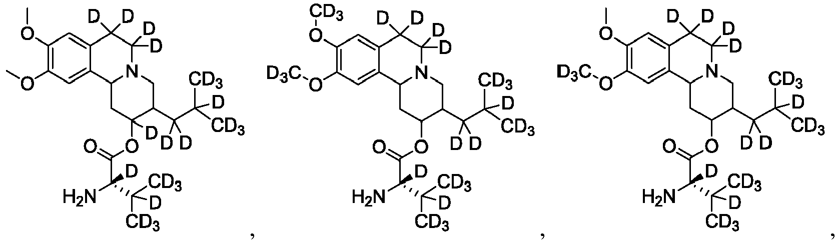 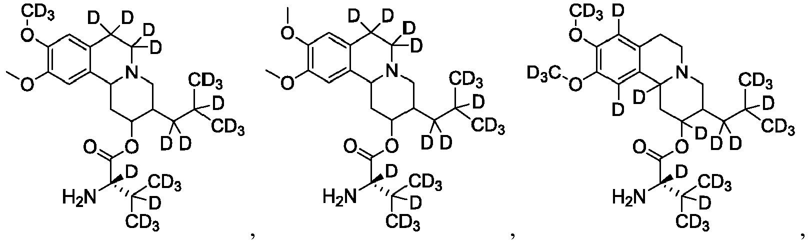 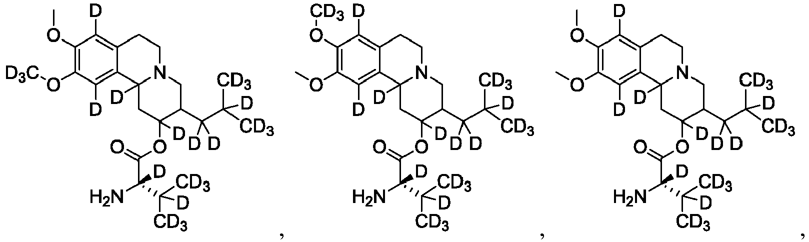 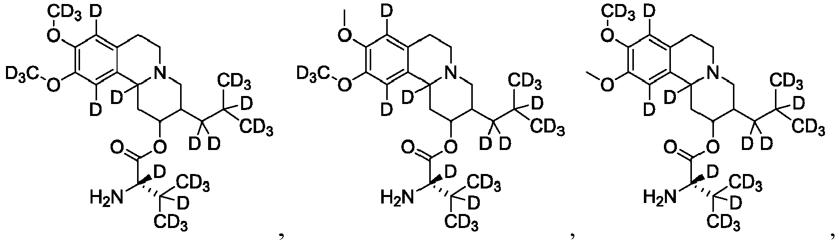 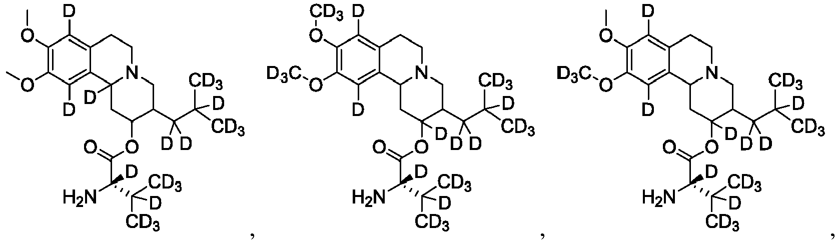 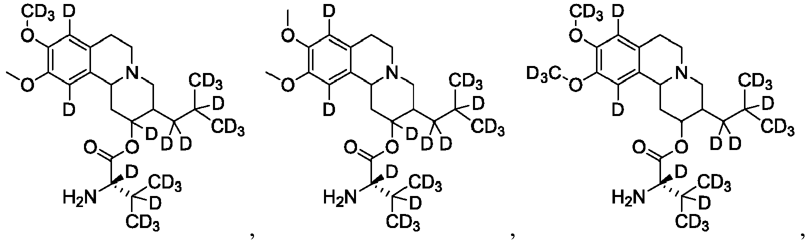 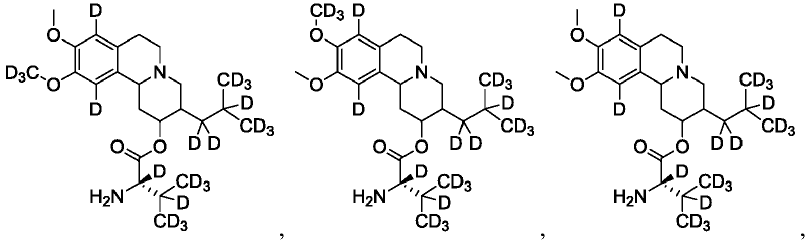 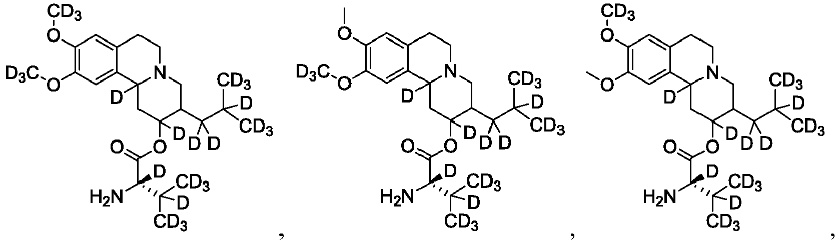 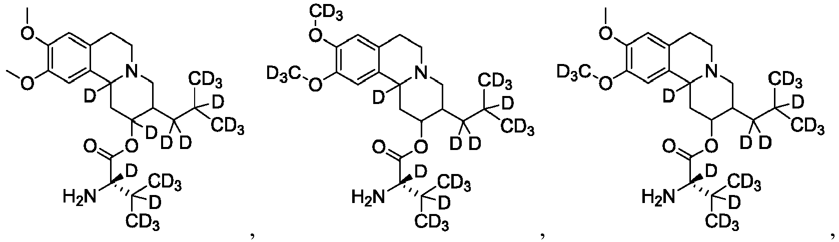 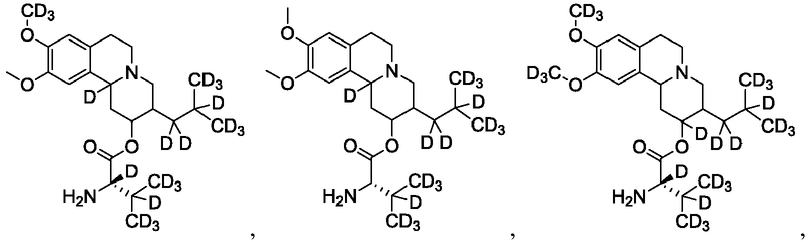 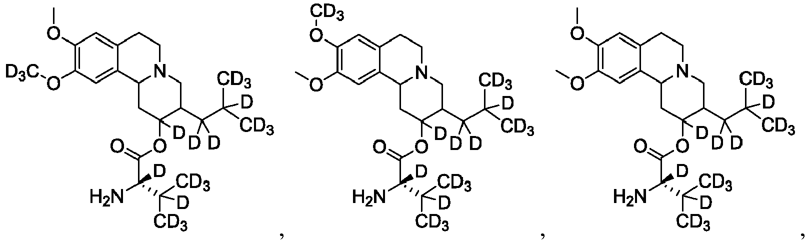 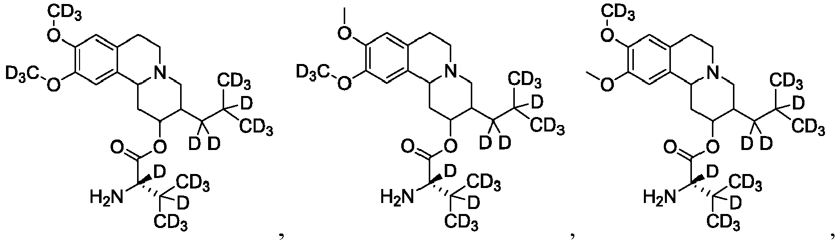 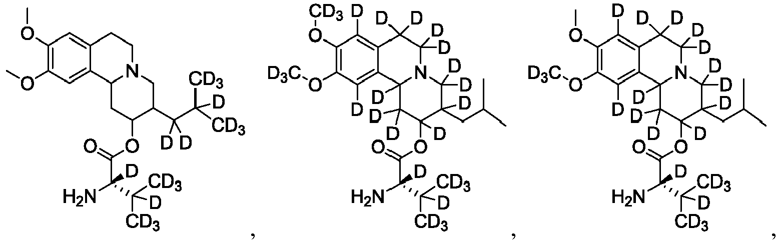 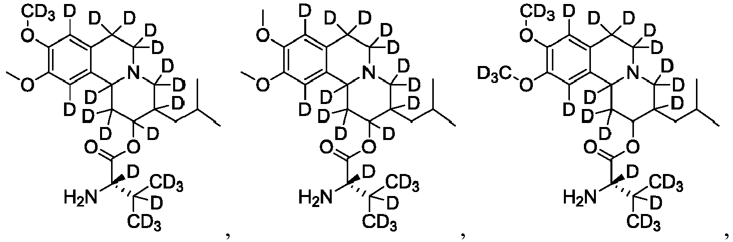 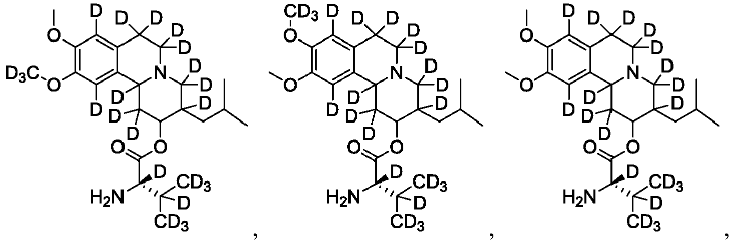 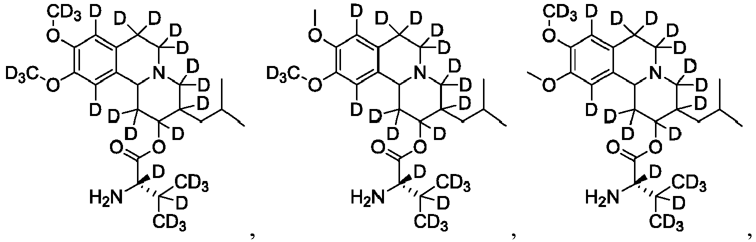 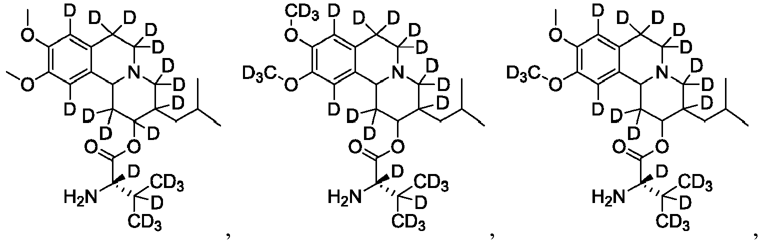 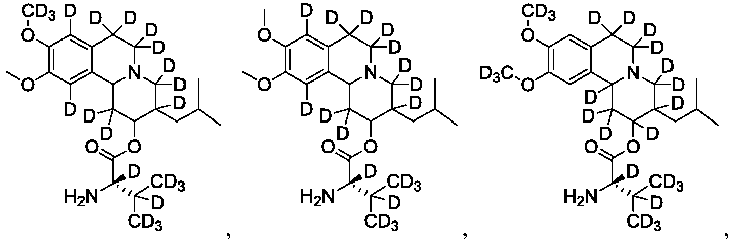 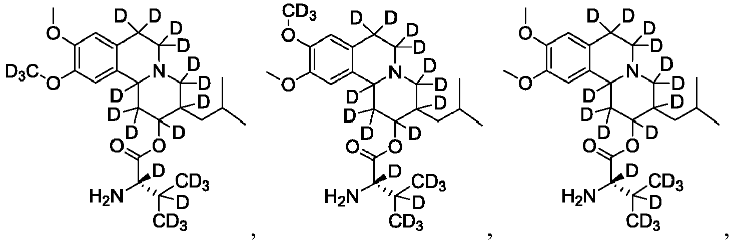 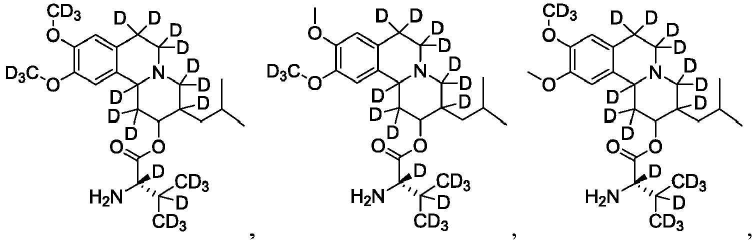 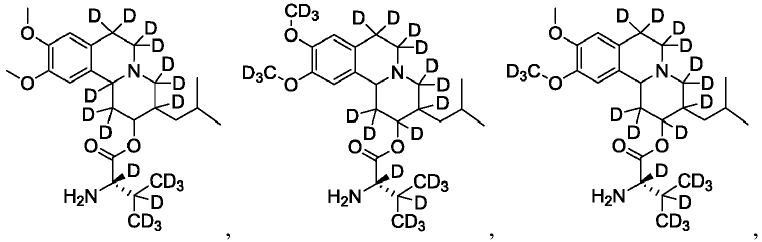 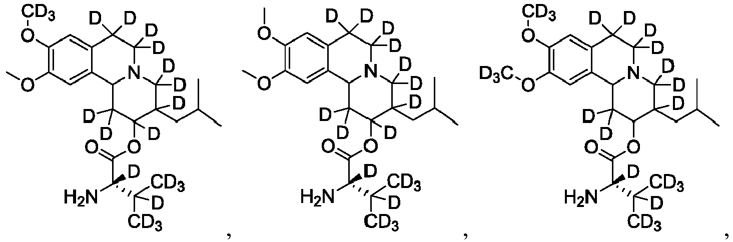 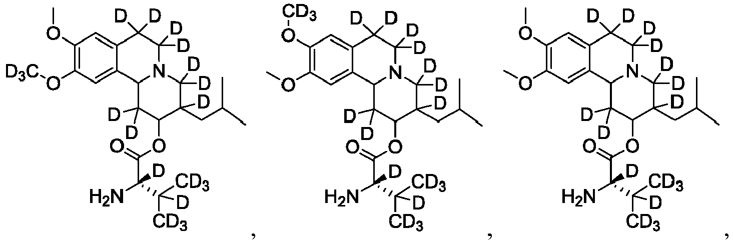 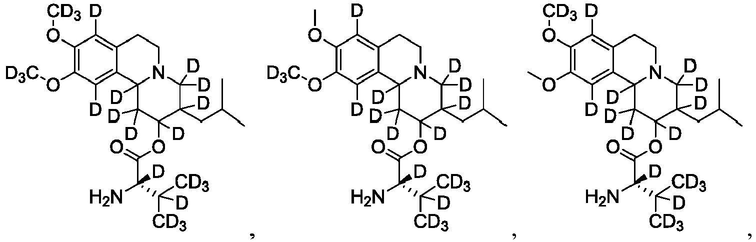 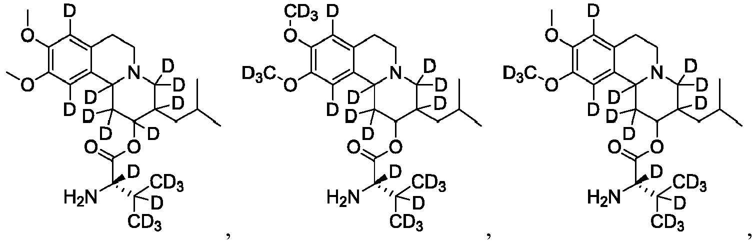 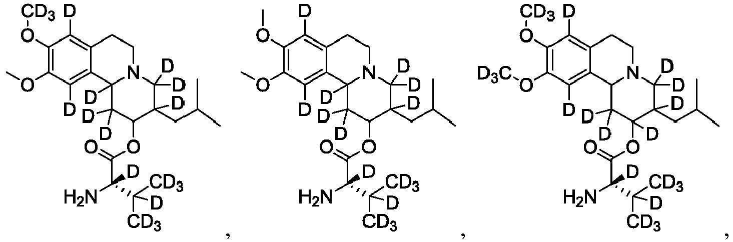 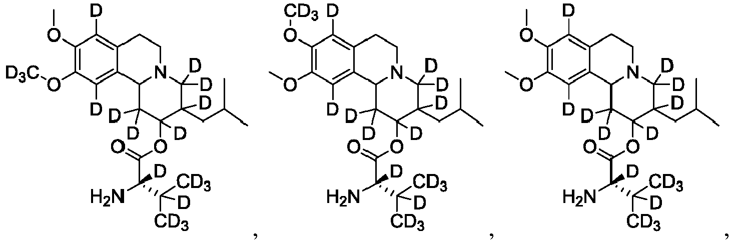 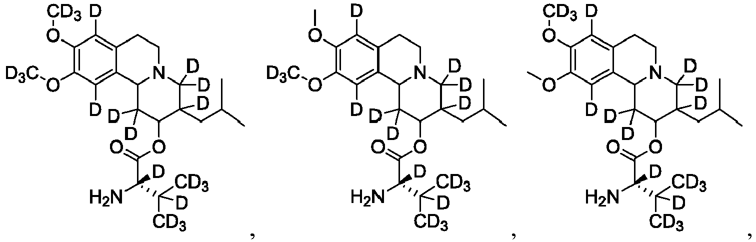 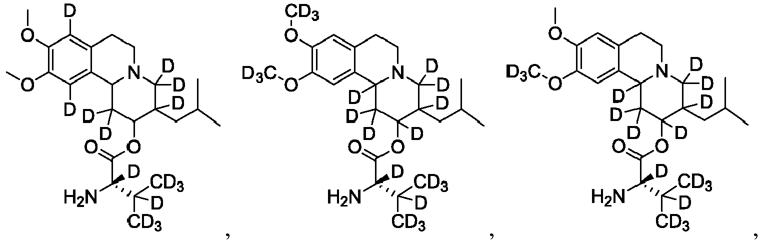 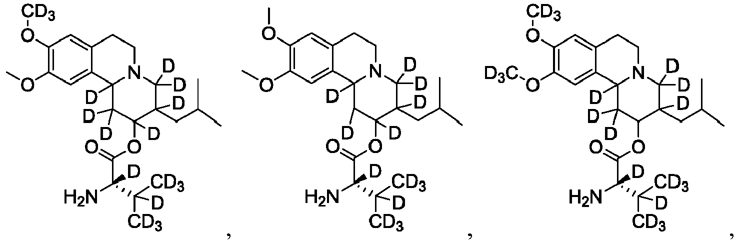 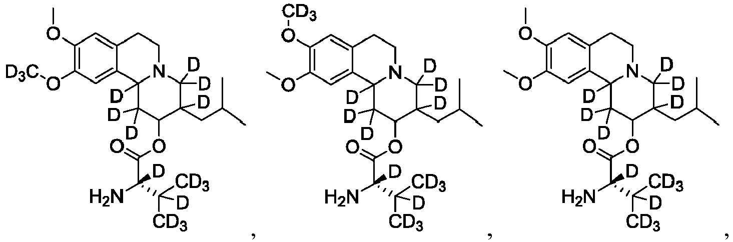 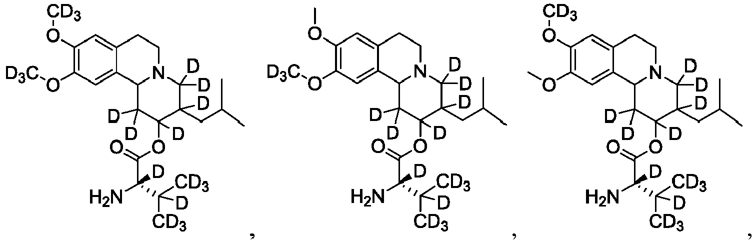 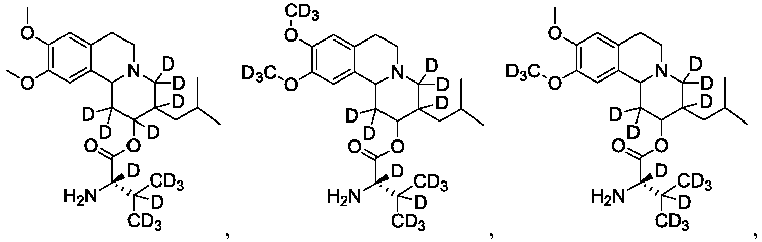 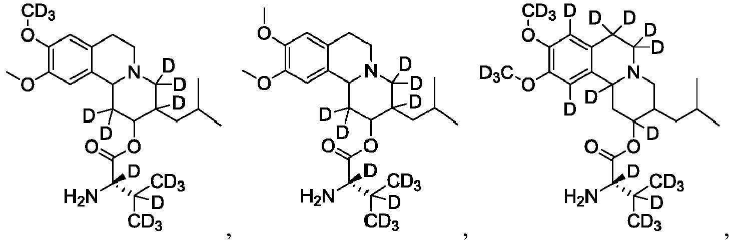 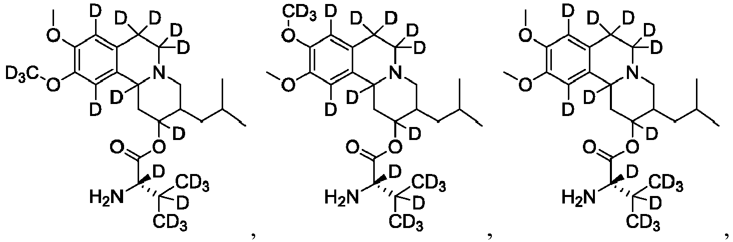 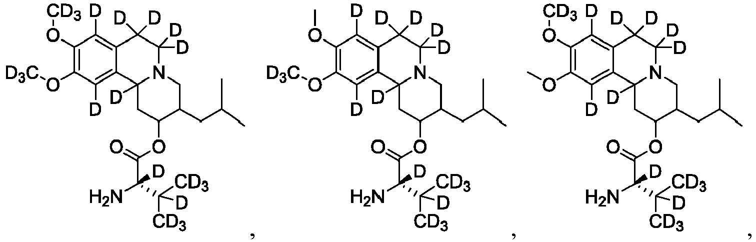 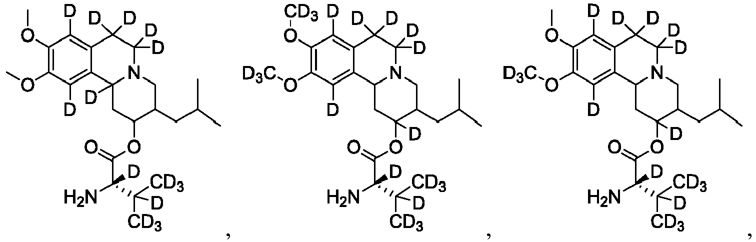 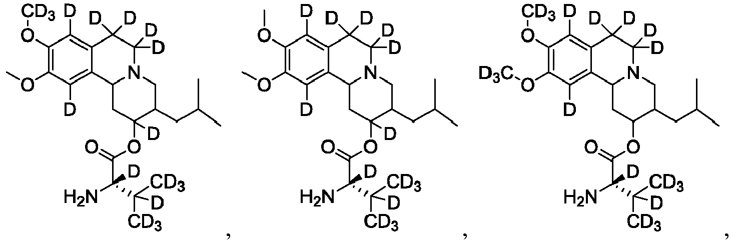 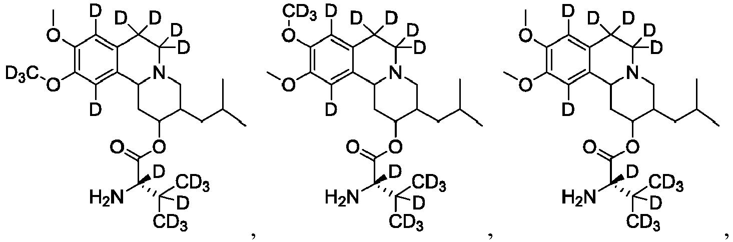 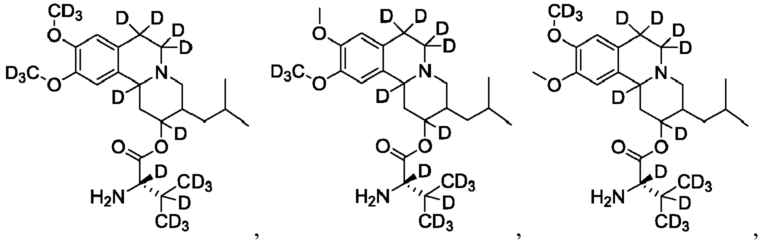 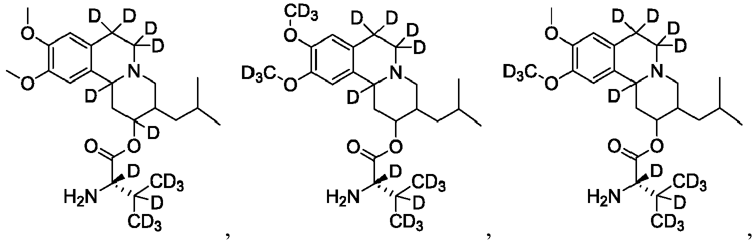 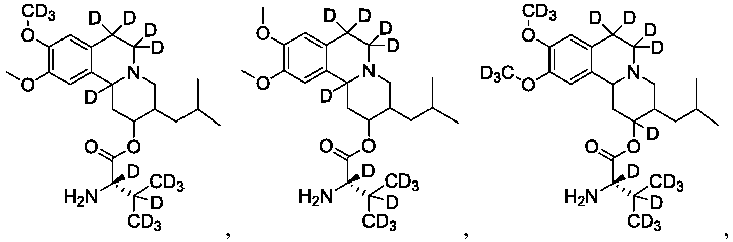 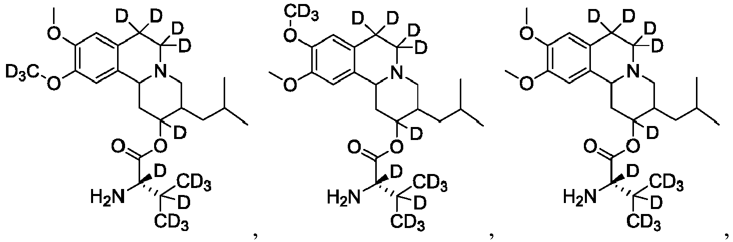 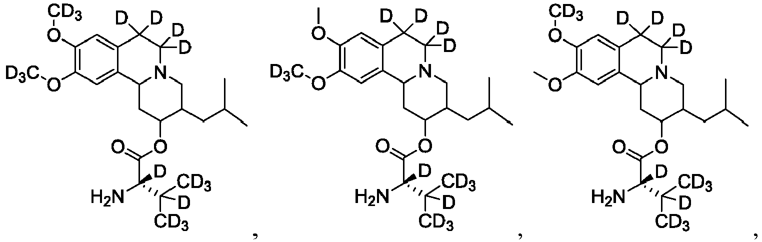 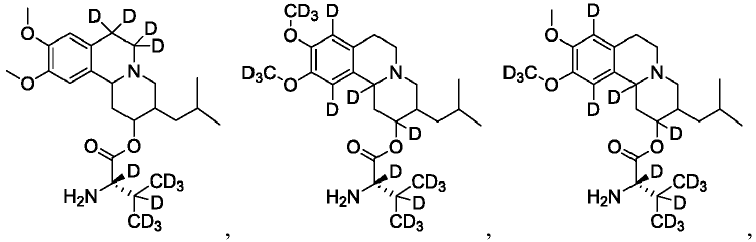 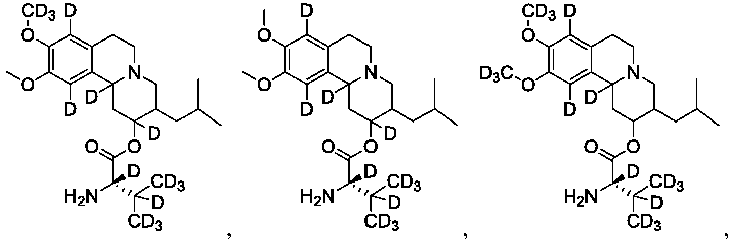 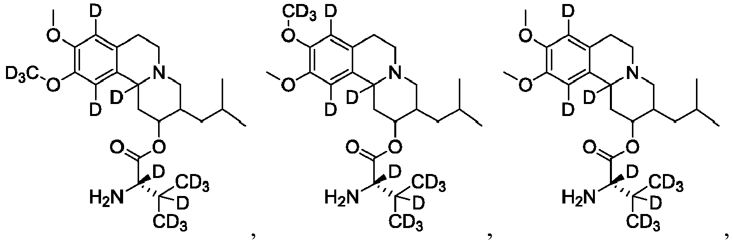 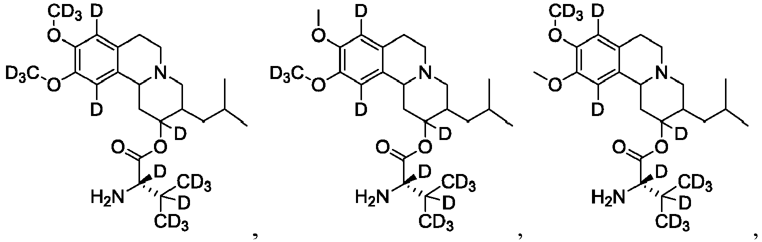 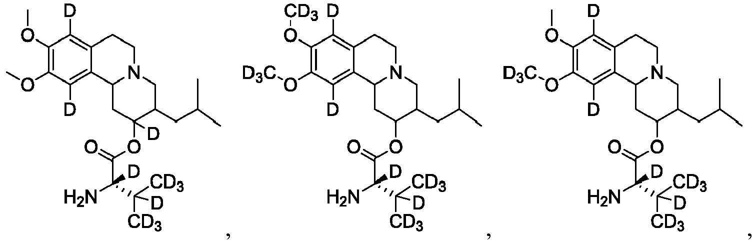 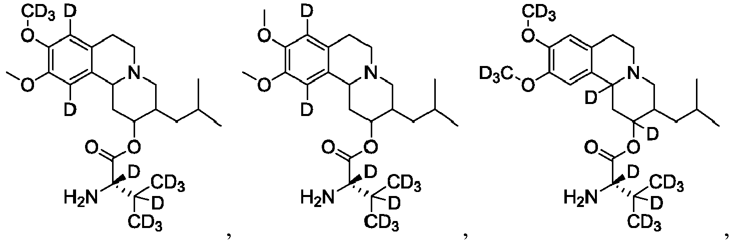 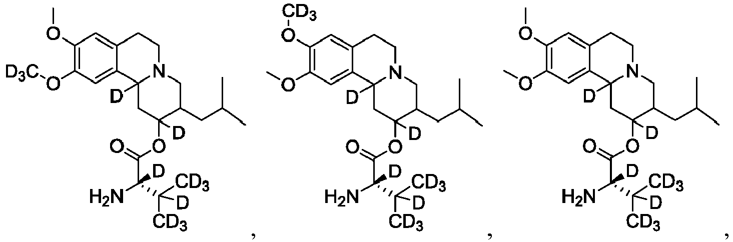 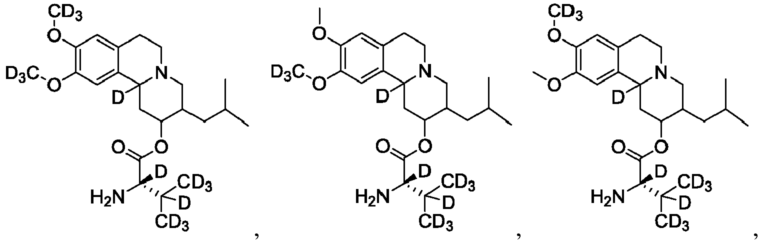 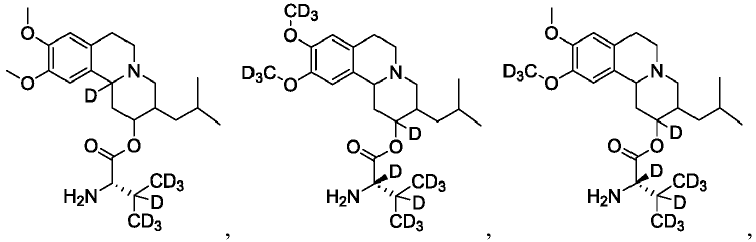 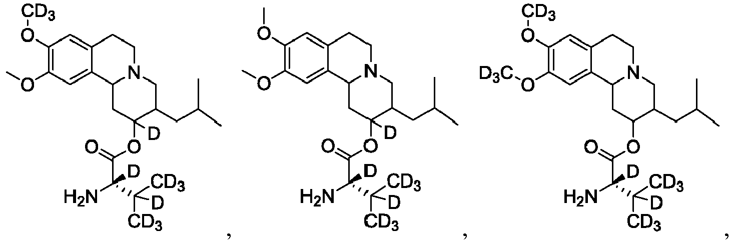 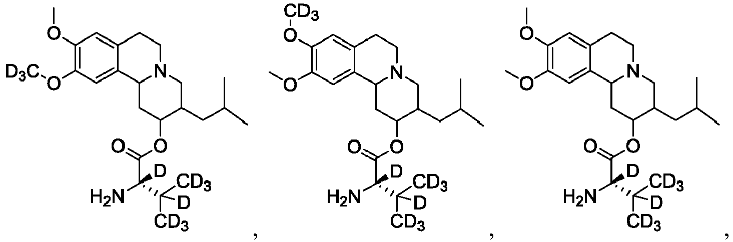 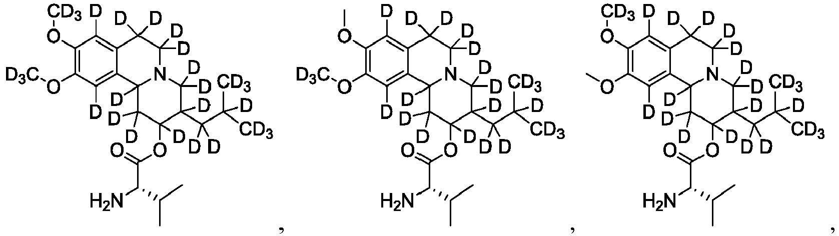 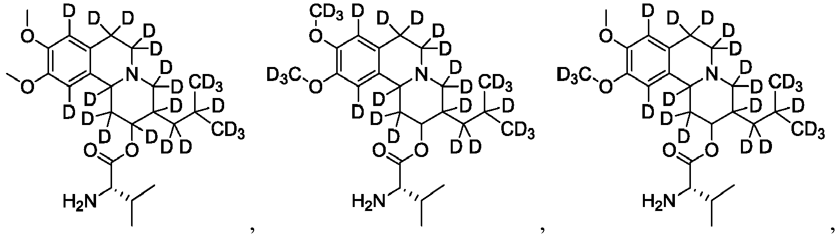 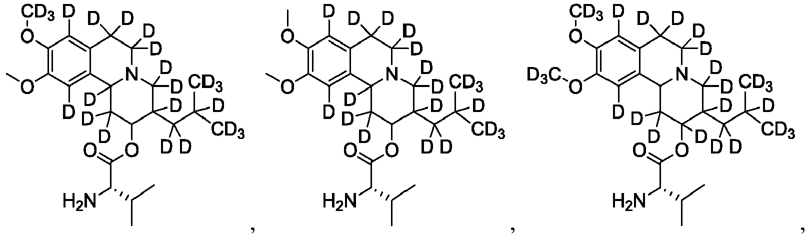 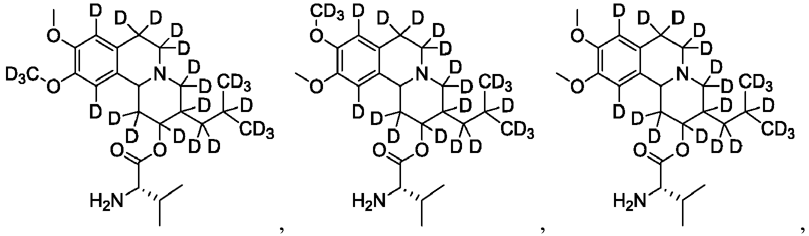 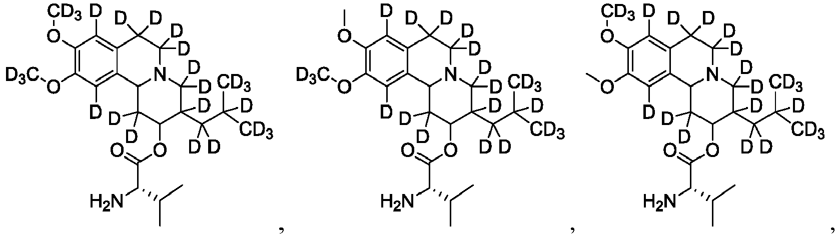 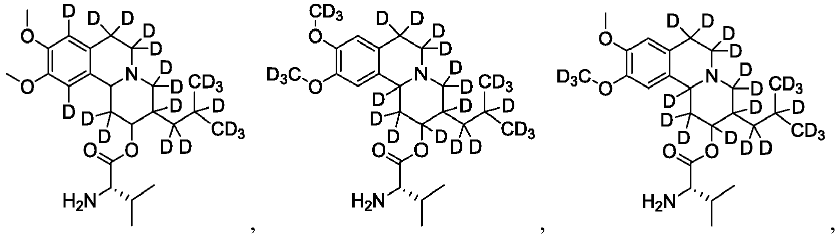 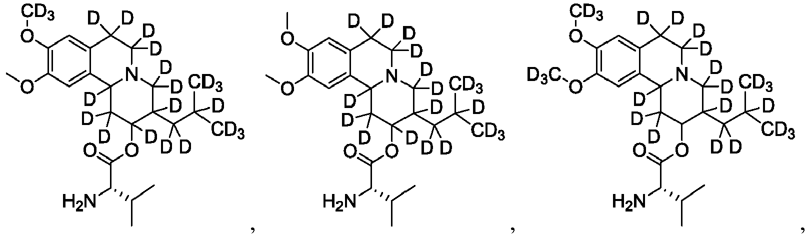 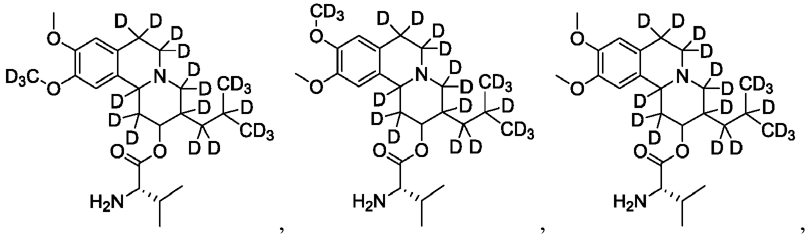 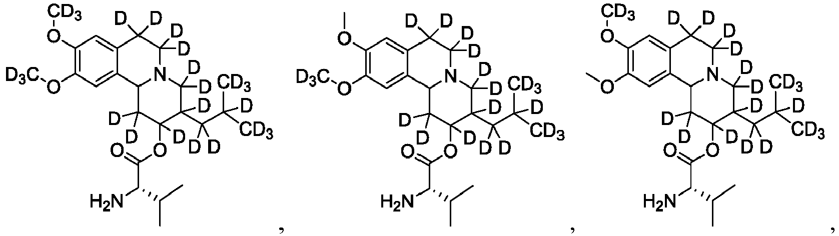 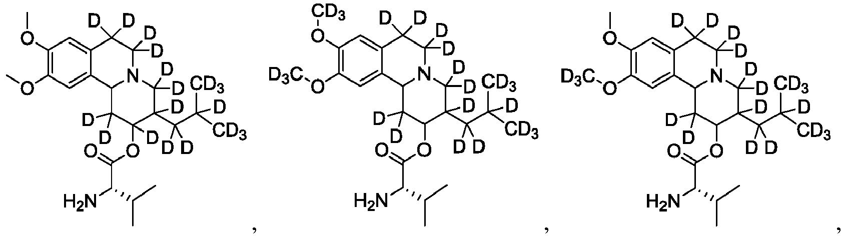 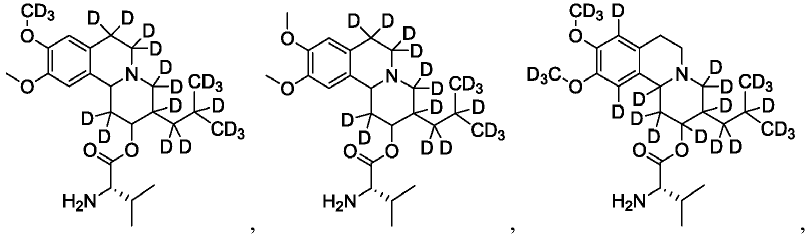 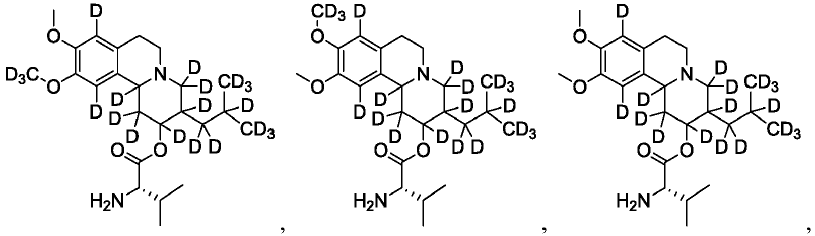 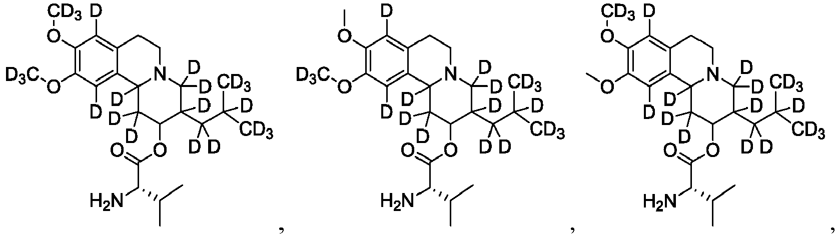 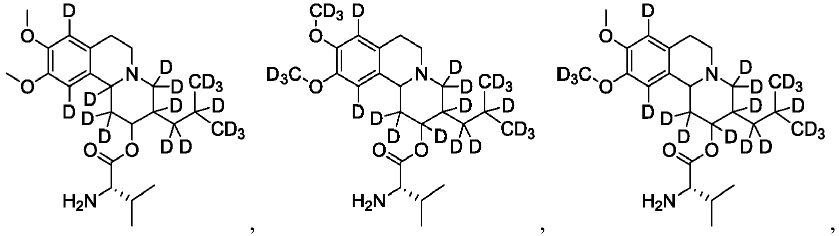 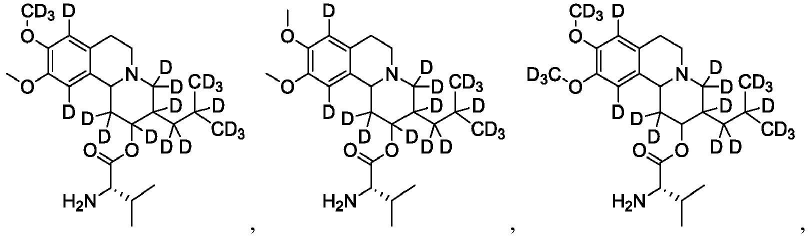 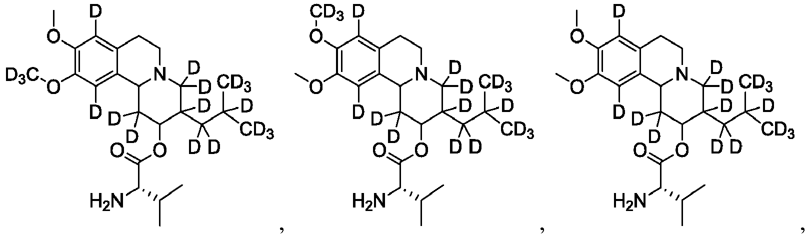 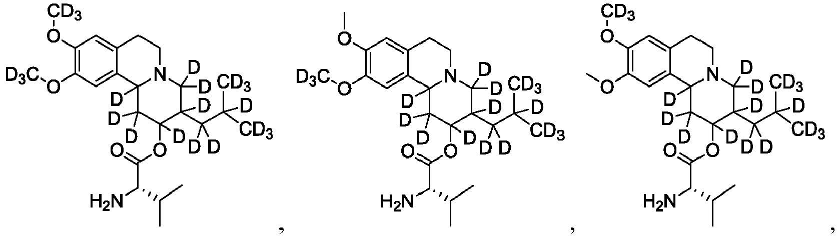 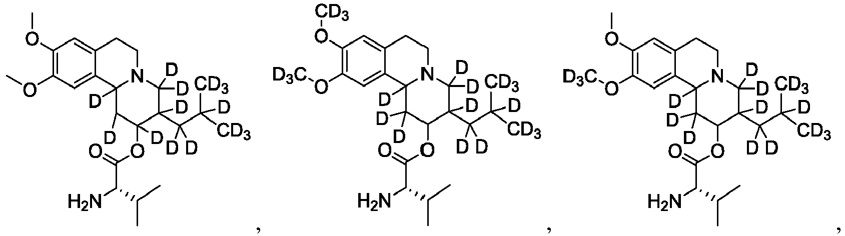 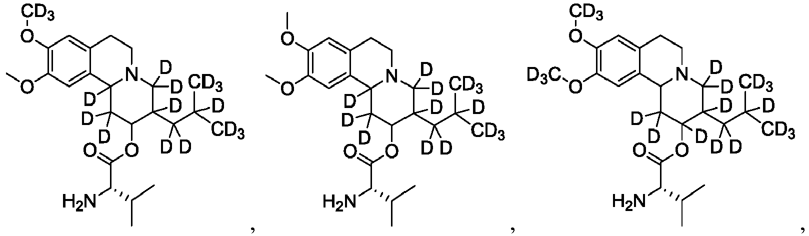 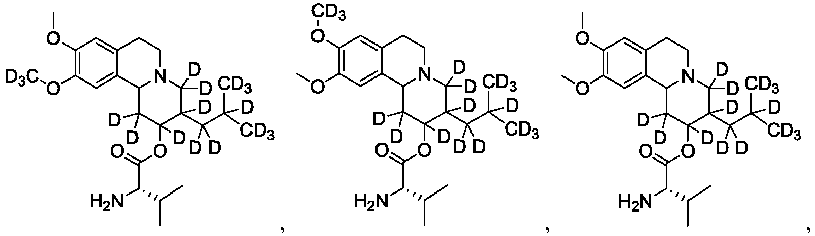 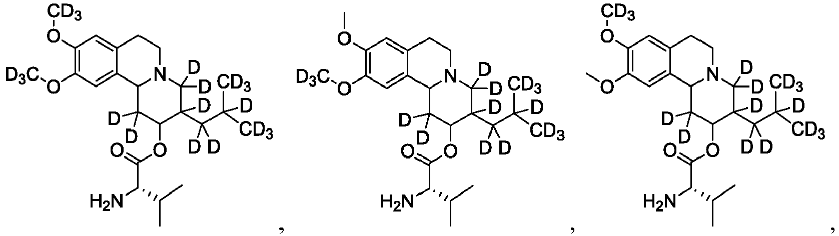 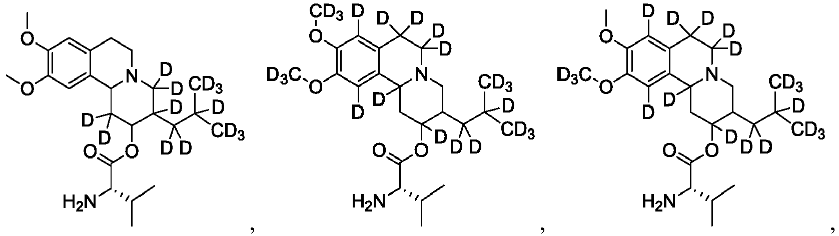 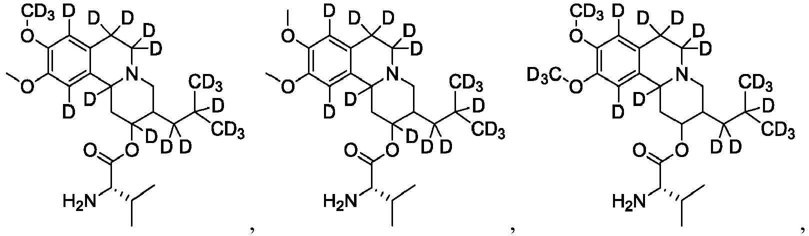 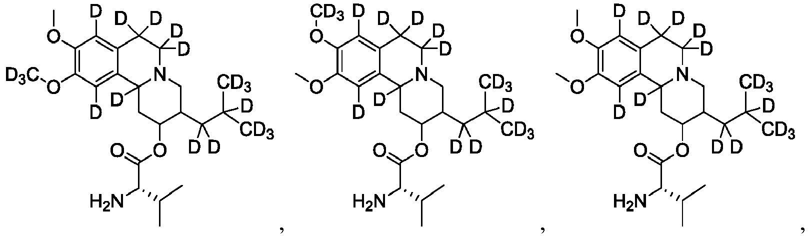 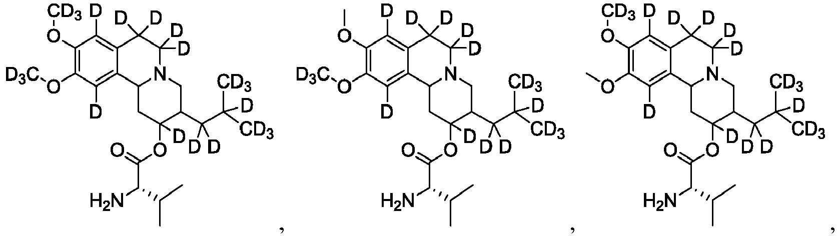 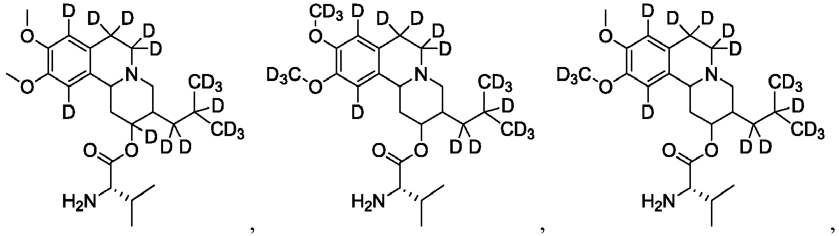 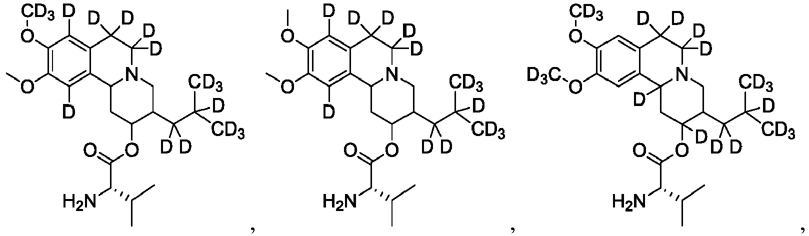 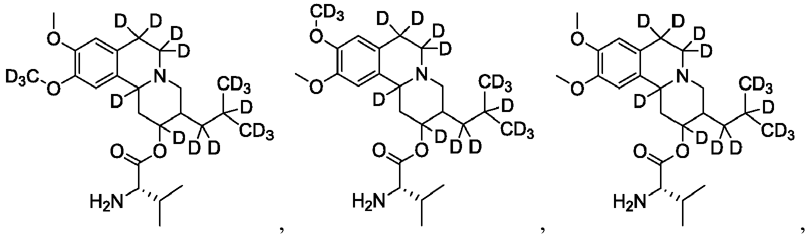 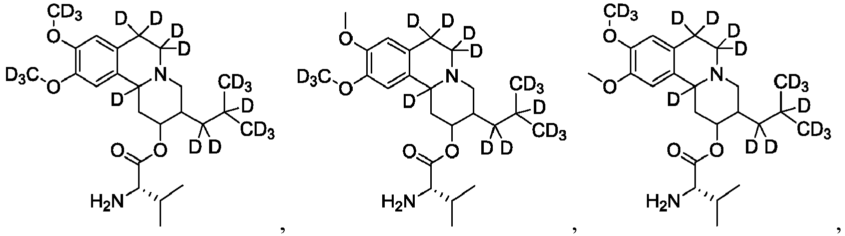 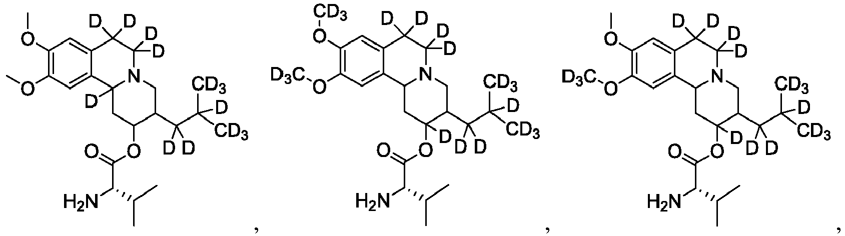 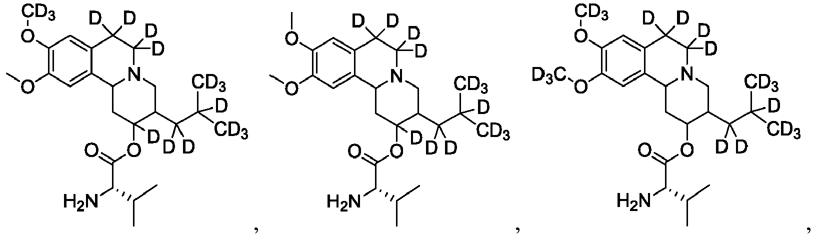 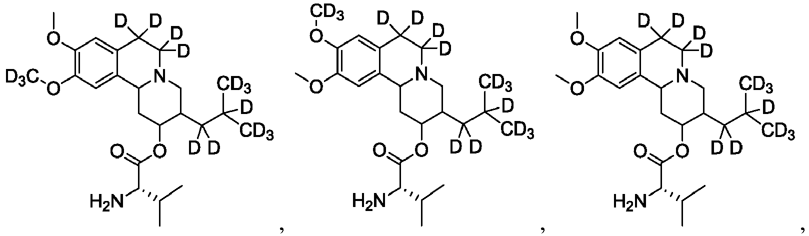 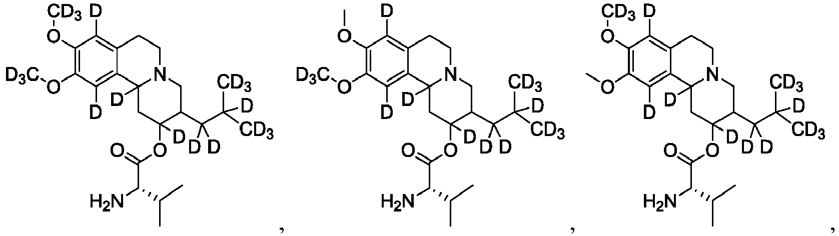 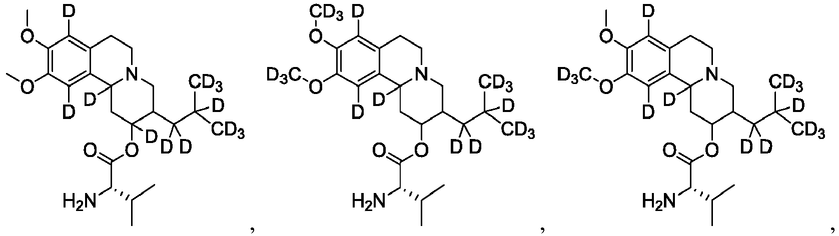 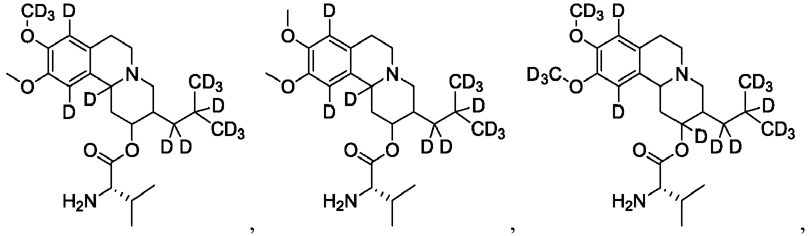 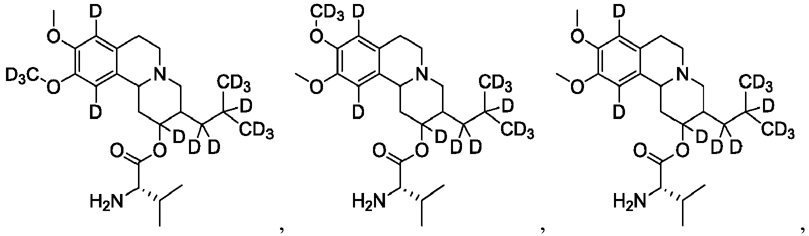 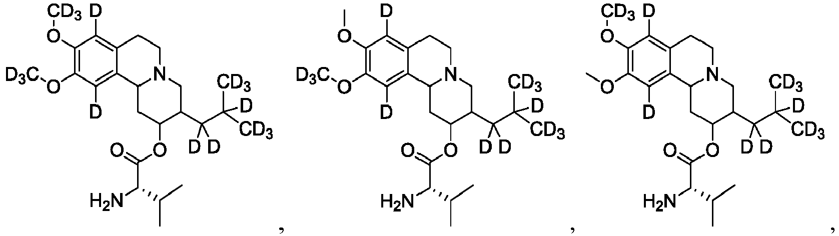 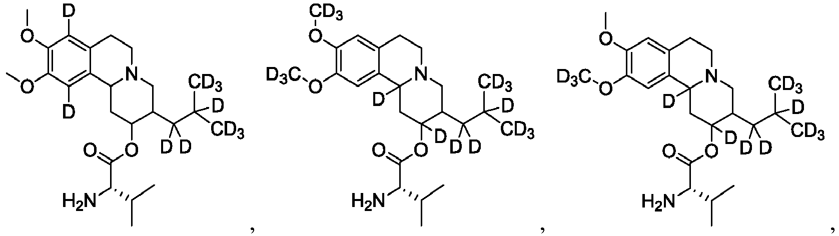 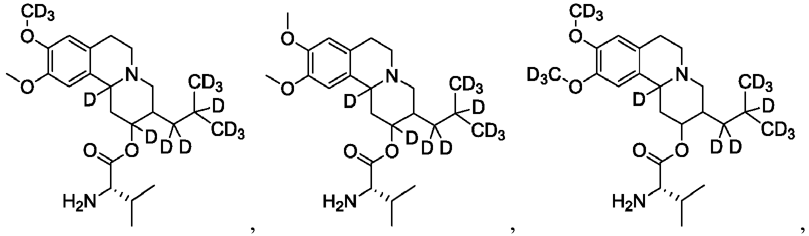 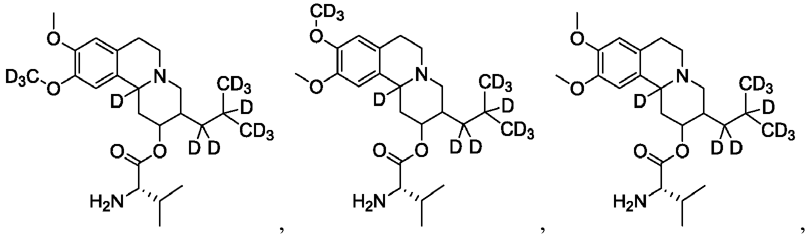 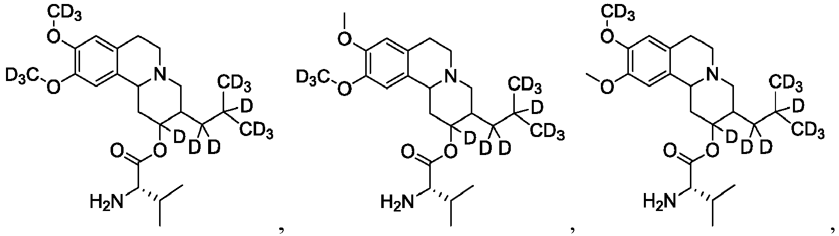 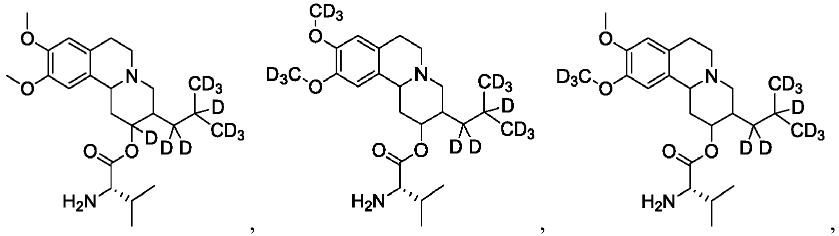 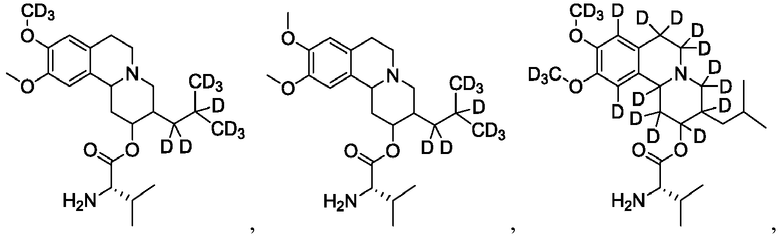 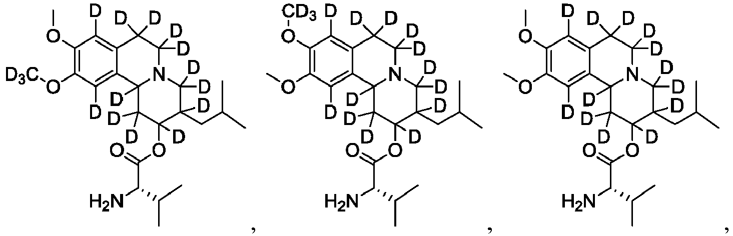 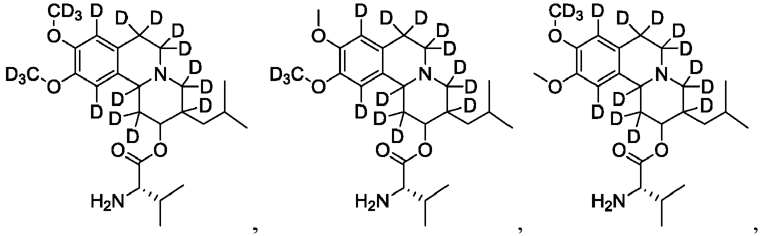 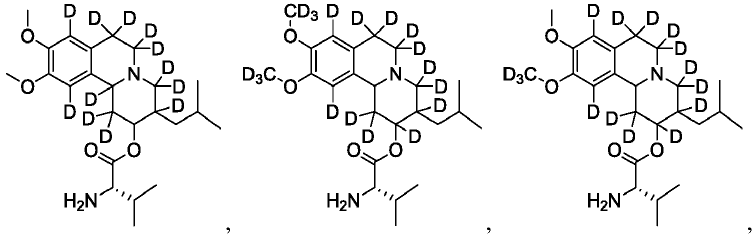 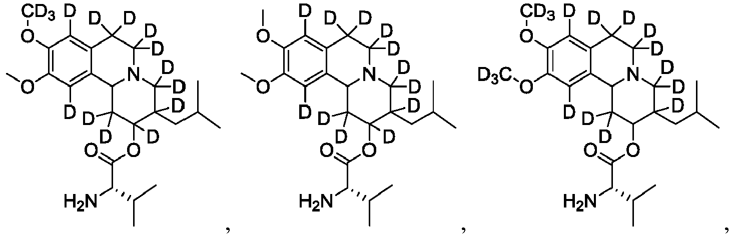 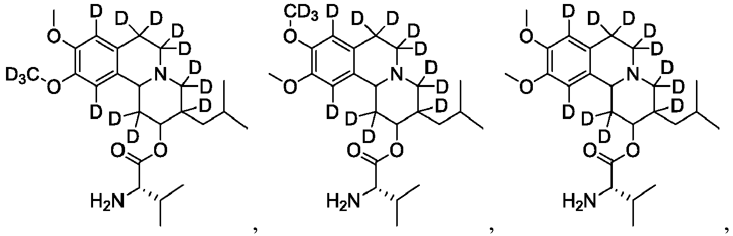 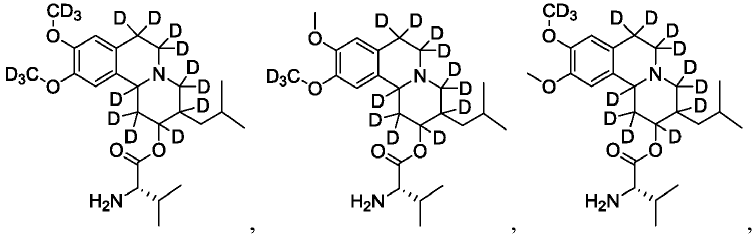 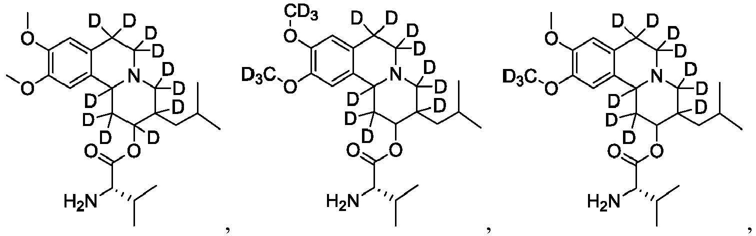 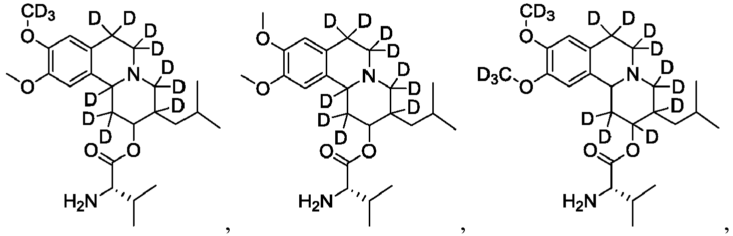 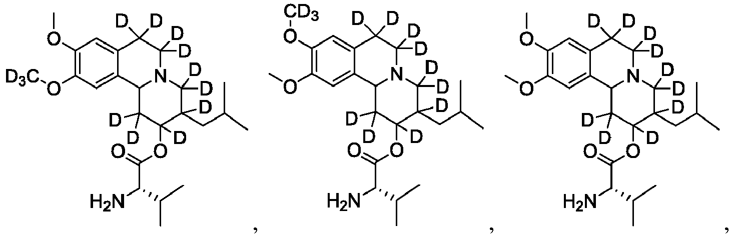 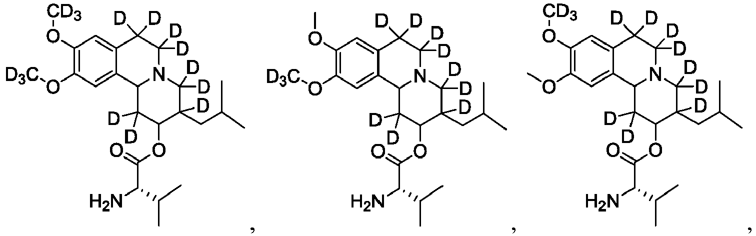 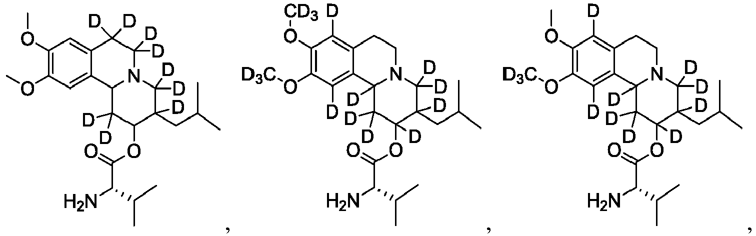 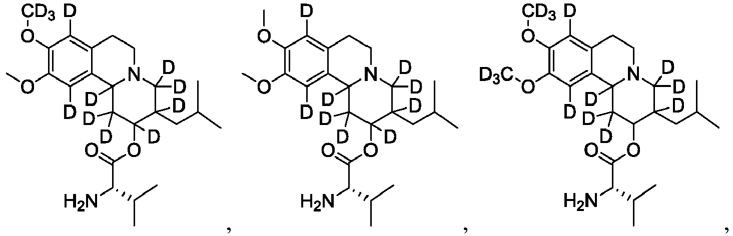 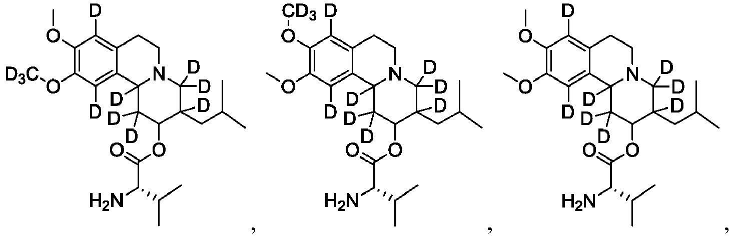 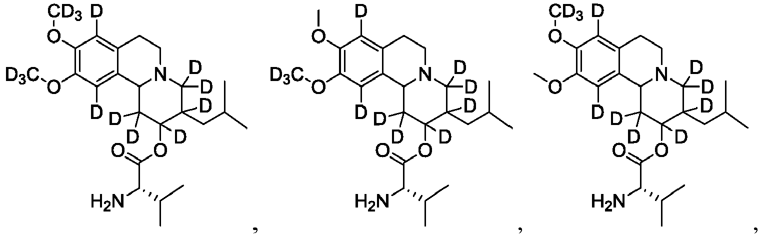 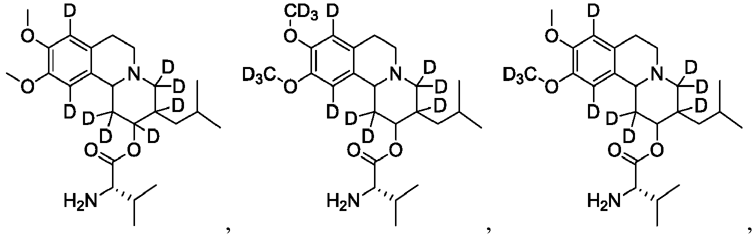 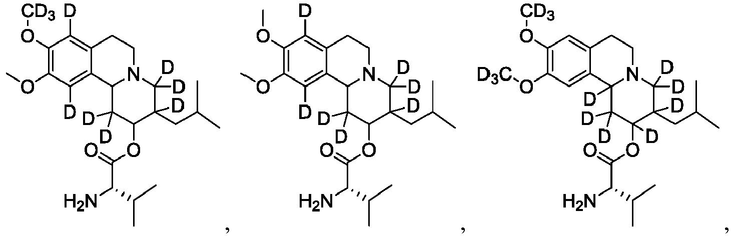 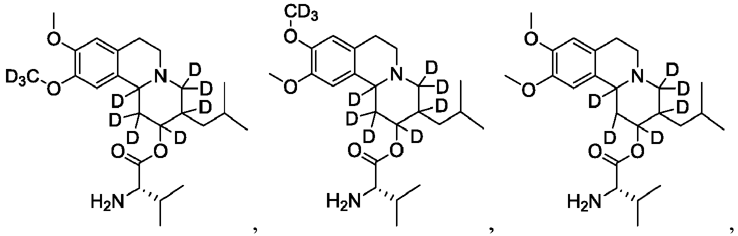 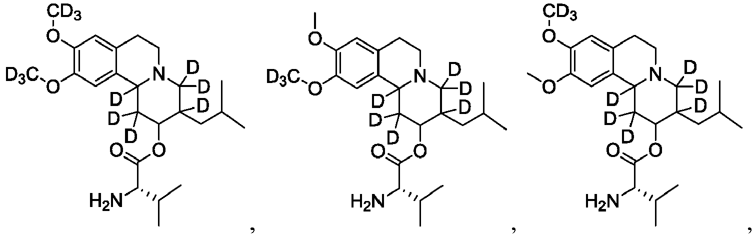 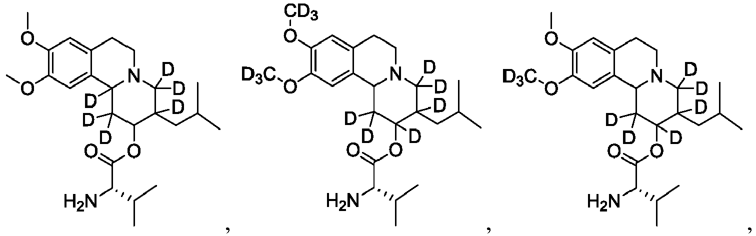 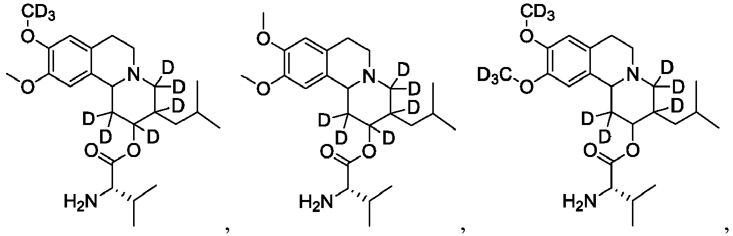 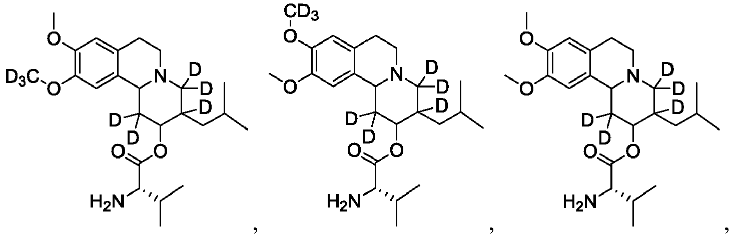 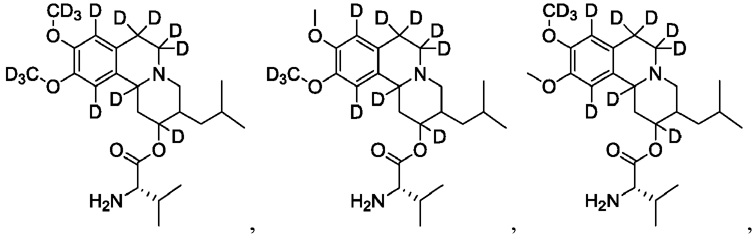 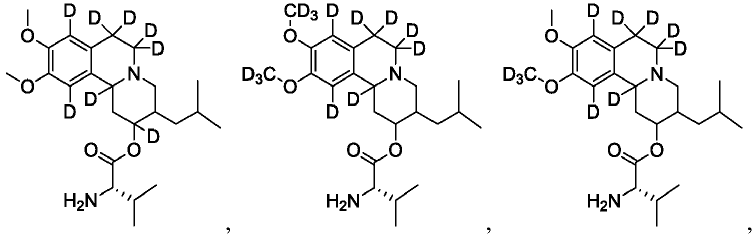 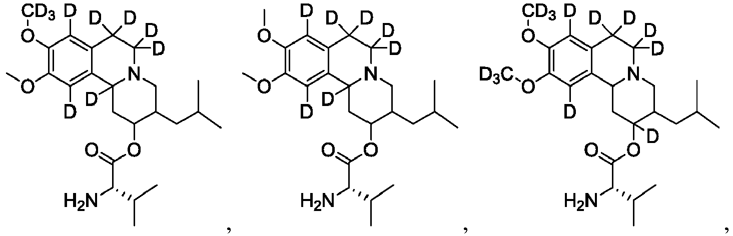 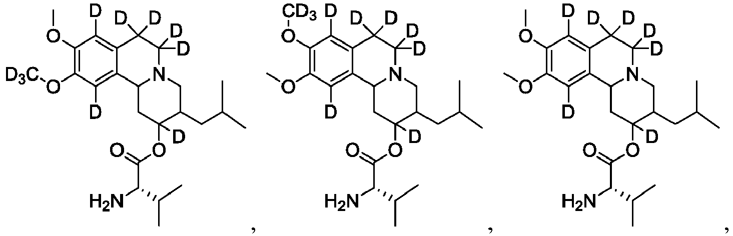 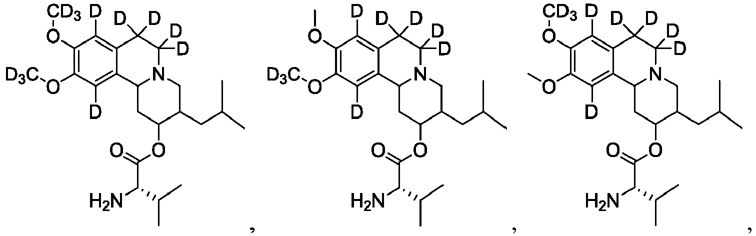 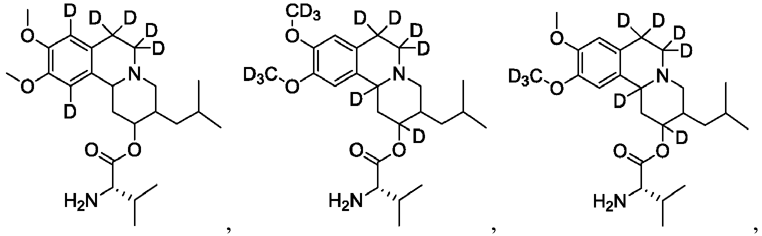 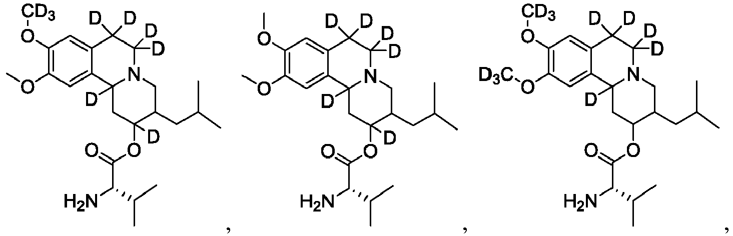 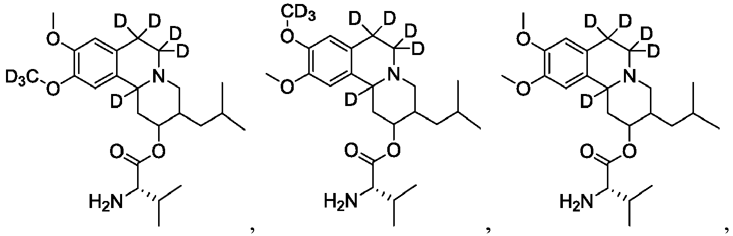 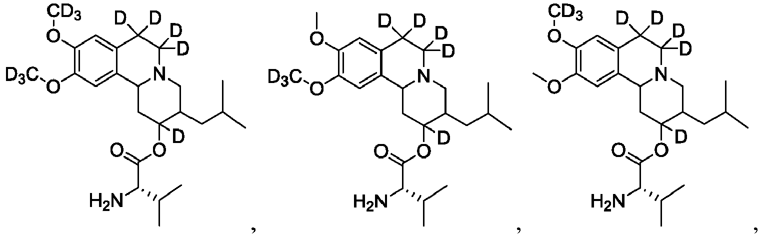 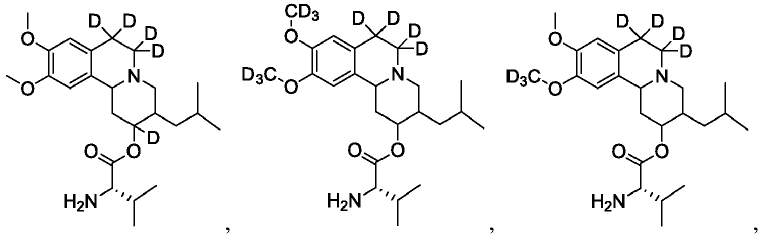 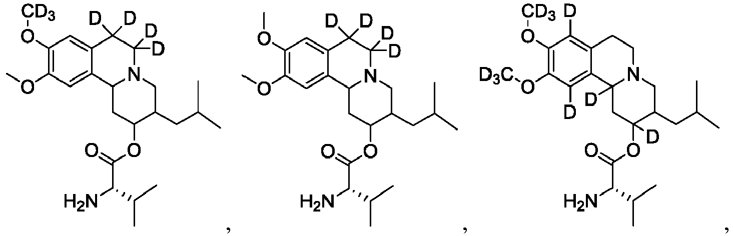 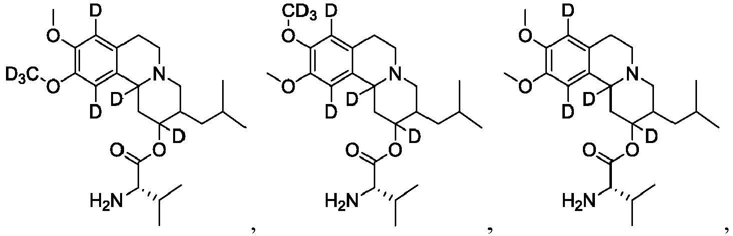 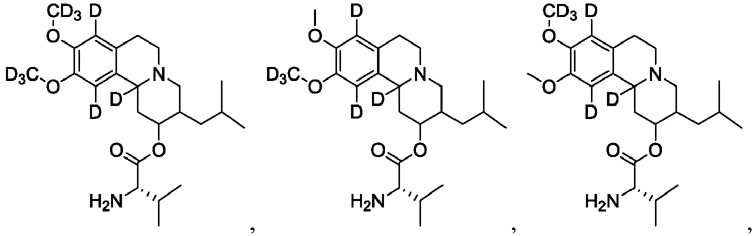 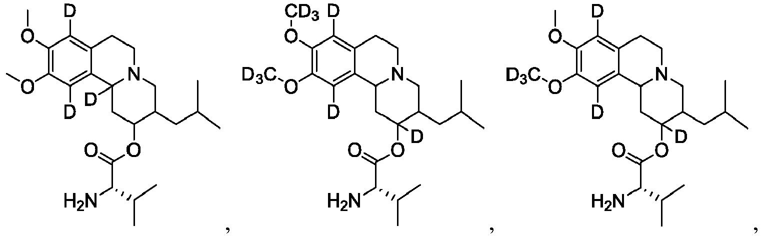 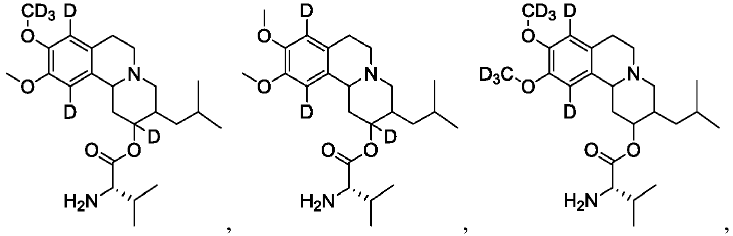 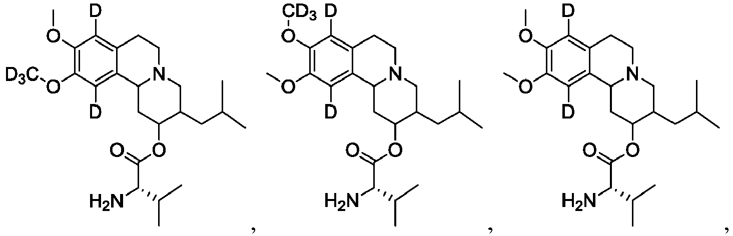 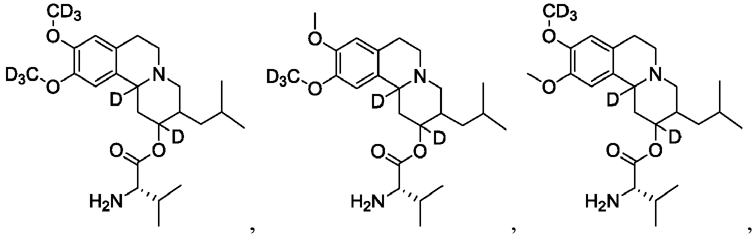 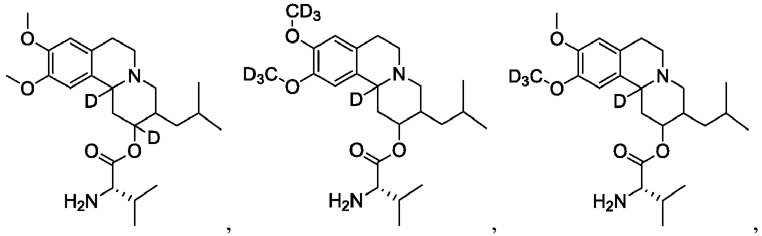 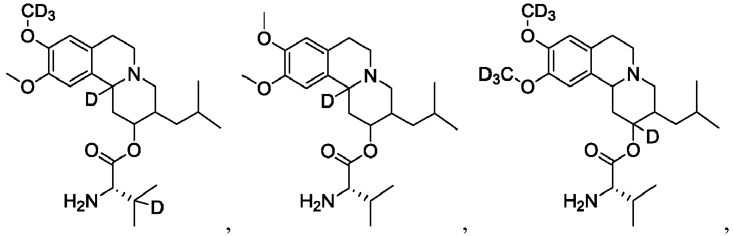 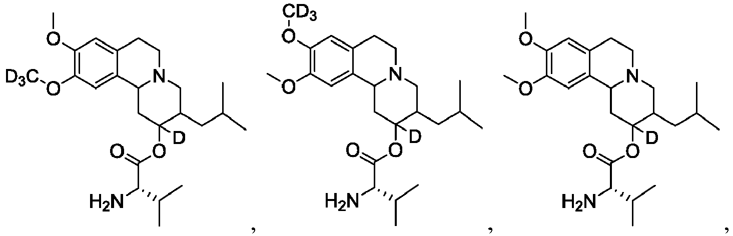 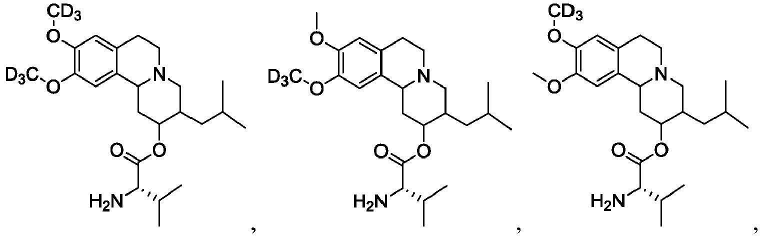 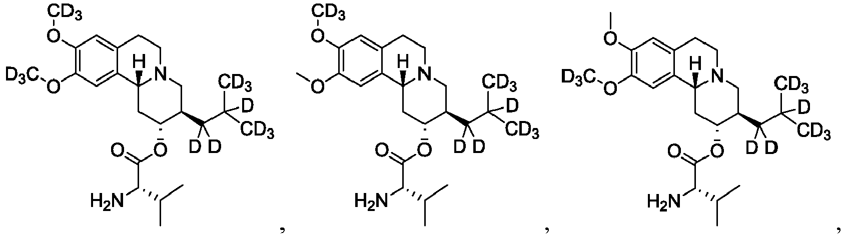 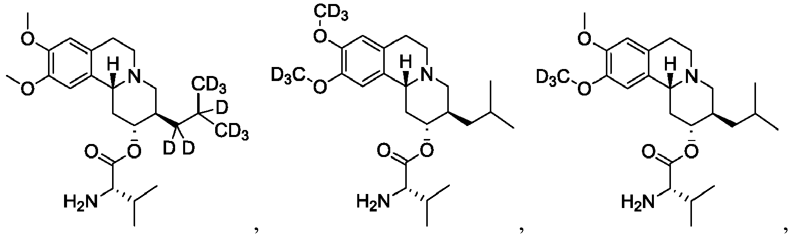 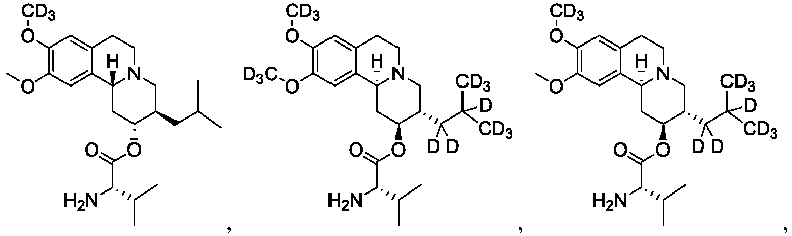 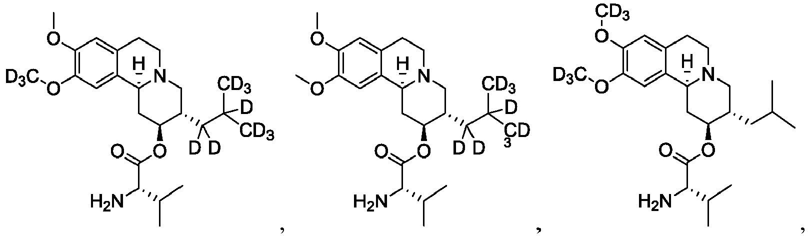 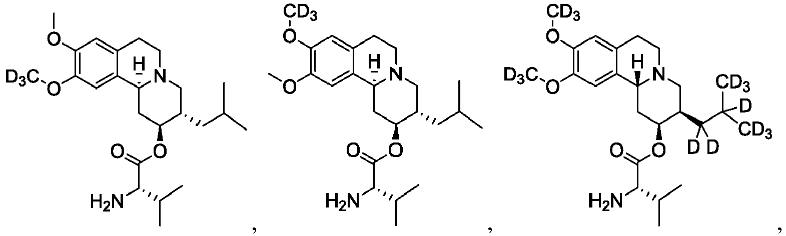 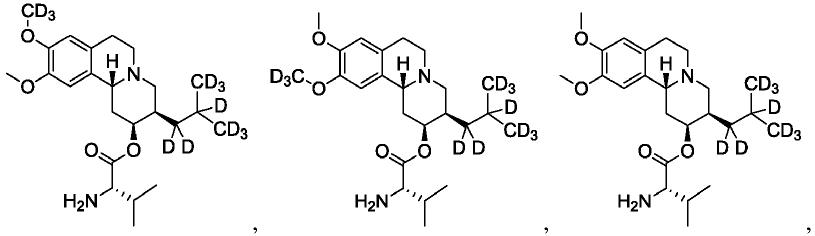 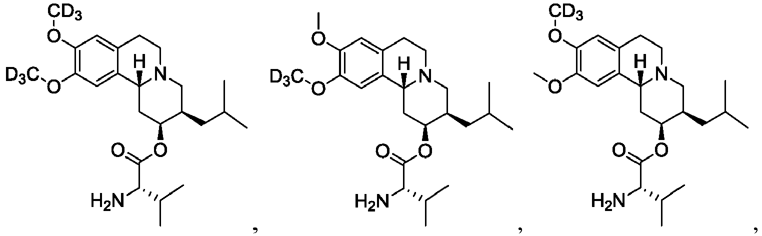 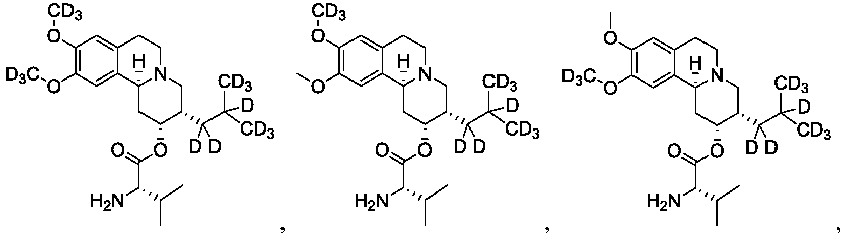 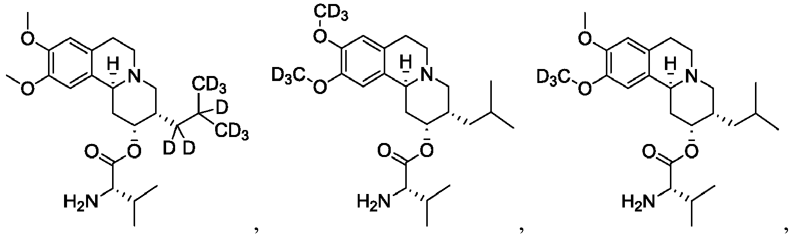 and

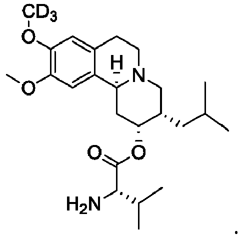

The following compounds can generally be made using the methods described above. It is expected that these compounds when made will have activity similar to those described in the examples above. 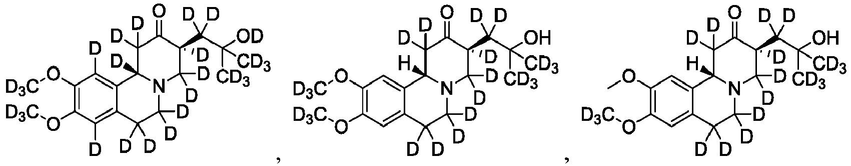 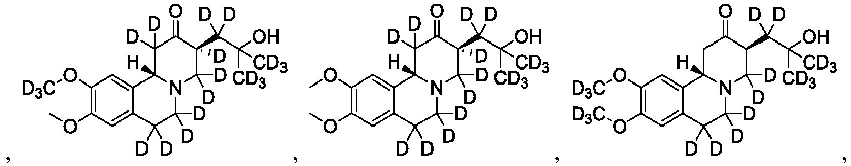 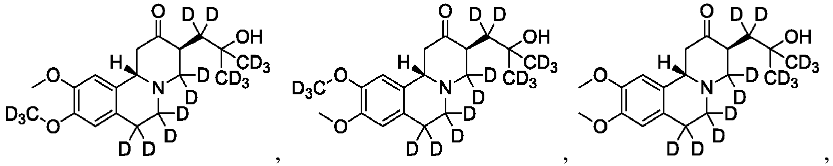 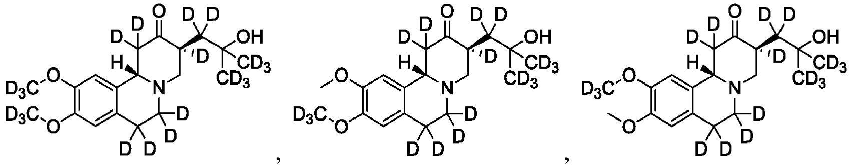 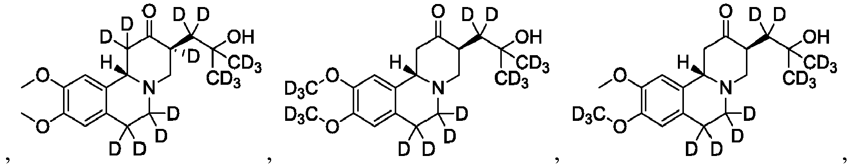 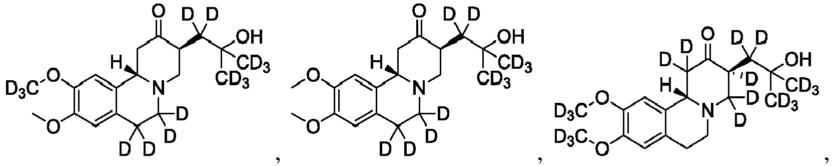 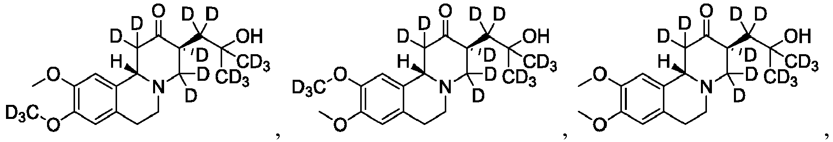 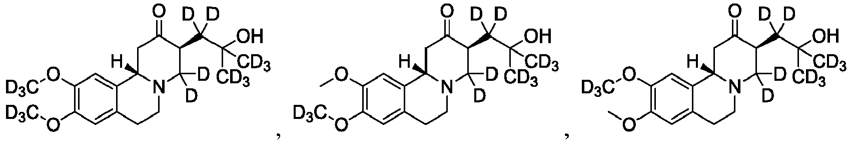 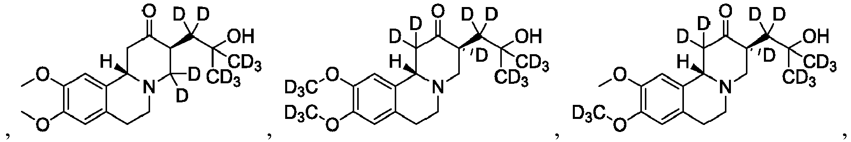 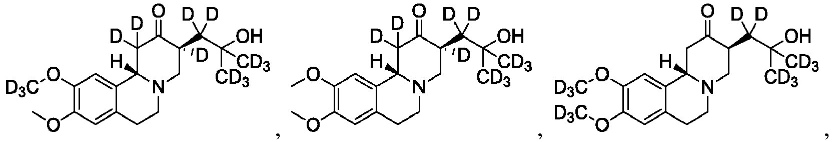 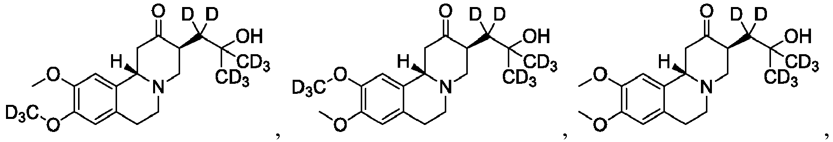 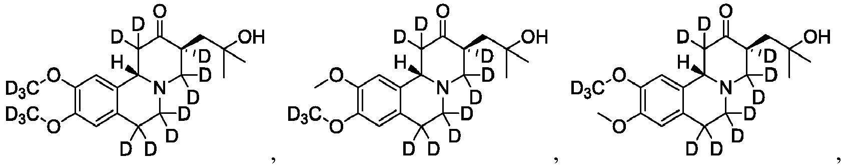 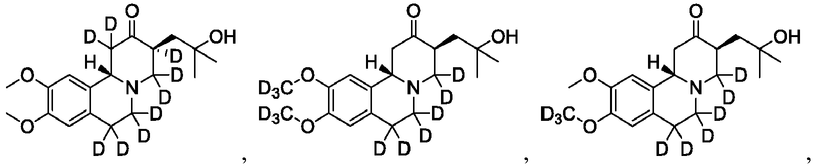 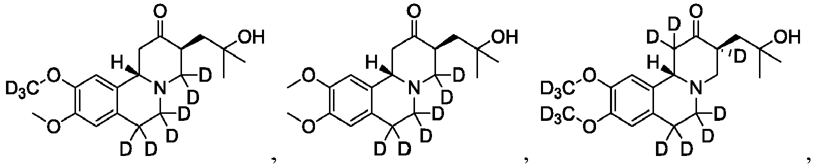 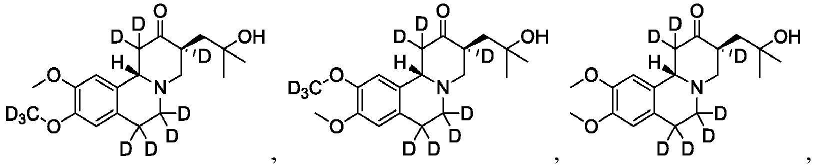 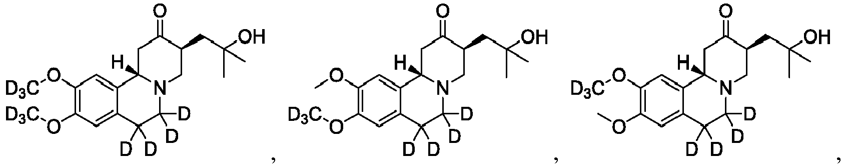 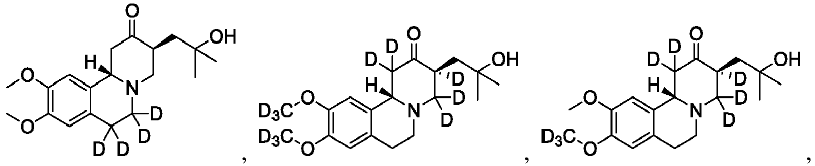 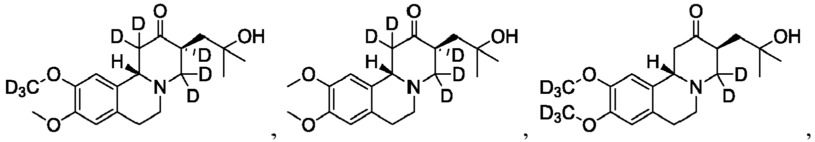 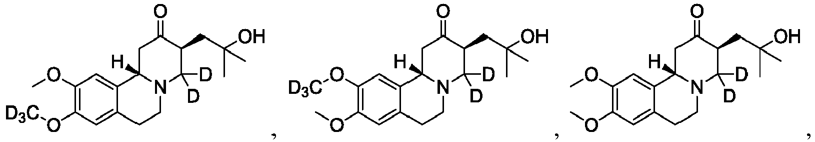 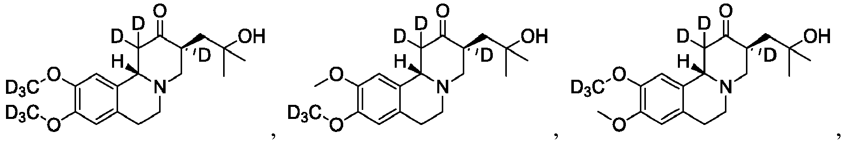 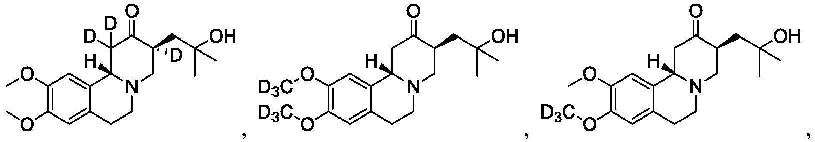 and 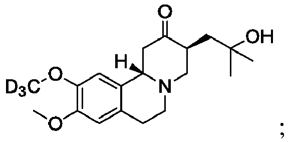 or the 3S,11bS enantiomer, or a racemic mixture of the the 3S, 11bS and 3R, 11bR enantiomers.

The following compounds can generally be made using the methods described above. It is expected that these compounds when made will have activity similar to those described in the examples above. 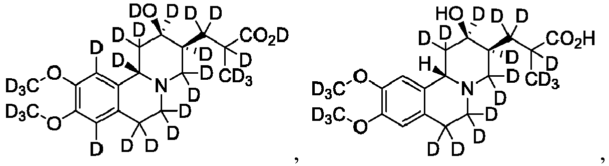 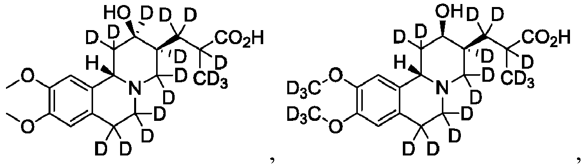 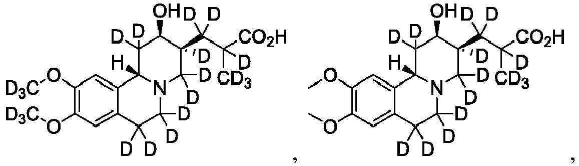 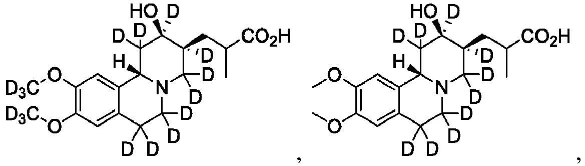 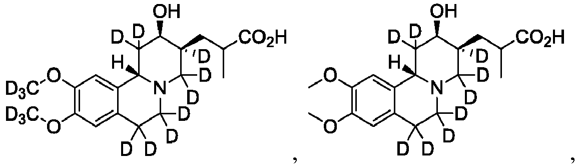 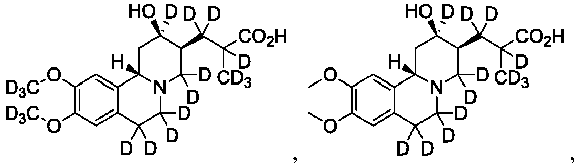 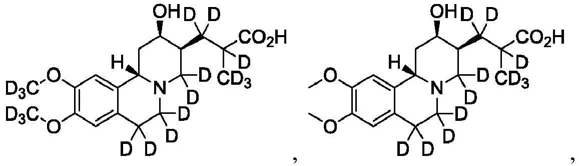 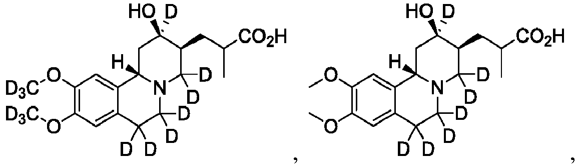 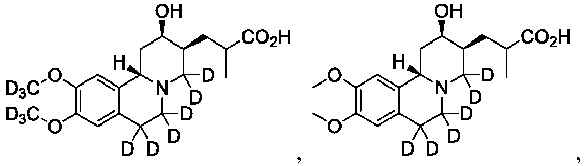 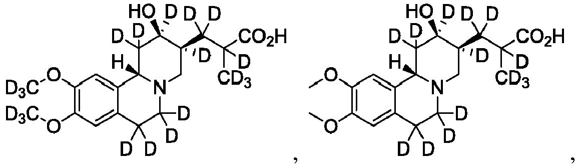 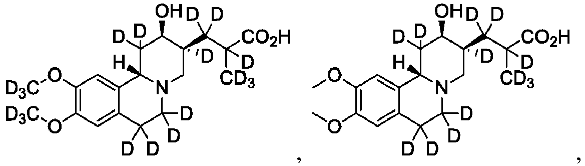 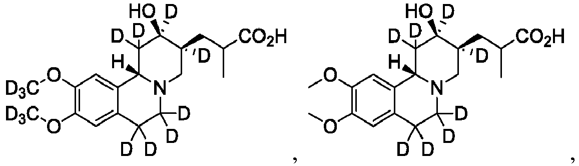 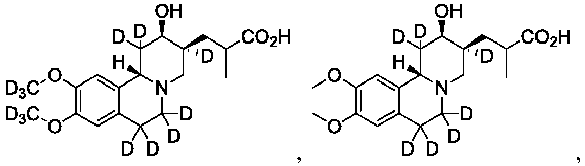 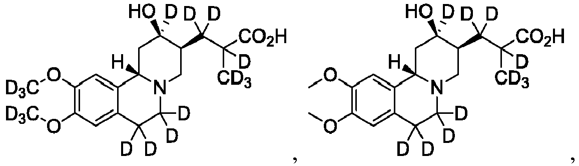 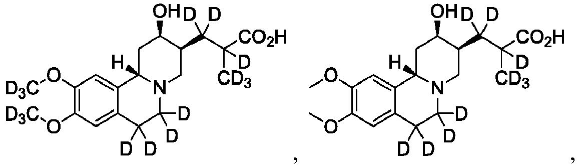 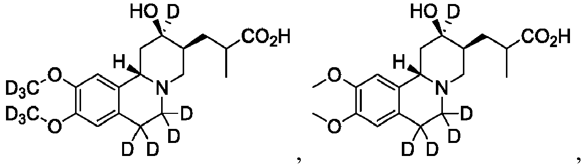 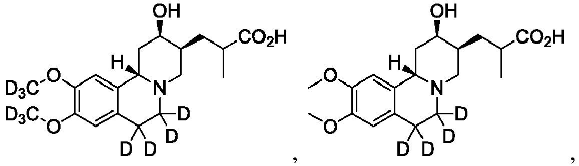 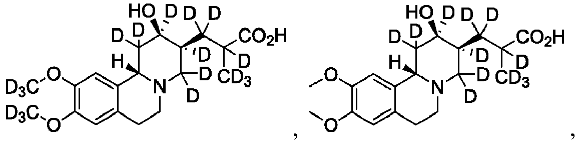 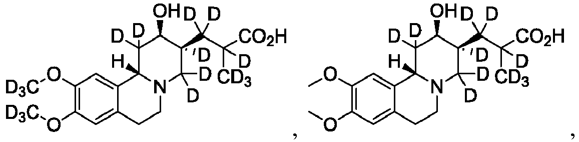 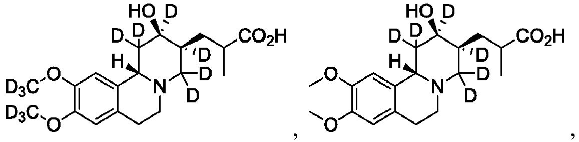 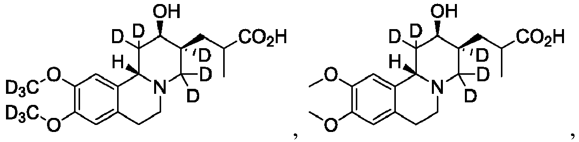 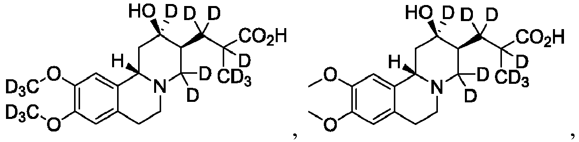 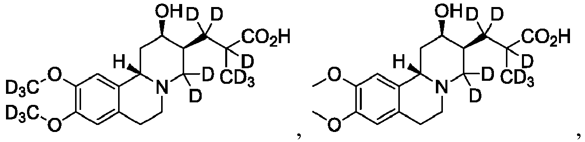 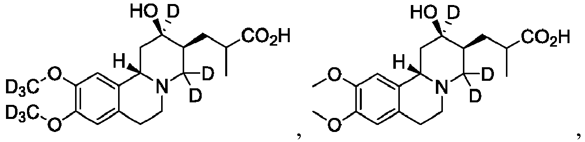 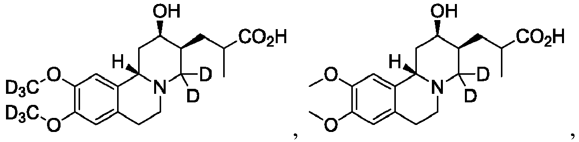 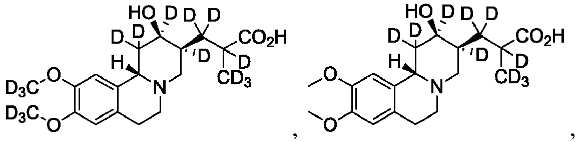 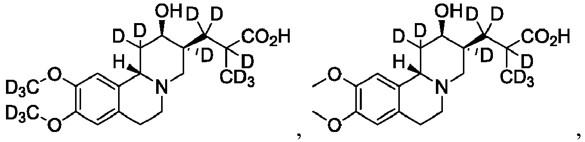 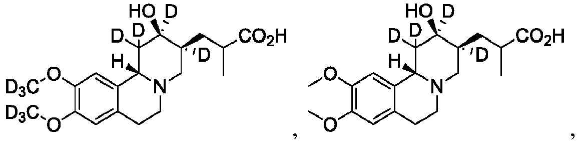 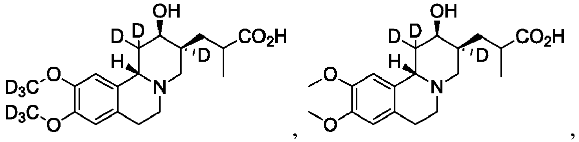 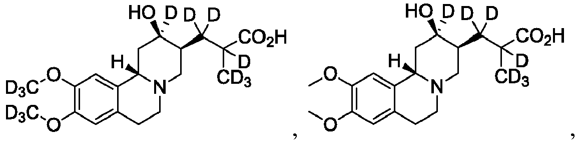 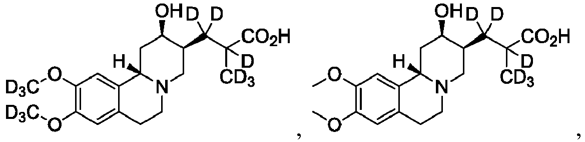 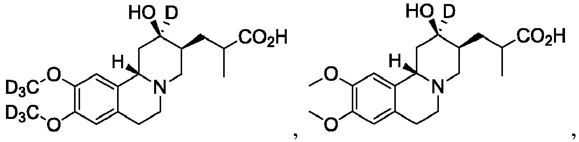 and 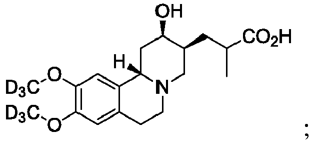 or a diastereomer, or mixture of diastereomers thereof.

Changes in the metabolic properties of the compounds disclosed herein as compared to their non-isotopically enriched analogs can be shown using the following assays. Compounds listed above which have not yet been made and/or tested are predicted to have changed metabolic properties as shown by one or more of these assays as well.

### Biological Activity Assays

### In vitro Human Liver Microsomal Stability Assay

Test compounds are dissolved in 50% acetonitrile / 50% H₂O for further dilution into the assay. Test compounds are combined with microsomes obtained from livers of the indicated species in the presence of a NADPH regenerating system (NRS) for incubation at 37°C in duplicate. For non-deuterated test compounds, the internal standard was the deuterated analog. For deuterated test compounds, the internal standard was the non-deuterated form. Samples were stored at -70°C for subsequent LC/MS/MS analysis.

The test compounds ar incubated at a concentration of 0.25µM with 4 mg/mL human liver microsomes for 60 minutes with samples taken at 0, 15, 30, 45 and 60 minutes. At each time point, the reaction is terminated with the addition of 100 µL acetonitrile containing internal standard. After vortexing, samples are centrifuged for 10 minutes at 14,000 rpm (RT) and the supernatants transferred to HPLC vials for LC/MS/MS analysis.

The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume is 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is performed using a 4000 QTrap ABI MS/MS detector in positive multiple reaction monitoring (MRM) mode.

Noncompartmental pharmacokinetic analyses are carried out using WinNonlin Professional (version 5.2, Pharsight, Mountain View, CA) and the terminal half life (t_{1/2}) calculated.

### In vitro Human S9 Liver Fraction Assay

Test compounds are dissolved in 50% acetonitrile / 50% H₂O for further dilution into the assay. Test compounds are combined with S9 liver fraction or liver cytosol in the presence of a NADPH regenerating system (NRS) for incubation at 37°C in duplicate as noted above for 60 minutes (see below). For non-deuterated test compounds, the internal standard is the deuterated analog. For deuterated test compounds, the internal standard is the non-deuterated form. Samples are stored at -70°C for subsequent LC/MS/MS analysis.

The test compounds are incubated at a concentration of 0.25µM with 4 mg/mL human S9 liver fraction for 60 minutes with samples taken at 0, 15, 30, 45 and 60 minutes. At each time point, the reaction is terminated with the addition of 100 µL acetonitrile containing internal standard. After vortexing, samples are centrifuged for 10 minutes at 14,000 rpm (RT) and the supernatants transferred to HPLC vials for LC/MS/MS analysis.

Analytical Method 1 - The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume is 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is performed using a 4000 QTrap ABI MS/MS detector in positive multiple reaction monitoring (MRM) mode.

Analytical Method 2 - The analytes are separated by reverse-phase HPLC using Agilent Eclipse XBD C19*150 columns. The LC mobile phase is 0.1% formic acid in water (A) and 0.1% formic acid in ACN (B). The flow rate is 1 mL/minute and the injection volume was 10µL.

| Time (minutes) | A (%) | B (%) |
|---|---|---|
| 3.5 | 75 | 25 |
| 4.5 | 10 | 90 |
| 6.2 | 10 | 90 |
| 6.3 | 75 | 25 |
| 6.5 | System Controller | Stop |

After chromatographic separation of the analytes, quantiation is performed using a 4000 QTrap ABI MS/MS detector in positive multiple reaction monitoring (MRM) mode.

Noncompartmental pharmacokinetic analyses are carried out using WinNonlin Professional (version 5.2, Pharsight, Mountain View, CA) and the terminal half life (t_{1/2}) calculated.

### In vitro metabolism using human cytochrome P₄₅₀ enzymes

Test compounds are dissolved in 50% acetonitrile / 50% H₂O for further dilution into the assay. Test compounds at a final concentration of 0.25µM are combined with recombinant human CYP1A2, CYP3A4 or CYP2D6 in microsomes obtained from Baculovirus infected insect cells (Supersomes™, Gentest, Woburn, MA) in the presence of a NADPH regenerating system (NRS) for incubation at 37°C for 0, 15, 30, 45 or 60 minutes. At each time point, the reaction is terminated with the addition of 100 µL ACN containing an internal standard. For deuterated test compounds, the internal standard is the non-deuterated form. After vortexing, samples are centrifuged for 10 minutes at 14,000 rpm (room temperature) and the supernatants transferred to HPLC vials for LC/MS/MS analysis. Samples are stored at -70°C for subsequent LC/MS/MS analysis.

The analytes are separated by reverse-phase HPLC using Phenomenex columns (Onyx Monolithic C18, 25 X 4.6 mm). The LC mobile phase is 0.1% Formic acid (A) and methanol (B). The flow rate is 1 mL/minute and the injection volume was 10µL.

| Time (minutes) | A (%) | B (%) |
|---|---|---|
| 0.1 | 90 | 10 |
| 0.6 | 10 | 90 |
| 1.2 | 10 | 90 |
| 1.3 | 90 | 10 |
| 2.0 | System Controller | Stop |

After chromatographic separation of the analytes, quantiation is perfomed using a 4000 QTrap ABI MS/MS detector in positive multiple reaction monitoring (MRM) mode.

### Monoamine Oxidase A Inhibition and Oxidative Turnover

The procedure is carried out using the methods described by Weyler et al., Journal of Biological Chemistry 1985, 260, 13199-13207, which is hereby incorporated by reference in its entirety. Monoamine oxidase A activity is measured spectrophotometrically by monitoring the increase in absorbance at 314 nm on oxidation of kynuramine with formation of 4-hydroxyquinoline. The measurements are carried out, at 30 °C, in 50mM sodium phosphate buffer, pH 7.2, containing 0.2% Triton X-100 (monoamine oxidase assay buffer), plus 1 mM kynuramine, and the desired amount of enzyme in 1 mL total volume.

### Monooamine Oxidase B Inhibition and Oxidative Turnover

The procedure is carried out as described in Uebelhack et al., Pharmacopsychiatry 1998, 31(5), 187-192, which is hereby incorporated by reference in its entirety.

### Determination of tetrabenazine and an active metabolite by HPLC

The procedure is carried out as described in Roberts et al., Journal of Chromatography, Biomedical Applications 1981, 226(1), 175-82, which is hereby incorporated by reference in its entirety.

### Pharmacokinetic assays of tetrabenazine and its major metabolite in man and rat

The procedure is carried out as described in Mehvar, et al., Drug Metabolism and Disposition 1987, 15(2), 250-5, which is hereby incorporated by reference in its entirety.

### Detecting tetrabenazine metabolites in animals and man

The procedure is carried out as described in Schwartz, et al., Biochemical Pharmacology 1966, 15(5), 645-55, which is hereby incorporated by reference in its entirety.

### Mass spectrometric determination of tetrabenazine

The procedure is carried out as described in Jindal, et al., Journal of Chromatography, Biomedical Applications 1989, 493(2), 392-7, which is hereby incorporated by reference in its entirety.

### In Vitro Radioligand Binding Assay

The procedure is carried out as described in Scherman et al., Journal of Neurochemistry 1988, 50(4), 1131-36, which is hereby incorporated by reference in its entirety.

### In Vitro Radioligand Binding Assay

The procedure is carried out as described in Kilbourn et al., Synapse 2002, 43(3), 188-194, which is hereby incorporated by reference in its entirety.

### In Vitro Radioligand Binding Assay

The procedure is carried out as described in Kilbourn et al., European Journal of Pharmacology 1997, 331(2-3), 161-68, which is hereby incorporated by reference in its entirety.

### ³H-Histamine Transport Assay

The procedure is carried out as described in Erickson et al., Journal of Molecular Neuroscience 1995, 6(4), 277-87, which is hereby incorporated by reference in its entirety.

From the foregoing description, one skilled in the art can ascertain the essential characteristics of this invention, and without departing from the spirit and scope thereof, can make various changes and modifications of the invention to adapt it to various usages and conditions.

Aspects and embodiments of the present invention are described with reference to the numbered clauses set out below:
1. A method of treatment of Parkinson's disease levodopa-induced dyskinesia comprising the administration of a therapeutically effective amount of a compound of structural Formula I 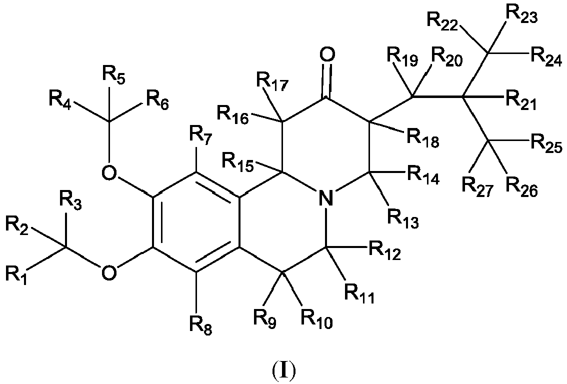 or a salt, stereoisomer, or racemic mixture thereof, wherein:
   R₁-R₂₇ are independently selected from the group consisting of hydrogen and deuterium; and
   at least one of R₁-R₂₇ is deuterium.
2. The method as recited in Clause 1 wherein at least one of R₁-R₂₇ independently has deuterium enrichment of no less than about 10%.
3. The method as recited in Clause 1 wherein at least one of R₁-R₂₇ independently has deuterium enrichment of no less than about 50%.
4. The method as recited in Clause 1 wherein at least one of R₁-R₂₇ independently has deuterium enrichment of no less than about 90%.
5. The method as recited in Clause 1 wherein at least one of R₁-R₂₇ independently has deuterium enrichment of no less than about 98%.
6. The method as recited in Clause 1 wherein said compound has the structural formula: 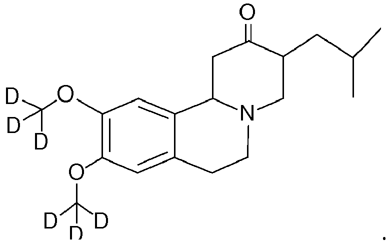
7. A method of treatment of oppositional defiant disorder comprising the administration of a therapeutically effective amount of a compound of structural Formula II 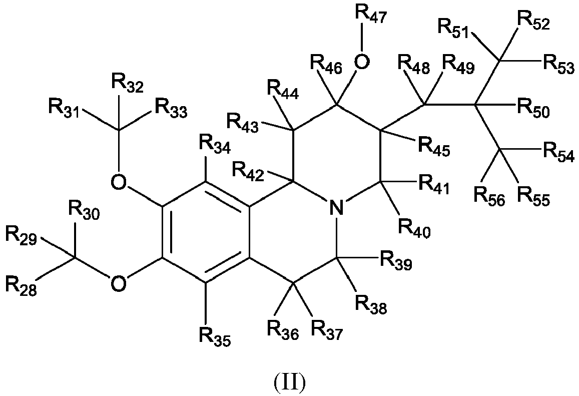 or a salt, stereoisomer, or racemic mixture thereof, wherein:
   R₂₈-R₅₆ are independently selected from the group consisting of hydrogen and deuterium; and
   at least one of R₂₈-R₅₆ is deuterium.
8. The method as recited in Clause 7 wherein at least one of R₂₈-R₅₆ independently has deuterium enrichment of no less than about 10%.
9. The method as recited in Clause 7 wherein at least one of R₂₈-R₅₆ independently has deuterium enrichment of no less than about 50%.
10. The method as recited in Clause 7 wherein at least one of R₂₈-R₅₆ independently has deuterium enrichment of no less than about 90%.
11. The method as recited in Clause 7 wherein at least one of R₂₈-R₅₆ independently has deuterium enrichment of no less than about 98%.
12. The method as recited in Clause 7 wherein said compound has the structural formula: 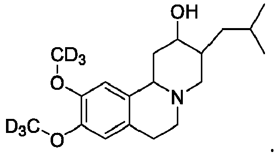
13. The method of any one of Clauses 7-12, wherein said compound is the alpha stereoisomer.
14. The method of any one of Clauses 7-12, wherein said compound is the beta stereoisomer.
15. The method as recited in Clause 7 wherein said compound has the structural formula: 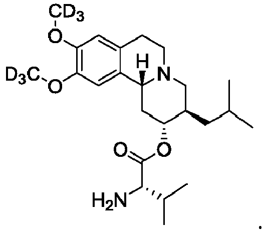
16. A method of treatment of Parkinson's disease levodopa-induced dyskinesia comprising the administration of a therapeutically effective amount of a compound of structural Formula III 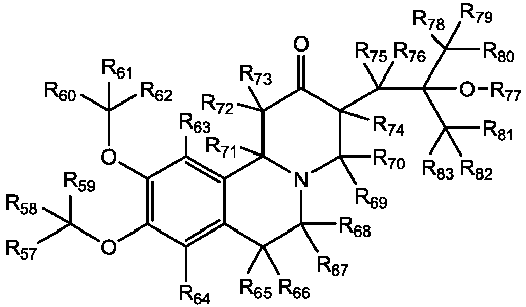 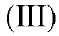 or a salt, stereoisomer, or racemic mixture thereof, wherein:
   R₅₇-R₈₃ are independently selected from the group consisting of hydrogen and deuterium; and
   at least one of R₅₇-R₈₃ is deuterium.
17. The method as recited in Clause 16 wherein at least one of R₅₇-R₈₃ independently has deuterium enrichment of no less than about 10%.
18. The method as recited in Clause 16 wherein at least one of R₅₇-R₈₃ independently has deuterium enrichment of no less than about 50%.
19. The method as recited in Clause 16 wherein at least one of R₅₇-R₈₃ independently has deuterium enrichment of no less than about 90%.
20. The method as recited in Clause 16 wherein at least one of R₅₇-R₈₃ independently has deuterium enrichment of no less than about 98%.
21. The method as recited in Clause 16 wherein said compound has the structural formula: 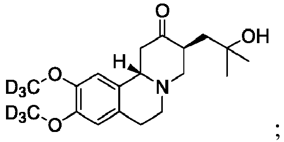 or the 3S,11bS enantiomer, 3R,11bR enantiomer, or a racemic mixture of the the 3S,11bS and 3R,11bR enantiomers.
22. A method of treatment of Parkinson's disease levodopa-induced dyskinesia comprising the administration of a therapeutically effective amount of a compound of structural Formula IV 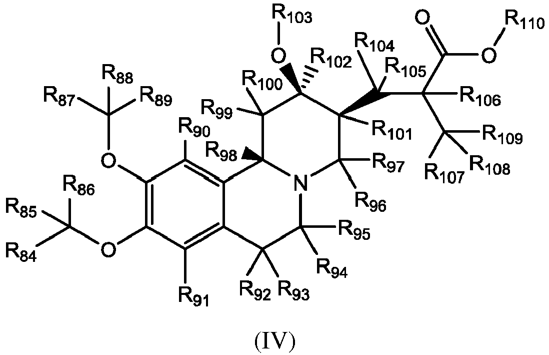 or a salt, stereoisomer, or racemic mixture thereof, wherein:
   R₈₄-R₁₁₀ are independently selected from the group consisting of hydrogen and deuterium; and
   at least one of R₈₄-R₁₁₀ is deuterium.
23. The method as recited in Clause 22 wherein at least one of R₈₄-R₁₁₀ independently has deuterium enrichment of no less than about 10%.
24. The method as recited in Clause 22 wherein at least one of R₈₄-R₁₁₀ independently has deuterium enrichment of no less than about 50%.
25. The method as recited in Clause 22 wherein at least one of R₈₄-R₁₁₀ independently has deuterium enrichment of no less than about 90%.
26. The method as recited in Clause 22 wherein at least one of R₈₄-R₁₁₀ independently has deuterium enrichment of no less than about 98%.
27. The method as recited in Clause 22 wherein said compound has the structural formula: 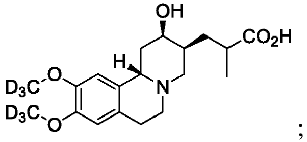 or a diastereomer, or mixture of diastereomers thereof.
28. The method as recited in any one of Clauses 6, 12, 15, 21, and 27 wherein each position represented as D has deuterium enrichment of no less than about 10%.
29. The method as recited in any one of Clauses 6, 12, 15, 21, and 27 wherein each position represented as D has deuterium enrichment of no less than about 50%.
30. The method as recited in any one of Clauses 6, 12, 15, 21, and 27 wherein each position represented as D has deuterium enrichment of no less than about 90%.
31. The method as recited in any one of Clauses 6, 12, 15, 21, and 27 wherein each position represented as D has deuterium enrichment of no less than about 98%.
32. The method as recited in any one of Clauses 1-31 further comprising the administration of an additional therapeutic agent.
33. The method as recited in Clause 32 wherein said additional therapeutic agent is selected from the group consisting of dopamine precursors, DOPA decarboxylase inhibitors, catechol-O-methyl transferase (COMT) inhibitors, dopamine receptor agonists, neuroprotective agents, NMDA antagonists, and anti-psychotics.
34. The method as recited in Clause 33 wherein said dopamine precursor is levodopa.
35. The method as recited in Clause 33 wherein said DOPA decarboxylase inhibitor is carbidopa.
36. The method as recited in Clause 33 wherein said catechol-O-methyl transferase (COMT) inhibitor is selected from the group consisting of entacapone and tolcapone.
37. The method as recited in Clause 33 wherein said dopamine receptor agonist is selected from the group consisting of apomorphine, bromocriptine, ropinirole, and pramipexole.
38. The method as recited in Clause 33 wherein said neuroprotective agent is selected from the group consisting of selegeline and riluzole.
39. The method as recited in Clause 33 wherein said NMDA antagonist is amantidine.
40. The method as recited in Clause 33 wherein said anti-psychotic is clozapine.
41. The method as recited in any one of Clauses 1-31, wherein said treatment results in at least one of the clinical effects selected from the group consisting of:
   a. improved Unified Parkinson's Disease Rating Scale scores;
   b. improved Abnormal Involuntary Movement Scale scores;
   c. improved Goetz Dyskinesia Rating Scale scores;
   d. improved Unified Dyskinesia Rating Scale scores;
   e. improved PDQ-39 Parkinson's Disease Questionnaire scores; and
   f. improved Global Primate Dyskinesia Rating Scale scores.
42. The method as recited in any one of Clauses 1-31, further resulting in at least one effect selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
43. The method as recited in any one of Clauses 1-31, further resulting in at least two effects selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
44. The method as recited in any one of Clauses 1-31, wherein the method effects a decreased metabolism of the compound per dosage unit thereof by at least one polymorphically-expressed cytochrome P₄₅₀ isoform in the subject, as compared to the corresponding non-isotopically enriched compound.
45. The method as recited in Clause 44, wherein the cytochrome P₄₅₀ isoform is selected from the group consisting of CYP2C8, CYP2C9, CYP2C19, and CYP2D6.
46. The method as recited any one of Clauses 1-31, wherein said compound is characterized by decreased inhibition of at least one cytochrome P₄₅₀ or monoamine oxidase isoform in said subject per dosage unit thereof as compared to the non-isotopically enriched compound.
47. The method as recited in Clause 46, wherein said cytochrome P₄₅₀ or monoamine oxidase isoform is selected from the group consisting of CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.
48. The method as recited in any one of Clauses 1-31, wherein the method reduces a deleterious change in a diagnostic hepatobiliary function endpoint, as compared to the corresponding non-isotopically enriched compound.
49. The method as recited in Clause 48, wherein the diagnostic hepatobiliary function endpoint is selected from the group consisting of alanine aminotransferase ("ALT"), serum glutamic-pyruvic transaminase ("SGPT"), aspartate aminotransferase ("AST," "SGOT"), ALT/AST ratios, serum aldolase, alkaline phosphatase ("ALP"), ammonia levels, bilirubin, gamma-glutamyl transpeptidase ("GGTP," "γ-GTP," "GGT"), leucine aminopeptidase ("LAP"), liver biopsy, liver ultrasonography, liver nuclear scan, 5'-nucleotidase, and blood protein.
50. A compound of structural Formula I 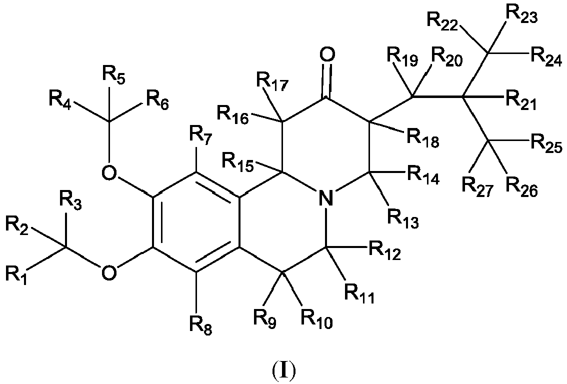 or a salt, stereoisomer, or racemic mixture thereof, wherein:
   R₁-R₂₇ are independently selected from the group consisting of hydrogen and deuterium; and
   at least one of R₁-R₂₇ is deuterium;
   for use in the treatment of Parkinson's disease levodopa-induced dyskinesia.
51. The compound as recited in Clause 50 wherein at least one of R₁-R₂₇ independently has deuterium enrichment of no less than about 10%.
52. The compound as recited in Clause 50 wherein at least one of R₁-R₂₇ independently has deuterium enrichment of no less than about 50%.
53. The compound as recited in Clause 50 wherein at least one of R₁-R₂₇ independently has deuterium enrichment of no less than about 90%.
54. The compound as recited in Clause 50 wherein at least one of R₁-R₂₇ independently has deuterium enrichment of no less than about 98%.
55. The compound as recited in Clause 50 wherein said compound has the structural formula: 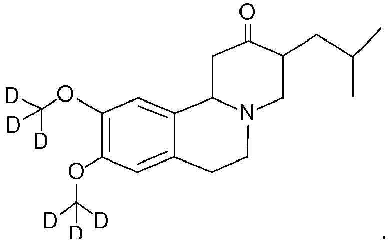
56. A compound of structural Formula II 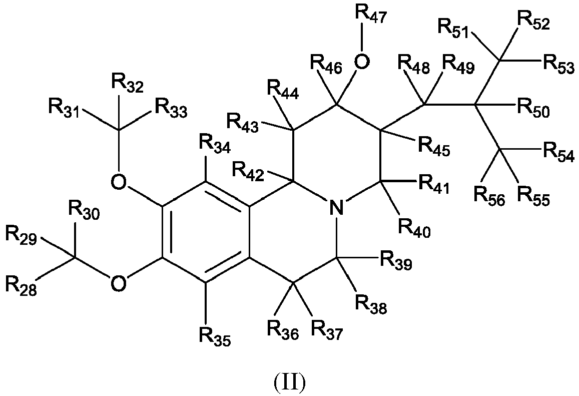 or a salt, stereoisomer, or racemic mixture thereof, wherein:
   R₂₈-R₅₆ are independently selected from the group consisting of hydrogen and deuterium; and
   at least one of R₂₈-R₅₆ is deuterium;
   for use in the treatment of Parkinson's disease levodopa-induced dyskinesia.
57. The compound as recited in Clause 56 wherein at least one of R₂₈-R₅₆ independently has deuterium enrichment of no less than about 10%.
58. The compound as recited in Clause 56 wherein at least one of R₂₈-R₅₆ independently has deuterium enrichment of no less than about 50%.
59. The compound as recited in Clause 56 wherein at least one of R₂₈-R₅₆ independently has deuterium enrichment of no less than about 90%.
60. The compound as recited in Clause 56 wherein at least one of R₂₈-R₅₆ independently has deuterium enrichment of no less than about 98%.
61. The compound as recited in Clause 56 wherein said compound has the structural formula: 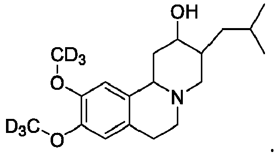
62. The compound of any one of Clauses 56-61, wherein said compound is the alpha stereoisomer.
63. The compound of any one of Clauses 56-61, wherein said compound is the beta stereoisomer.
64. The compound as recited in Clause 56 wherein said compound has the structural formula: 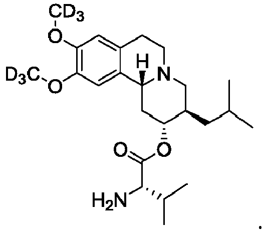
65. A compound of structural Formula III 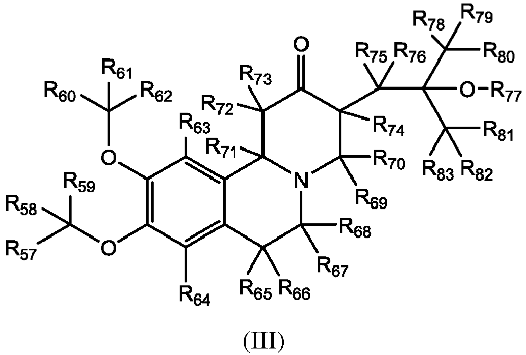 or a salt, stereoisomer, or racemic mixture thereof, wherein:
   R₅₇-R₈₃ are independently selected from the group consisting of hydrogen and deuterium; and
   at least one of R₅₇-R₈₃ is deuterium;
   for use in the treatment of Parkinson's disease levodopa-induced dyskinesia.
66. The compound as recited in Clause 65 wherein at least one of R₅₇-R₈₃ independently has deuterium enrichment of no less than about 10%.
67. The compound as recited in Clause 65 wherein at least one of R₅₇-R₈₃ independently has deuterium enrichment of no less than about 50%.
68. The compound as recited in Clause 65 wherein at least one of R₅₇-R₈₃ independently has deuterium enrichment of no less than about 90%.
69. The compound as recited in Clause 65 wherein at least one of R₅₇-R₈₃ independently has deuterium enrichment of no less than about 98%.
70. The compound as recited in Clause 65 wherein said compound has the structural formula: 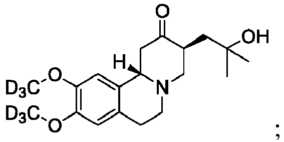 or the 3S,11bS enantiomer, 3R,11bR enantiomer, or a racemic mixture of the the 3S,11bS and 3R,11bR enantiomers.
71. A compound of structural Formula IV 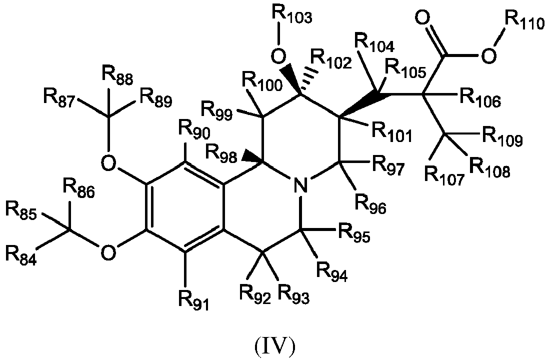 or a salt, stereoisomer, or racemic mixture thereof, wherein:
   R₈₄-R₁₁₀ are independently selected from the group consisting of hydrogen and deuterium; and
   at least one of R₈₄-R₁₁₀ is deuterium;
   for use in the treatment of Parkinson's disease levodopa-induced dyskinesia.
72. The compound as recited in Clause 71 wherein at least one of R₈₄-R₁₁₀ independently has deuterium enrichment of no less than about 10%.
73. The compound as recited in Clause 71 wherein at least one of R₈₄-R₁₁₀ independently has deuterium enrichment of no less than about 50%.
74. The compound as recited in Clause 71 wherein at least one of R₈₄-R₁₁₀ independently has deuterium enrichment of no less than about 90%.
75. The compound as recited in Clause 71 wherein at least one of R₈₄-R₁₁₀ independently has deuterium enrichment of no less than about 98%.
76. The compound as recited in Clause 71 wherein said compound has the structural formula: 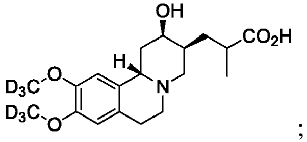 or a diastereomer, or mixture of diastereomers thereof.
77. The compound as recited in any one of Clauses 55, 61, 64, 70, and 76 wherein each position represented as D has deuterium enrichment of no less than about 10%.
78. The compound as recited in any one of Clauses 55, 61, 64, 70, and 76 wherein each position represented as D has deuterium enrichment of no less than about 50%.
79. The compound as recited in any one of Clauses 55, 61, 64, 70, and 76 wherein each position represented as D has deuterium enrichment of no less than about 90%.
80. The compound as recited in any one of Clauses 55, 61, 64, 70, and 76 wherein each position represented as D has deuterium enrichment of no less than about 98%.
81. The compound as recited in any one of Clauses 50-80 further comprising the administration of an additional therapeutic agent.
82. The compound as recited in Clause 81 wherein said additional therapeutic agent is selected from the group consisting of dopamine precursors, DOPA decarboxylase inhibitors, catechol-O-methyl transferase (COMT) inhibitors, dopamine receptor agonists, neuroprotective agents, NMDA antagonists, and anti-psychotics.
83. The compound as recited in Clause 82 wherein said dopamine precursor is levodopa.
84. The compound as recited in Clause 82 wherein said DOPA decarboxylase inhibitor is carbidopa.
85. The compound as recited in Clause 82 wherein said catechol-O-methyl transferase (COMT) inhibitor is selected from the group consisting of entacapone and tolcapone.
86. The compound as recited in Clause 82 wherein said dopamine receptor agonist is selected from the group consisting of apomorphine, bromocriptine, ropinirole, and pramipexole.
87. The compound as recited in Clause 82 wherein said neuroprotective agent is selected from the group consisting of selegeline and riluzole.
88. The compound as recited in Clause 82 wherein said NMDA antagonist is amantidine.
89. The compound as recited in Clause 82 wherein said anti-psychotic is clozapine.
90. The compound as recited in any one of Clauses 50-80, wherein said treatment results in at least one of the clinical effects selected from the group consisting of:
   a. improved Unified Parkinson's Disease Rating Scale scores;
   b. improved Abnormal Involuntary Movement Scale scores;
   c. improved Goetz Dyskinesia Rating Scale scores;
   d. improved Unified Dyskinesia Rating Scale scores;
   e. improved PDQ-39 Parkinson's Disease Questionnaire scores; and
   f. improved Global Primate Dyskinesia Rating Scale scores.
91. The compound as recited in any one of Clauses 50-80, further resulting in at least one effect selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
92. The compound as recited in any one of Clauses 50-80, further resulting in at least two effects selected from the group consisting of:
   a. decreased inter-individual variation in plasma levels of said compound or a metabolite thereof as compared to the non-isotopically enriched compound;
   b. increased average plasma levels of said compound per dosage unit thereof as compared to the non-isotopically enriched compound;
   c. decreased average plasma levels of at least one metabolite of said compound per dosage unit thereof as compared to the non-isotopically enriched compound;
   d. increased average plasma levels of at least one metabolite of said compound per dosage unit thereof as compared to the non-isotopically enriched compound; and
   e. an improved clinical effect during the treatment in said subject per dosage unit thereof as compared to the non-isotopically enriched compound.
93. The compound as recited in any one of Clauses 50-80, wherein the method effects a decreased metabolism of the compound per dosage unit thereof by at least one polymorphically-expressed cytochrome P₄₅₀ isoform in the subject, as compared to the corresponding non-isotopically enriched compound.
94. The compound as recited in Clause 93, wherein the cytochrome P₄₅₀ isoform is selected from the group consisting of CYP2C8, CYP2C9, CYP2C19, and CYP2D6.
95. The compound as recited any one of Clauses 50-80, wherein said compound is characterized by decreased inhibition of at least one cytochrome P₄₅₀ or monoamine oxidase isoform in said subject per dosage unit thereof as compared to the non-isotopically enriched compound.
96. The compound as recited in Clause 95, wherein said cytochrome P₄₅₀ or monoamine oxidase isoform is selected from the group consisting of CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.
97. The compound as recited in any one of Clauses 50-80, wherein the method reduces a deleterious change in a diagnostic hepatobiliary function endpoint, as compared to the corresponding non-isotopically enriched compound.
98. The compound as recited in Clause 97, wherein the diagnostic hepatobiliary function endpoint is selected from the group consisting of alanine aminotransferase ("ALT"), serum glutamic-pyruvic transaminase ("SGPT"), aspartate aminotransferase ("AST," "SGOT"), ALT/AST ratios, serum aldolase, alkaline phosphatase ("ALP"), ammonia levels, bilirubin, gamma-glutamyl transpeptidase ("GGTP," "γ-GTP," "GGT"), leucine aminopeptidase ("LAP"), liver biopsy, liver ultrasonography, liver nuclear scan, 5'-nucleotidase, and blood protein.

## Claims

1. A compound of the following formula 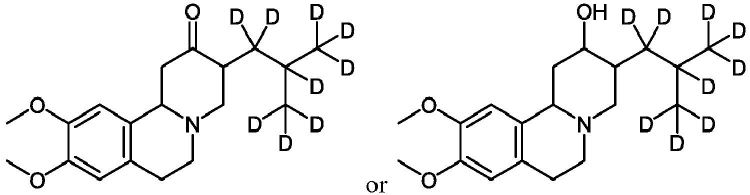 or a salt, stereoisomer, or racemic mixture thereof;
for use in the treatment of Parkinson's disease levodopa-induced dyskinesia.

2. The compound as recited in claim 1, wherein each position represented as D has a deuterium enrichment of no less than 10%.

3. The compound as recited in claim 1, wherein each position represented as D has a deuterium enrichment of no less than 50%.

4. The compound as recited in claim 1, wherein each position represented as D has a deuterium enrichment of no less than 90%.

5. The compound as recited in claim 1, wherein each position represented as D has a deuterium enrichment of no less than 98%.

6. The compound as recited in any one of the preceding claims, further comprising the administration of an additional therapeutic agent.

7. The compound as recited in claim 6, wherein said additional therapeutic agent is selected from the group consisting of dopamine precursors, DOPA decarboxylase inhibitors, catechol-O-methyl transferase (COMT) inhibitors, dopamine receptor agonists, neuroprotective agents, NMDA antagonists, and anti-psychotics.

8. The compound as recited in claim 7, wherein said dopamine precursor is levodopa.

9. The compound as recited in claim 7, wherein said DOPA decarboxylase inhibitor is carbidopa.

10. The compound as recited in claim 7, wherein said catechol-O-methyl transferase (COMT) inhibitor is selected from the group consisting of entacapone and tolcapone.

11. The compound as recited in claim 7, wherein said dopamine receptor agonist is selected from the group consisting of apomorphine, bromocriptine, ropinirole, and pramipexole.

12. The compound as recited in claim 7, wherein said neuroprotective agent is selected from the group consisting of selegeline and riluzole.

13. The compound as recited in claim 7, wherein said NMDA antagonist is amantidine.

14. The compound as recited in claim 7, wherein said anti-psychotic is clozapine.
